(19) Europäisches Patentamt
European Patent Office
Office européen des brevets

(11) EP 1 358 144 B1

(12) **EUROPÄISCHE PATENTSCHRIFT**

(45) Veröffentlichungstag und Bekanntmachung des Hinweises auf die Patenterteilung:
**27.12.2006 Patentblatt 2006/52**

(21) Anmeldenummer: **01987736.4**

(22) Anmeldetag: **16.10.2001**

(51) Int Cl.:
***C07C 49/00*** *(2006.01)*

(86) Internationale Anmeldenummer:
**PCT/EP2001/011992**

(87) Internationale Veröffentlichungsnummer:
**WO 2002/032844 (25.04.2002 Gazette 2002/17)**

(54) **EPOTHILON-SYNTHESEBAUSTEINE I: UNSYMMETRISCH SUBSTITUIERTE ACYLOINE UND ACYLOINDERIVATE, VERFAHREN ZU DEREN HERSTELLUNG SOWIE DEREN VERWENDUNG ZUR HERSTELLUNG VON EPOTHILONEN UND EPOTHILONDERIVATEN**

EPOTHILONE SYNTHESIS COMPONENTS I: ASYMMETRICALLY SUBSTITUTED ACYLOINS AND ACYLOIN DERIVATIVES, METHOD FOR THE PRODUCTION THEREOF AND METHOD FOR THE PRODUCTION OF EPITHILONE AND EPOTHILONE DERIVATIVES

COMPOSANTS SYNTHETIQUES I D'EPOTHILONE : ACYLOINES ET DERIVES D'ACYLOINE A SUBSTITUTION ASYMETRIQUE, LEUR PROCEDE DE PRODUCTION ET PROCEDE DE PRODUCTION D'EPOTHILONE ET DE DERIVES D'EPOTHILONE

(84) Benannte Vertragsstaaten:
**AT BE CH CY DE DK ES FI FR GB GR IE IT LI LU MC NL PT SE TR**

(30) Priorität: **16.10.2000 DE 10051136**
**13.07.2001 DE 10134172**

(43) Veröffentlichungstag der Anmeldung:
**05.11.2003 Patentblatt 2003/45**

(73) Patentinhaber: **R&D-Biopharmaceuticals**
**82152 Martinsried (DE)**

(72) Erfinder:
- **WESSJOHANN, Ludger, A.**
  **06114 Halle/Saale (DE)**
- **SCHEID, Günther**
  **84036 Landshut (DE)**
- **BORNSCHEUER, Uwe**
  **17489 Greifswald (DE)**
- **HENKE, Erik**
  **70180 Stuttgart (DE)**
- **KUIT, Wouter**
  **NL-7161 KK Neede (NL)**
- **ORRU, Romano**
  **NL-1057 NV Amsterdam (NL)**

(74) Vertreter: **Maiwald Patentanwalts GmbH**
**Elisenhof,**
**Elisenstrasse 3**
**80335 München (DE)**

(56) Entgegenhaltungen:
**EP-A- 0 404 672**

- **BIJOY P.; AVERY M.A.: 'Synthetic studies directed towards Epothilone A' TETRAHEDRON LETT. Bd. 39, 1998, Seiten 209 - 212**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft Acyloine, deren Derivate, Verfahren zu deren Herstellung sowie deren Verwendung zur Herstellung von Epothilonen und deren Derivaten. Ferner betrifft die Erfindung Epothilon-Synthesebausteine, Verfahren zu deren Herstellung und die Verwendung der Synthesebausteine zur Herstellung von Epothilonen und deren Derivaten.

Acyloine bzw. α-Hydroxyketone (und-aldehyde), sind wichtige funktionelle Einheiten in vielen biologisch aktiven Substanzen. Sie sind zudem wichtige Zwischenstufen für die Synthese und als kleine bifunktionelle Einheit bedeutend als doppelte Verknüpfungsstelle mit weiteren Synthesebausteinen, z.B. in der Heterocyclenchemie.

Dasselbe gilt für Derivate der Acyloine, insbesondere solche, in denen die Hydroxygruppe verestert oder anderweitig geschützt oder derivatisiert ist. Acyloine mit ungeschützter Hydroxygruppe werden auch als freie Acyloine bezeichnet. Die Keto- (oder Aldehyd-)gruppe kann vor allem durch Acetalbildung, Kondensation, z.B. zu Iminen usw., oder Alkenylierung, z.B. durch Wittig-Typ-Reaktionen, derivatisiert werden.

Allen α-monosubstituierten Acyloinen und deren Derivaten ist gemeinsam, dass sie am α-(OH)-Kohlenstoff, d.h. an der α-Hydroxyposition, ein Chiralitätszentrum besitzen, welches durch die benachbarte Ketogruppe leicht racemisieren kann. Bei vielen Acyloinen, besonders einfach bei freien Acyloinen (PG = H), kann es zusätzlich auch zu einem Acyloinshift, einem Austausch von Keto- und Hydroxygruppe über eine dihydroxyalkenartige Zwischenstufe führen. Dies geschieht bevorzugt unter Basenkatalyse.

Als unsymmetrisch substituierte Acyloine werden im Rahmen der vorliegenden Erfindung solche bezeichnet, die an der Ketogruppe und an der α-Hydroxymethylengruppe unterschiedliche Substituenten $R^1$ und $R^2$ tragen, also solche, die bei einem Acyloinshift ein Acyloin anderer Konstitution bilden. Benennungen der Substituenten beziehen sich immer auf die Standardformel, nicht die Shiftversion mit vertauschter Keto/Alkoholfunktion, die hier nur zu Demonstrationszwecken abgebildet ist.

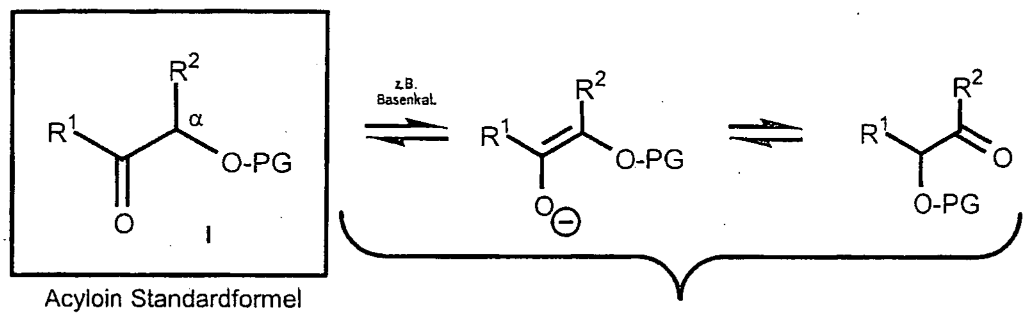


I

Acyloin Standardformel

Acyloinshift

Acyloine werden bevorzugt durch Acyloinkondensation aus Carbonsäurederivaten oder Aldehyden hergestellt, ein Verfahren, welches vor allem für symmetrische Acyloine geeignet ist. Weitere Verfahren nutzen die Oxidation von 1,2-Diolen und die Reduktion von 1,2-Dioxoverbindungen. Letztere Verfahren wurden auch asymmetrisch, auch mittels enzymatischer Reaktionen, durchgeführt (siehe z.B. Bioorg. Chemistry *21,* **1993,** 342; Bull. Chem. Soc. Jpn. **1994,** 3314; J. Chem. Soc., Perkin Trans **I 1991,** 1329, ibid. **1996,** 425; J. Chem. Soc., Chem. Commun. **1993;** 341, J. Org. Chem. *51,* **1986,** 25-36; ibid. **1997,** 1854). Allen vorstehend genannten Verfahren ist gemeinsam, daß sie nicht oder nur schlecht für die gezielte Synthese unsymmetrischer Acyloine anwendbar sind, da Kreuzkupplungen bzw. regioselektive Redoxreaktionen oft schwierig zu beherrschen oder in größerem Maßstab (Cofaktoren) durchführbar sind.

Acyloine und deren Derivate, insbesondere solche mit einem Allylsubstituenten als Rest $R^2$- also Homoallylacyloine (Homoallylalkohole) -, und mit einem Methylrest als bevorzugtem Rest $R^1$, sind zudem ausgezeichnete Bausteine für die Synthese von Epothilonen, wobei die Acyloineinheit sich vor allem als Kohlenstoffatome C15 und C16 gemäß der nachstehend angegebenen Epothilon-Nummerierung wiederfindet.

Epothilone sind Naturstoffe mit außerordentlicher biologischer Wirkung, z.B. als Mitosehemmer, Mikrotubulimodifizierende Agenzien, Cytotoxica oder Fungizide. Insbesondere verfügen sie über Paclitaxel-ähnliche Eigenschaften und übertreffen Paclitaxel (Taxol®) in einigen Tests noch an Aktivität. Sie befinden sich derzeit in klinischen Studien zur Behandlung von Krebsleiden.

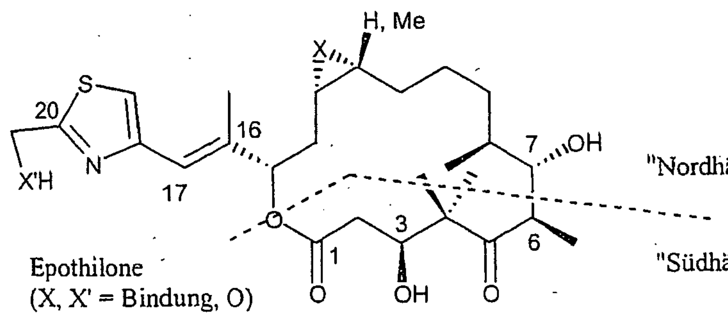

Epothilone
(X, X' = Bindung, O)

"Nordhälfte"

"Südhälfte"

Epothilon, insbesondere Epothilone B und D, weisen eine C7-C18(Methyl)-Einheit in der "Nordhälfte", die strukturell einer modifizierten Polyprenyl- (Polyisopren-) Kette entspricht, und z. B. nach den Deutschen Patentanmeldungen Nr. 197 13 970.1 und Nr. 100 51 136.8 darstellbar ist; sowie eine C1-C6(Methyl)-Einheit in der "Südhälfte" auf, die aus Aldol-Typ-Reaktionen, z. B. gemäß der Deutschen Offenlegungsschrift Nr. 197 01 758.4, (1998), darstellbar ist.

Die Begriffe Strukturelement, Synthesebaustein und Baustein werden im Rahmen der vorliegenden Erfindung synonym verwendet. Die Nummerierung bei Epothilonbausteinen folgt derjenigen des Epothilons (siehe oben). Insbesondere Substituenten und besonders Methylverzweigungen sind bei der Benennung der Bausteine als C(Zahl)-C (Zahl)-Bereich gewöhnlich nicht gesondert erwähnt, sind aber ausdrücklich-jedoch nicht zwingend - mit eingeschlossen.

Bislang wurden zur Herstellung des Struktureiements C7-C21 der Epothilone oder von Untereinheiten, insbesondere C7-C15/16 und C11-C15/16-Bausteinen, üblicherweise Allylverbindungen einschließlich Prenylderivaten, synthetisiert, wobei die Allylverbindungen mit schwierig zugänglichen oder in der falschen Oxidationsstufe befindlichen C15-C16-Methylbausteinen verknüpft wurden.

Bei Verfahren zur Herstellung der Epothilon-Nordhälfte, bei denen an C15 und C16 eine für die Epothilon-Synthese korrekte Oxidationsstufe vorlag, insbesondere gemäß der deutschen Patentanmeldung 100 51 136.8 entstehen üblicherweise Racemate. Somit weisen alle bislang bekannten Methoden zum Aufbau der Epothilon-Nordhälfte den Nachteil auf, daß eine asymmetrische Synthese nur schwierig durchzuführen ist.

In Tetrahedron Letters 39 (1998) 209 - 212 wird die enantioselektive Synthese einer $C_7$ - $C_{15}$ Aldehyd-Untereinheit von Epothilon A beschrieben.

EP 0 404 672 beschreibt die Synthese von Terpen-Derivaten und ihre Verwendung bei der Parfümherstellung und der Vitamin A-Synthese.

α-Hydroxyketone mit mindestens einem chiralem Zentrum an der α-HydroxyPosition sind somit wichtige Vorstufen von biologisch aktiven Substanzen, wie beispielsweise Polyketiden und Terpenoiden und insbesondere von Epothilonen und deren Derivaten. Ein wirtschaftliches Herstellungsverfahren ist deshalb von großer Bedeutung.

Eine optimale und wirtschaftliche, gegebenenfalls enzymkatalysierte Herstellung von an der α-Hydroxy-Position nicht-racemischen Acyloinen sollte vorteilhafterweise eine Reihe von Bedingungen erfüllen, wie beispielsweise eine hohe Enantioselektivität, bezogen auf die α-Hydroxy-Position des Acyloins; eine hohe Diastereoselektivität; eine gute Raum-Zeit-Ausbeute (kurze Reaktionszeiten, hohe Umsätze bezogen auf ein Enantiomer, hohe Edukt- und Produkt-Konzentrationen); eine hohe Substratbreite des Enzyms, eine hohe chemische Ausbeute des gewünschten Produkts; geringe Katelysatormengen (insbesondere Enzymmengen), eine leichte Reinigung der Syntheseprodukte ; eine gute Löslichkeit von Edukt und Produkt unter den Reaktionsbedingungen; sowie eine kostengünstige Synthese, d.h. leicht herstellbare Edukte, gute Handhabbarkeit der Edukte, Lösungsmittel, Reagenzien und Enzyme.

Aufgabe der vorliegenden Erfindung ist es daher, die vorstehend beschriebenen Nachteile des Standes der Technik zu überwinden und insbesondere ein Verfahren bereitzustellen, das möglichst viele der vorstehend genannten Bedingungen erfüllt.

Insbesondere ist es eine Aufgabe der vorliegenden Erfindung, ein alternatives und verbessertes Verfahren bereitzustellen, mit dem vorzugsweise unsymmetrisch substituierte Acyloine und Acyloinderivate ggf. mit hohem Enantiomerenüberschuß an α-Hydroxy-Position der Acyloine hergestellt werden können. Ferner ist es eine Aufgabe der vorliegenden Erfindung, Verbindungen bereitzustellen, die als Bausteine in der Synthese von Polyketiden und Terpenoiden, insbesondere von Epothilonen und/oder deren Derivaten eingesetzt werden können und die insbesondere an der α-Hydroxy-Position der Acyloin-Einheit nicht racemisch sind.

Wie bereits vorstehend erwähnt wurde Strukturelemente C7-C16-Me der Epothilone oder Untereinheiten, insbesondere C7-C15-Bausteine, bisher mit Prenylderivaten aus nucleophilen Prenylmetallderivaten synthetisiert, z.B. gemäß der in der deutschen Offenlegungsschrift Nr. 197 13 970.1 offenbarten Methode. Diese Methode weist zwar deutliche Vorteile gegenüber anderen Methoden zum Aufbau dieses Strukturelementes auf, enthält jedoch den Nachteil,

daß Prenylmetallspezies, insbesondere solche des Bariums erzeugt werden müssen. Dies ist teuer, aufwendig und führt auch zu Nebenprodukten, z.B. durch Allylumlagerung ($S_N2$'-Angriff) während der Reaktion. Ebenso ist entweder eine Oxidation an C15 oder C16 erforderlich, um die Sauerstofffunktion einzuführen, oder diese Funktion ist passend mitzubringen, was relativ aufwendige Bausteine erfordert, die zudem für weitere Synthesen oft Probleme aufwerfen.

Bei dem Strukturelement C1-C6-Me der Epothilone ergab sich nach den Verfahren des Stands der Technik oft ein Problem bei der Anknüpfung an C7. Diese soll *syn*-spezifisch erfolgen. In bisherig bekannten Verfahren, die vor allem mit Basen erzeugte Enolate nutzen, sind Nebenreaktionen aufgrund dieser Reagenzien möglich, und/oder die Partner können nur mit Hilfe bestimmter Schutzgruppen oder durch geeignete Beeinflussung durch das Stereozentrum an C3 in ausreichender Ausbeute korrekt verknüpft werden.

Eine weitere Aufgabe der vorliegenden Erfindung besteht daher auch darin, die vorstehend beschriebenen Nachteile des Standes der Technik bei der Synthese der Strukturelemente C1-C6 bzw. C7-C16 der Epothilone zu überwinden und insbesondere wenig aufwändige, kostengünstige Verfahren möglichst frei von Nebenreaktionen bereitzustellen, mit denen die gewünschten Strukturelemente in möglichst hoher Ausbeute bereitgestellt werden.

Weitere Aufgaben ergeben sich aus der nachfolgenden Beschreibung.

Zur Lösung der vorstehend genannten Aufgaben dienen die in den unabhängigen Ansprüchen definierten Merkmale.

Vorteilhafte Ausgestaltungen sind in den Unteransprüchen beschrieben.

Die Aufgabe der Erfindung wird insbesondere gelöst, indem Verbindungen mit der allgemeinen Formel I

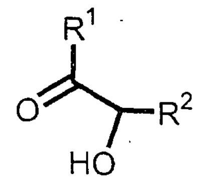

I

bereitgestellt werden, wobei

$R^1$ ausgewählt wird aus der Gruppe bestehend aus H, Methyl, $CH_nF_{3-n}$ (n=0-3), Ethyl oder Propyl; und bevorzugt Methyl ist; und
$R^2$ ein Allylderivat vom Typ A (Anknüpfung bei X)

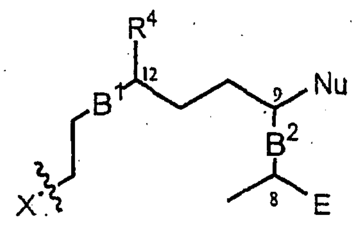

**A**

ist, wobei
$B^1$ und/oder $B^2$ für eine Einfach- oder Doppelbindung als *E*-(*trans*)-Form, *Z*-(*cis*)-Form oder *E*/*Z*-Gemisch oder ein Epoxid als *E*-(*trans*)-Form, *Z*-(*cis*)-Form oder *E*/*Z*-Gemisch und/oder Kombinationen davon steht; und vorzugsweise für Einfach-und Doppelbindungen steht und besonders bevorzugt $B^1$ für eine Z-Doppelbindung oder ein Epoxid und $B^2$ für eine Einfachbindung steht;
$R^4$ Methyl, Ethyl, $CH_nF_{3-n}$ (n = 0-3), oder Halogen ist; E $CH_3$, $CH_2OH$, $CH_2OPG$, CH=O, $CO_2R$, oder $CO_2PG$ ist; R H, Methyl oder Ethyl ist; Nu, H oder Alkyl ist;
und PG für das angegebene Verknüpfungs- atom oder die angegebene funktionelle Gruppe übliche Schutzgruppen bezeichnet, ausgeschlossen eine Verbindung mit $R^1$=Methyl, $B^1$=Doppel bindung, $R^4$=Methyl, Nu-H, $B^2$=Doppelbindung und E=Methyl.

Bei einer bevorzugten Ausführungsform der vorliegenden Erfindung ist die erfindungsgemäße Verbindung an

der α-Hydroxyposition der allgemeinen Formel I nicht racemisch bzw. optisch aktiv. In dieser Ausführungsform sind die erfindungsgemäßen Verbindungen besonders geeignet für den Einsatz in der wirtschaftlichen Synthese von Polyketiden und Terpenoiden, insbesondere von Epothilonen und deren Derivaten, da auf diese Weise eine hohe Enantioselektivität und Diastereoselektivität gewährleistet ist, wodurch insbesondere eine klinische Anwendung der Endprodukte ermöglicht wird.

An der α-Hydroxyposition der allgemeinen Formel I racemische, wie vorstehend beschriebene Verbindungen sind jedoch ebenfalls Gegenstand der vorliegenden Erfindung.

Besonders bevorzugt ist die Verbindung ist an der α-Hydroxyposition der allgemeinen Formel 1 racemisch und/oder nicht racemisch.

Falls der Rest $R^2$ in der allgemeinen Formel I ein Allylrest ist, wird $R^2$ ausgewählt aus der Gruppe bestehend aus 3-Alkylallyl, 3,3-Dialkylallyl, E- oder Z-3-Halogenallyl und 3,3-Dihalogenallyl.

Einebesonders bevorzugte erfindungsgemäße Verbindung weist als Rest $R^2$ in der allgemeinen Formel I ein Allylderivat vom Typ A (Anknüpfung an Formel I bei X)

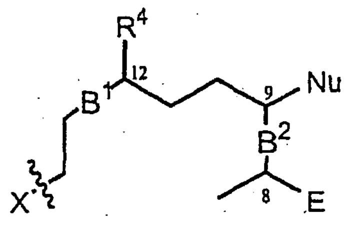

A

auf, wobei

$B^1$ und/oder $B^2$ für eine Einfach- oder eine Doppelbindung als *E*-(*trans*)-Form, *Z*-(*cis*)-Form oder *E*/*Z*-Gemisch oder ein Epoxid als *E*-(*trans*)-Form, *Z*-(*cis*)-Form oder *E*/*Z*-Gemisch und/oder Kombinationen davon steht; und vorzugsweise $B^1$ für eine Z-Doppelbindung oder ein Epoxid und $B^2$ für eine Einfachbindung steht;
$R^4$ Methyl, Ethyl, $CH_nF_{3-n}$ (n = 0-3), oder Halogen ist; E $CH_3$, $CH_2OH$, $CH_2OPG$, CH=O, $CO_2R$, oder $CO_2PG$ ist; R H, Methyl oder Ethyl ist; Nu H oder Alkyl ist; und PG für das angegebene Verknüpfungsatom oder die angegebene funktionelle Gruppe übliche schutzgruppe bezeichnet, ausgeschlossen eine Verbindung mit $R^4$=Methyl, $B^1$=Doppelbindung, $R^4$=Methyl, Nu=H, $B^2$= Doppelbindung und E=Methyl.

Für die Verwendung als Epothilon-Baustein ist es besonders vorteilhaft, wenn Rest $R^2$ bzw. A ausgewählt wird aus der Gruppe bestehend aus einem Neryl- und einem Geranyl-Rest, insbesondere aus der Gruppe bestehend aus einem an der ω-Position zum Alkohol, zum Aldehyd oder zur Carbonsäure oxidierten und/oder ω-1/2-hydrierten Neryl- bzw. Geranylderivat. Beispiele für den Rest $R^2$ können somit ausgewählt werden aus der Gruppe bestehend aus einem ω-Hydroxy- und einem ω-Oxo-(ω-1/2)-dihydro-nerylrest.

Ebenfalls von besonderem Vorteil im Hinblick auf einen möglichen Einsatz der erfindungsgemäßen Verbindungen in der Synthese der Epothilon-Nordhälfte ist es, wenn die absolute Konfiguration an der α-Hydroxyposition der allgemeinen Formel I der natürlichen Konfiguration von Epothilonen an Position C15 entspricht.

Bei einer weiteren möglichen Ausführungsform der vorliegenden Erfindung ist die α-Hydroxygruppe der allgemeinen Formel I durch eine Schutzgruppe PG geschützt. Insbesondere ist die Schutzgruppe PG eine Acylgruppe Besonders bevorzugt wird die Schutzgruppe PG ausgewählt aus der Gruppe bestehend aus einer Acetyl-, Propionyl-, Butyroyl und Benzoyl-Gruppe.

Ein Beispiel der erfindungsgemäßen Verbindung weist die allgemeine Formel III

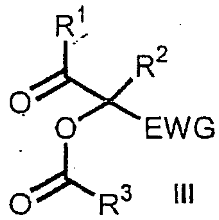

III

auf, in der

$R^2$ = Propinyl und/oder Propinylderivate vom Typ $CH_2$-C≡C-R mit R = Alkyl, Homoallyl, $CH_2X$;
$R^3$ ausgewählt wird aus der Gruppe bestehend aus Alkyl, Benzyl und Phenyl; und bevorzugt ausgewählt aus der Gruppe bestehend aus Methyl, Ethyl und Propyl; und EWG eine elektronenziehende funktionelle Gruppe wie $CO_2PG$ ist.

Ein weiteres Beispiel für die erfindungsgemäßen Verbindungen weist die allgemeine Formel IV

IV

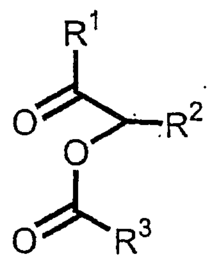

auf, in der

$R^2$ = Propinyl und/oder Propinylderivate vom Typ $CH_2$-C≡C-R mit R= Alkyl, Homoallyl, $CH_2X$; und
$R^3$ ausgewählt wird aus der Gruppe bestehend aus Alkyl, Benzyl und Phenyl; und bevorzugt ausgewählt aus der Gruppe bestehend aus Methyl, Ethyl und Propyl.

Bei einem weiteren Aspekt der vorliegenden Erfindung wird eine Verbindung bereitgestellt, bei der die α-Hydroxygruppe der allgemeinen Formel I

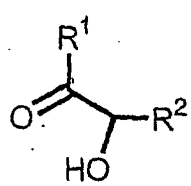

I

durch eine nicht-racemische chirale Acylgruppe geschützt ist, wobei
$R^1$ ausgewählt wird aus der Gruppe bestehend aus H, Methyln $CH_nF_{3-n}$ (n = 0-3), Ethyl oder Propyl und bevorzugt Methyl ist; und
$R^2$ ein Allylderivat vom Typ A (Anknüpfung bei X)

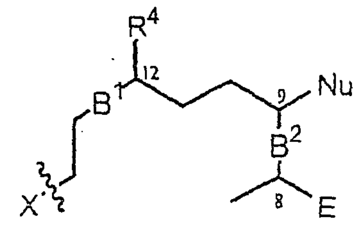

A

ist, wobei

$B^1$ und/oder $B^2$ für eine Einfach- oder Doppelbindung als *E*-(*trans*)-Form, *Z*-(*cis*)-Form oder *E*/*Z*-Gemisch oder ein Epoxid als *E*-(*trans*)-Form, *Z*-(*cis*)-Form oder *E*/*Z*-Gemisch und/oder Kombinationen davon steht; und vorzugsweise $B^1$ für eine Z-Doppelbindungoder ein Epoxid und $B^2$ für eine Einfachbindung steht;

$R^4$ Methyl, Ethyl, $CH_nF_{3-n}$ (n = 0-3), oder Halogen ist; E $CH_3$, $CH_2OH$, $CH_2OPG$, CH=O, $CO_2R$, oder $CO_2PG$ ist; R H, Methyl oder Ethyl ist, Nu H oder Alkyl ist,

und PG für das angegebene Verknüpfungsatom oder die angegebene funktionelle Gruppe übliche Schutzgruppe bezeichnet, ausgeschlossen eine Verbindung mit $R^1$=Methyl, $B^1$=Doppelbindung, $R^4$=Methyl, Nu=H, $B^2$=Doppelbindung und E=Methyl.

Derartige an der α-Hydroxy-Position mit nicht-racemischen Acylgruppen versehene Verbindungen sind besonders geeignet als Baustein für die Synthese von Polyketiden und Terpenoiden, Epothilonen und/oder deren Derivaten, da sie eine Diastereomerentrennung der Synthesebausteine ermöglichen und folglich nach Abspaltung der Acylgruppe die Herstellung enantiomerenangereicherter Acyloine bzw. Acyloinderivate und weiterer Epothilonbausteine gewährleistet ist.

Insbesondere wird die α-Hydroxygruppe der allgemeinen Formel 1 durch eine nichtracemische chirale Acylgruppe ausgewählt aus der Gruppe bestehend aus 2-Alkoxy- und 2-(N,N-Di-PG-amino)acylgruppen geschützt. Besonders bevorzugt ist die α-Hydroxygruppe der all gemeinen Formel I mit optisch aktiver 2-Alkoxymandelsäure oder 2-Alkoxymilchsäure und vorzugsweise mit 2-Methoxymandelsäure als nicht racemischer Schutzgruppe verestert.

Weitere Beispiele für nicht-racemische chirale Schutzgruppen, insbesondere nicht-racemische chirale Acylgruppen sind dem Fachmann bekannt.

Bei einem weiteren Aspekt der vorliegenden Erfindung wird eine Verbindung mit der allgemeinen Formel VI

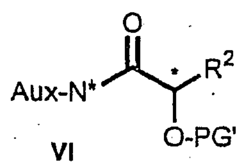

VI

bereitgestellt, wobei

Aux-N* ein N-O-Alkyl, vorzugsweise N-O-Methyl, und/oder ein Evans-Typ-Auxiliar vom Oxazolidinon- oder Imidazolidinon-Typ ist;

$R^2$ ein Allylderivat vom Typ A (Anknüpfung bei X)

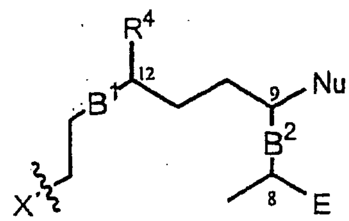

A

ist, wobei

$B^1$ und/oder $B^2$ für eine Einfach- oder eine Doppelbindung als *E*-(*trans*)-Form, *Z*-(*cis*)-Form oder *E*/*Z*-Gemisch oder ein Epoxid als *E*-(*trans*)-Form, Z-(*cis*)-Form oder *E*/*Z*-Gemisch und/oder Kombinationen davon steht; und vorzugsweise $B^1$ für eine Z-Doppelbindung oder ein Epoxid und $B^2$ für eine Einfachbindung steht;

$R^4$ Methyl, Ethyl, $CH_nF_{3-n}$ (n = 0-3), oder Halogen ist; E $CH_3$, $CH_2OH$, $CH_2OPG$, CH=O, $CO_2R$ oder $CO_2PG$ ist; R-H, Methyl oder Ethyl ist; Nu H oder Alkyl ist; und PG für das angegebene Verknüpfungsatom oder die angegebene funktionelle Gruppe übliche Schutzgruppe bezeichnet, ausgeschlossen eine Verbindung mit $R^1$ = Methyl, $B^2$ = Doppelbindung, $R^4$ = Methyl, Nu = H, $B^2$ = Doppelbindung und E = Methyl. und vorzugsweise Rest $R^2$ ausgewählt wird aus der Gruppe bestehend aus einem Neryl- und einem Geranyl-Rest, und besonders bevorzugt Rest $R^2$ ausgewählt wird aus der Gruppe bestehend aus einem an der ω-Position zum Alkohol, zum Aldehyd oder zur Carbonsäure oxidierten und/oder ω-1/2-hydrierten Neryl- bzw

Geranylderivat, und am meisten bevorzugt Rest $R^2$ ausgewählt wird aus der Gruppe bestehend aus einem ω-Hydroxy- und ω-Oxo-(ω-1/2)-dihydro-neryl; und PG' ausgewählt wird aus der Gruppe bestehend aus H und Schutzgruppen, die bei üblicher Enolisierung der auxiliarmodifizierten Zwischenstufen nicht reagieren, und vorzugsweise ausgewählt wird aus der Gruppe bestehend aus Silyl, Benzyl und Oxymethylderivaten.

Mit den vorstehend genannten erfindungsgemäßen, mit einem Evans-Typ-Auxiliar versehenen Acyloinen bzw. Acyloin-Derivaten ist ebenfalls eine enantiomerenangereicherte Synthese von Polyketiden und Terpenoiden, Epothilonen und/oder deren Derivaten möglich.

Vorzugsweise ist das Evans-Typ-Auxiliar Aux-N* ein optisch aktives Oxazolidinon- oder N-Methylimidazolidinon mit der allgemeinen Formel

Aux-N* = 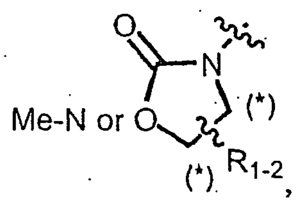

wobei

R an den die Chiralitätszentren darstellenden Positionen 4 und/oder 5 des Oxazolidinons bzw. N-Methylimidazolidinons ausgewählt wird aus der Gruppe bestehend aus Methyl, Phenyl, Isopropyl, Benzyl, polymeren Resten und Kombinationen davon.

Beispielsweise leitet sich das Evans-Typ-Auxiliar von Ephedrinen ab.

Bei einem weiteren Aspekt der vorliegenden Erfindung wird eine Verbindung mit der allgemeinen Formel V

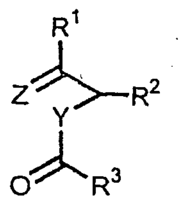

**V**

bereitgestellt, wobei

Y ausgewählt wird aus der Gruppe bestehend aus S, NH, N-PG, NR, 0; und vorzugsweise ausgewählt wird aus der Gruppe bestehend aus NH, N-PG, NR und O; und besonders bevorzugt O ist;

$R^1$ ausgewählt wird aus der Gruppe bestehend aus H, Methyl, $CH_nF_{3-n}$ (n=0-3), Ethyl oder Propyl und bevorzugt Methyl ist; und

$R^2$ ein Allylderivat vom Typ A (Anknüpfung bei X)

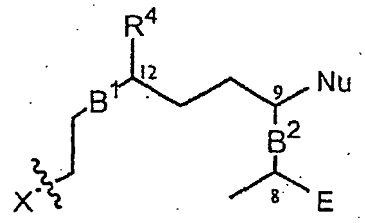

A

ist, wobei

$B^1$ und/oder $B^2$ für eine Einfach- oder eine Doppelbindung als *E*-(*trans*)-Form, Z-(*cis*)-Form oder *E*/*Z*-Gemisch oder ein Epoxid als *E*-(*trans*)-Form, *Z*-(*cis*)-Form oder *E*/*Z*-Gemisch und/oder Kombinationen davon steht; und vorzugsweise $B^1$ für eine Z-Doppelbindung oder ein Epoxid und $B^2$ für eine Einfachbindung steht;

$R^4$ Methyl, Ethyl, $CH_nF_{3-n}$ (n = 0-3), oder Halogen ist; E $CH_3$, $CH_2OH$, $CH_2OPG$, CH=O, $CO_2R$, oder $CO_2PG$ ist R H, Methyl oder Ethyl ist;

Nu H oder Alkyl ist; und PG für das angegebene Verknüpfungsatom oder die angegebene funktionelle Gruppe übliche Schutzgruppe bezeichnet, ausgeschlossen eine Verbindung mit $R^1$=Methyl, $B^1$=Doppelbingung, $R^4$=Methyl, Mu=H, $B^2$=Doppelbindung und E=Methyl.

$R^3$ ausgewählt wird aus der Gruppe bestehend aus Methyl, Ethyl und Propyl; und Z ausgewählt wird aus der Gruppe bestehend aus =O, =N-Nu, =CH-Hetaryl, =CH-Aryl und =$PR_3$; und vorzugsweise =CH-Hetaryl ist; und besonders bevorzugt ausgewählt wird aus der Gruppe bestehend aus (*E*)-(2-Methylthiazol-4-yl)-CH= und (*E*)-(2-Methyloxazol-4-yl)-CH=ist; wobei alle Gruppen Z in (*E*)-form, (*Z*)-Form oder als (*E*/*Z*)-Gemisch vorliegen können,

Eine derartige Verbindung mit der allgemeinen Formel V, insbesondere wenn sie an der $\alpha$-Hydroxyposition der allgemeinen Formel V nicht-racemisch bzw. optisch aktiv ist und/oder Z ein Carbonylrest ist, stellt ein besonders bevorzugtes Strukturelement für die Epothilon-Synthese dar.

Bei einem weiteren Gegenstand der vorliegenden Erfindung wird ein Verfahren zur Herstellung der vorstehend beschriebenen erfindungsgemäßen $\alpha$-Hydroxyketon-Verbindungen bereitgestellt, das mindestens einen der nachfolgenden Schritte umfaßt:

a) Umsetzung von Verbindungen der allgemeinen Formel II

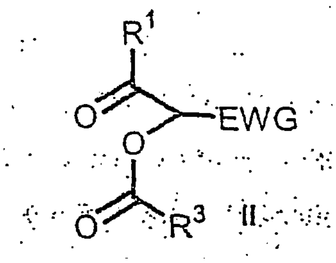

II

wobei

$R^1$ ausgewählt wird aus der Gruppe bestehend aus H, Methyl, $CH_nF_{3-n}$ (n=0-3), Ethyl oder Propyl;

$R^3$ ausgewählt aus der Gruppe bestehend aus Alkyl, Benzyl und Phenyl oder ein chiraler, nicht racemischer Rest R* ist; und besonders bevorzugt ausgewählt aus der Gruppe bestehend aus Methyl, Ethyl und Propyl und EWG eine elektronenziehende funktionelle Gruppe wie $CO_2PG$ und bevorzugt $CO_2$-t-Bu ist;

mit einem Allylderivat vom Typ A (Anknüpfung bei X)

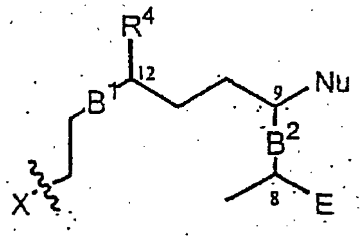

A

wobei X ausgewählt wird aus der Gruppe bestehend aus Cl, Br, I, O-Tosyl, Methylsulfonat, Trifluormethylsulfonat, Alkanoaten und Arylcarboxylaten;
$B^1$ und/oder $B^2$ für eine Einfach- oder eine Doppelbindung als *E*-(*trans*)-Form, *Z*-(*cis*)-Form oder *E*/*Z*-Gemisch oder ein Epoxid als *E*-(*trans*)-Form, Z-(*cis*)-Form oder *E*/*Z*-Gemisch und/oder Kombinationen davon steht; und vorzugsweise $B^1$ für eine Z-Doppelbindung oder ein Epoxid und $B^2$ für eine Einfachbindung steht;
$R^4$ Methyl, Ethyl, $CH_nF_{3-n}$ (n = 0-3), oder Halogen ist; E $CH_3$, $CH_2OH$, $CH_2OPG$, CH=O, $CO_2R$ oder $CO_2PG$ ist; R.H, Methyl oder Ethyl ist; Nu H oder Alkyl ist; und PG für das angegebene VerKnüpfungsatom oder die angegebene funktionelle Gruppe übliche Schutzgruppe bezeichnet, ausgeschlossen eine Verbindung mit $R^1$=Methyl, $B^1$=Doppelbindung, $R^4$=Methyl, Nu=H, $B^2$=Doppelbindung und E=Methyl.
zu Verbindungen der allgemeinen Formel III

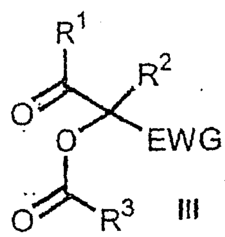

III ;

b) Umsetzung der Verbindung der allgemeinen Formel III zu Verbindungen der allgemeinen Formel IV

IV

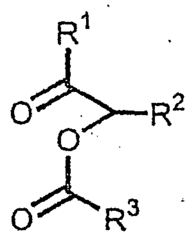

,

beispielsweise durch Alkyldecarboxylierung oder Verseifen/Decarboxylierung von Verbindungen der allgemeinen Formel III mit EWG = $CO_2PG$, vorzugsweise nach der dem Fachmann bekannten Krapchp-Methode, besonders bevorzugt ausgehend von III mit EWG = $CO_2tBu$ in Gegenwart geeignet starker Säuren, vorzugsweise flüchtiger und/oder wasserfreier Säuren, besonders bevorzugt Trifluoressigsäure;

c) Solvolyse der Verbindung der allgemeinen Formel IV zu freien α-Hydroxyketonen der allgemeinen Formel I.

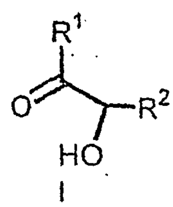

I

in Gegenwart geeigneter Lösemittel und ggf, geeigneter Katalysatoren, und/oder

d) Racematspaitendo Veresterung von Verbindungen der allgemeinen Formel I.

Die vorstehend beschriebenen Verfahrensschritte a) bis d) können in beliebiger, dem Fachmann geläufiger und vorzugsweise in der vorstehend angegebenen Reihenfolge durchgeführt werden. Ferner können je nach gewünschtem Produkt einer oder mehrere der Verfahrensschritte weggelassen werden.

Die Umwandlung der Verbindungen der allgemeinen Formel II mit Resten $R^2$ erfolgt beispielsweise durch dem Fachmann geläufige Propinylierungen oder Allyllierungen, wobei die Reste $R^2$ an ihrer Verknüpfungsstelle vorzugsweise mit H; OH; oder Halogen oder anderen üblichen Abgangsgruppen und deren Kombinationen; bevorzugt Cl, Br, I, O-Tosyl, Methylsulfonat, Trifluormethylsulfonat, Alkanoat und Arylcarboxylate; und besonders bevorzugt H, Cl, Br; versehen sind.

Insbesondere erfolgt die Solvolyse in Schritt c) des erfindungsgemäßen Verfahrens durch Hydrolyse mit Wasser und/oder Umesterung mit Alkoholen.

Als Katalysatoren für die Solvolyse in Schritt c) sind basische, saure und/oder Lewissäurekatalysatoren denkbar. Vorzugsweise werden die Katalysatoren ausgewählt werden aus der Gruppe bestehend aus Alkalihydroxiden, Erdalkalihydroxiden, Alkalihydrogencarbonaten, Erdalkalihydrogencarbonate, Alkalicarbonaten, Erdalkalicarbonaten, Stickstoffbasen, anorganischen Säuren, organischen Säuren, insbesondere aromatischen Sulfonsäuren oder polymergebundenen Säuren, und Lewissäurekatalysatoren wie Lanthanidensalzen, Titansalzen, insbesondere Titan(IV)alkoxiden.

Einige Lewissäurekatalysatoren können entsprechend ihrer chemischen Eigenschaften nur unter wasserfreien Bedingungen eingesetzt werden.

Geeignete Lösemittel sind dem Fachmann bekannt. Beispiele werden nachstehend im Zusammenhang mit weiteren erfindungsgemäßen Verfahren zur Synthese von Epothilon-Synthesebausteinen angegeben.

Die Reaktionstemperatur liegt im Bereich von -80˚C bis 150˚C, je nach Reaktivität und Lösemittelsystem, vorzugsweise jedoch im Bereich von 0˚C bis 90˚C, und besonders bevorzugt im Bereich von 15˚C bis 35˚C liegt.

Die Aufarbeitung erfolgt üblicherweise durch Ausschütteln mit den üblichen organischen Lösemitteln, Trocknen und gegebenenfalls destillative, kristallisierende und/oder chromatographische Aufreinigung.

Ferner ist bevorzugt, daß die Solvolyse in Schritt c) durch die milde basische Hydrolyse mit leicht basischen Katalysatoren, bevorzugt in wässerigen und/oder alkoholischen Lösungen, mit höheren Alkalicarbonaten oder verdünnten Alkalihydroxiden als Katalysator erfolgt, wobei vorzugsweise derart während der Reaktion zudosiert wird, daß der pH-Wert den einer Carbonatlösung nicht übersteigt. Die Reaktion erfolgt dabei bevorzugt in Methanol oder Ethanol mit wässriger Natrium- oder Kaliumcarbonatlösung.

Bei weiteren, im folgenden beschriebenen Ausfühningsformen werden erfindungsgemäße Verfahren beschrieben, mit denen durch asymmetrische Synthese oder Racematspaltung, vorzugsweise jedoch durch enzymatische und/oder chemischphysikalische Racematspaltung nichtracemische, unsymmetrisch substituierte Acyloine und Acyloinester und deren Derivate hergestellt werden können, vorzugsweise solche Derivate, die als Epothilon-Synthesebausteine dienen können, ganz besonders solche die in der deutschen Patentanmeldung 100 51 136.8 beschrieben sind, auf deren Offenbarung hiermit ausdrücklich Bezug genommen wird.

Racematspaltungen sind bislang nur selten an Acyloinen durchgeführt worden, nahezu ausschließlich an symmetrischen Acyloinen, ohne Acyloinshiftproblematik oder mit einem schlechterem Ergebnis bezüglich Enantiomerenreinheit und/oder Ausbeute und/oder Drehwert, und/oder aufwendigeren als den im folgenden beschriebenen Verfahren (siehe z.B. J. Chem. Soc. Chem. Commun. **1997**, 1399; Tetrahedron: Asymmetry *10,* **1996,** 2207; ibid. **1997,** 2773; Tetrahedron Lett. **1997,** 6429). Ferner gibt es wenige Beispiele zur Racematspaltung von Homoallylalkoholen (z. B. Tetrahedron: Asymmetry *10*, **1999,** 315). Auch die C15-Racematspaltung fortgeschrittener Epothilonnordhälften mit Thiazolmethylidenseitenkette lieferte nur unzureichende Enantiomerenüberschüsse (Tetrahedron Lett. *41,* **2000,** 1863-1866; s.a. Synthesis **1999,** 1469). Die insbesondere für Epothilonbausteine wichtige Kombination zu Homoallylacyloinen, d.h. allylisch substituierten Acyloinen ($R^2$ = Allyl und Derivate, insbesondere **Y**) wurde bislang nicht erfolgreich der enzymatischen Racematspaltung unterworfen. Für diese Racemattrennungen müssen insbesondere Substrate mit geschützten und z. T. ungeschützten funktionellen Gruppen (z. B. 7-OH) umgesetzt werden, die die weitere Umsetzung zu Epothilonen und Epothilonderivaten erlaubt.

Wie im folgenden beschrieben wird, werden im Rahmen der vorliegenden Erfindung nun erstmals geeignete Bedingungen und insbesondere geeignete Enzyme für diese Umsetzungen zur Verfügung gestellt. Ferner wurde erstmals gefunden, dass bestimmte genetisch durch in vitro-Evolution veränderte Enzyme vorteilhaft für die Synthese dieser nichtracemischen Epothilonnordhälfte-Bausteine eingesetzt werden können.

In der folgenden Darstellung ist schematisch ein Beispiel für die Synthese racemischer Acyloinvorstufen und Acyloine beschrieben:

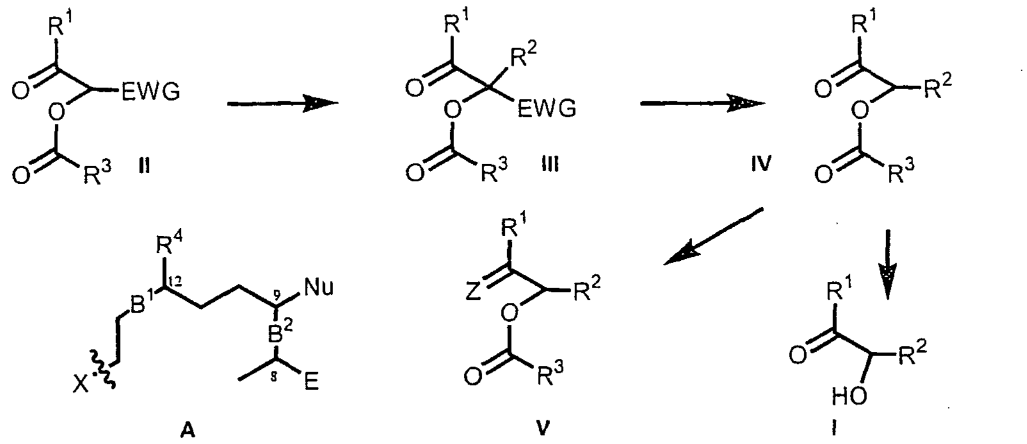

II III IV A V I

Überraschenderweise wurde gefunden, daß der Einsatz bestimmter nichtracemischer Acyloxygruppen als Schutzgruppen für die α-Hydroxygruppe von Acyloinen bzw. Acyloin-Derivaten die Diastereomerentrennung von Acyloinvorstufen und/oder geschützten Acyloine erlaubt, und somit beispielsweise nach Hydrolyse oder anderer Umsetzung, die Synthese enantiomerenangereicherter Acyloine bzw.
Acyloinderivate sowie ggf. fortgeschrittener Epothilonbausteine möglich ist.
Insbesondere wurde dabei gefunden, dass dies aus den Diastereomeren ganz oder größtenteils unter Erhalt eines Enantiomerenüberschusses gelingt.

In der folgenden Darstellung ist eine idealisierte Diastereomerentrennung veranschaulicht:

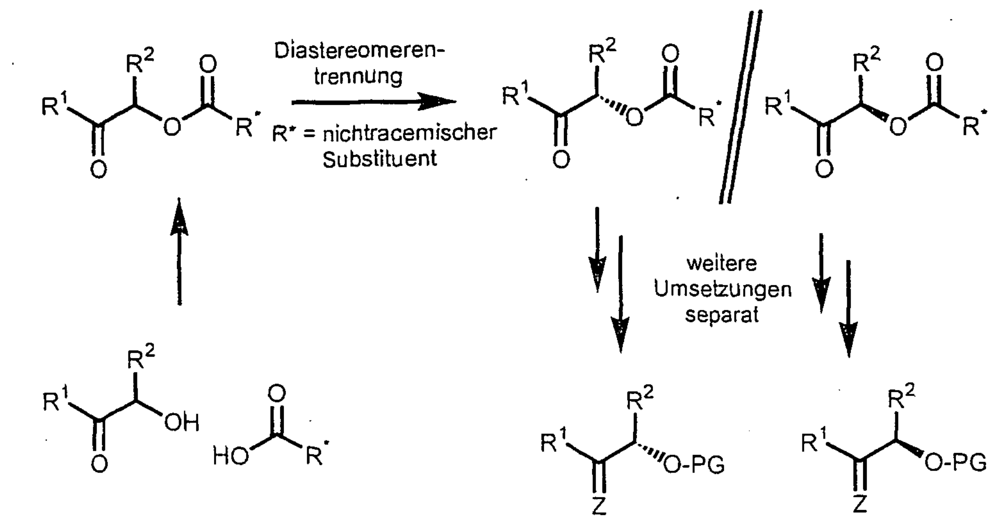


Somit wird bei einer besonders bevorzugten Ausführungsform der vorliegenden Erfindung ein Verfahren bereitgestellt, bei dem die Herstellung von an der α-Hydroxyposition nicht-racemischen Verbindungen der allgemeinen Formel I durch Umsetzung von Verbindungen mit den allgemeinen Formeln II, III und IV erfolgt, deren α-Hydroxygruppe durch eine nicht-racemische chirale Acylgruppe ($R^3 = R^*$) geschützt ist.

Insbesondere kann bei dieser Ausführungsform die α-Hydroxygruppe mit optisch aktiver 2-Alkoxymandelsäure oder 2-Alkoxymilchsäure und vorzugsweise mit 2-Methoxymandelsäure verestert werden.

Ferner wurde im Rahmen der vorliegenden Erfindung gefunden, daß auch die auxiliargesteuerte Synthese von derartigen Acyloinen mit 2-oxysubstituierten Acetylderivaten analog der Evans-Methodik gelingt.

In der folgenden Darstellung ist eine "enantioselektive" Synthese von Acyloinen veranschaulicht (N-Aux = chirales Auxiliar vom Evans-Typ, bevorzugt Oxazolidinon- oder insbesondere Imidazolidinon-Derivate):

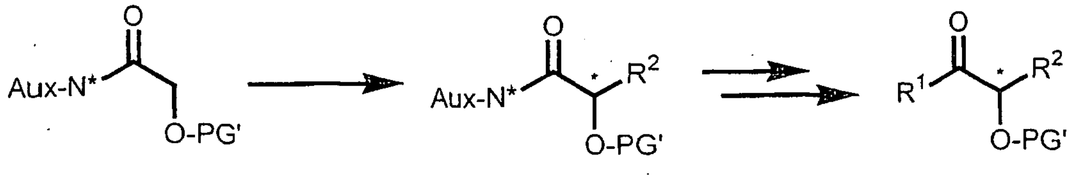

Somit erfolgt bei einer alternativen Ausführungsform des erfindungsgemäßen Verfahrens die Herstellung von an der α-Hydroxyposition nicht-racemischen Verbindungen der allgemeinen Formel I über Zwischenprodukte in Form von Verbindungen der allgemeinen Formel VI

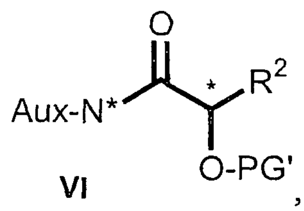

VI

,

und ggf. anschließende Reinigung durch Chromatographie, wobei PG' ausgewählt wird aus der Gruppe bestehend aus H und Schutzgruppen, die bei üblicher Enolisierung der auxiliamiodifizierten Zwischenstufen nicht reagieren, und vorzugsweise ausgewählt wird aus der Gruppe bestehend aus Silyl, Benzyl und Oxymethylderivaten.

Die Verbindungen mit der allgemeinen Formel VI werden bei dieser Ausführungsform insbesondere bereitgestellt durch Umsetzung von nicht-racemischen 4- und/oder 5-substituierten N-[O-PG]-Acetyl-N-methylimidazolidinonen, bei denen PG eine Schutzgruppe ist, mit α-Hydroxyketon-Verbindungen ggf. in Gegenwart von Base, vorzugsweise Lithiumdialkylamiden und/oder Alkalihexamethyldisilaziden. Die α-Hydroxyketon-Verbindungen umfassen den Rest $R_2$, insbesondere den Rest Y, besonders bevorzugt Nerylderivate, sowie geeignete Abgangsgruppen X.

Die Reinigung und gegebenenfalls Diastereomerentrennung erfolgt bevorzugt durch flüssigchromatographische Verfahren an achiralen Festphasen wie Kieselgel.

Besonders bevorzugt erfolgt die Herstellung von an der α-Hydroxyposition nicht-racemischen Verbindungen der allgemeinen Formel I durch Addition von Organometallverbindungen, vorzugsweise von Alkyllithium- oder Alkylmagnesiumverbindungen, besonders bevorzugt von Methyl- bzw. Ethyllithium bzw. Methyl- bzw. Ethylgrignardverbindungen, an Verbindungen der allgemeinen Formel VI.

Es wurde ferner überraschenderweise gefunden, dass racemisches freies Acyloin unter teilweiser, vorzugsweise überwiegender und besonders bevorzugt nahezu vollständiger Racematspaltung enzymatisch zu O-Acyl-acyloinen verestert werden kann.

In der folgenden Darstellung ist eine enzymatische Veresterung unter Racecnatspaltung idealisiert veranschaulicht:

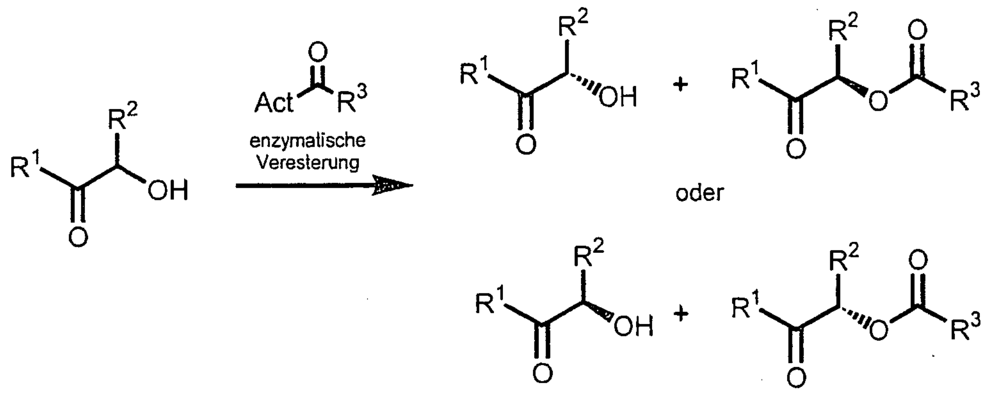


oder

Somit erfolgt bei einer weiteren alternativen und besonders bevorzugten Ausführungsform des erfindungsgemäßen Verfahrens die Herstellung von an der $\alpha$-Hydroxyposition nicht-racemischen Verbindungen der allgemeinen Formel I bzw. IV durch racematspaltende enzymatische Veresterung von Verbindungen des Typs I.

Insbesondere erfolgt die Veresterung mit einem Aktivester, vorzugsweise Isopropenyl- und/oder Vinylacetat.

Die die Veresterung katalysierenden Enzyme werden vorzugsweise ausgewählt aus der Gruppe bestehend aus Lipasen und Esterasen, und sind besonders bevorzugt Lipasen, beispielsweise Lipasen aus *Pseudomonas cepacia* und/oder *Candida antarctica.*

Alternativ werden Esterasen aus *Pseudomonas fluorescens* und/oder *Streptomyces diastatochromogenes* eingesetzt.

Beispielsweise wird eine Lösung des Acyloins in einem geeigneten organischem Lösemittel, bevorzugt einem Alkan, besonders bevorzugt Toluol; mit einer Lipase oder Esterase, auch in immobilisierter oder anderweitig zubereiteter Form, bevorzugt einer Lipase; und einem Aktivester, bevorzugt Isopropenyl- oder Vinylacetat, besonders bevorzugt in leichtem Überschuß; bevorzugt zwischen 10-70°C, besonders bevorzugt beim enzymatischen Optimum oder in einem Bereich von plus/minus 10°C um das enzymatische Optimum, ggf. unter Agitation, bevorzugt scherfreiem Rühren oder Schütteln, zur Reaktion gebracht, bis die gewünschte Umsetzung erfolgt ist. Die Reaktion wird durch Aufarbeitung oder bevorzugt Zentrifugieren beendet, das Lösemittel abgenommen und durch Vakuumverdampfung entfernt. Ester und nicht reagiertes freies Acyloin können destillativ, kristallisierend oder chromatographisch getrennt werden, bevorzugt chromatographisch an fester Phase, z. B. an Kieselgel oder Aluminiumoxid.

Ferner wurde im Rahmen der vorliegenden Erfindung überraschenderweise gefunden, daß es alternativ bei racemischen O-acylierten Acyloinen (PG = Acyl, bevorzugt Acetyl, Butyryl, Benzoyl) gelingt, mittels enzymatischer Hydrolyse eine Racematspaltung zu nicht-racemischem freiem Acyloin und nicht-racemischem O-Acyl-acyloin entgegengesetzter Stereochemie zu erreichen.

In der folgenden Darstellung ist eine derartige enzymatische hydrolytische Racematspaltung idealisiert veranschaulicht:

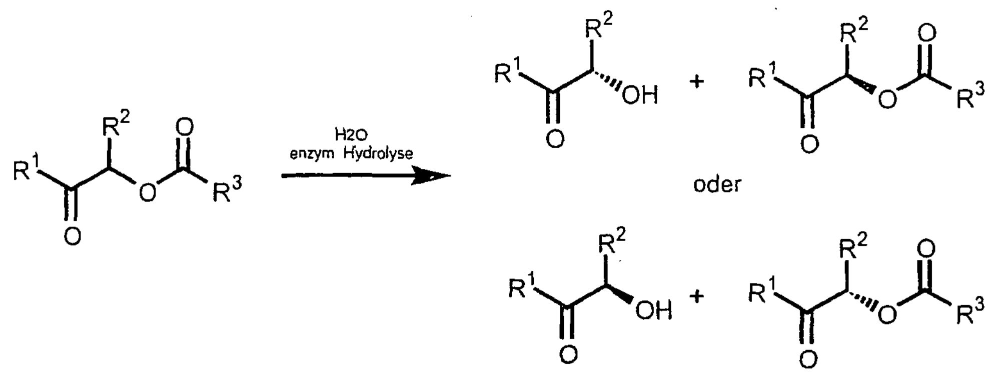


oder

Somit erfolgt bei einer weiteren alternativen und ebenfalls besonders bevorzugten Ausführungsform des erfindungsgemäßen Verfahrens die Herstellung von an der $\alpha$-Hydroxyposition nicht-racemischen Verbindungen der allgemeinen Formel I bzw. IV durch racematspaltende enzymatische Hydrolyse von Verbindungen des Typs IV.

Vorzugsweise wird das die Hydrolyse katalysierende Enzym ausgewählt aus der Gruppe bestehend aus Lipasen und Esterasen. Beispielsweise können Lipasen aus *Pseudomonas cepacia* und/oder *Candida antarctica* eingesetzt werden. Alternativ sind Esterasen aus *Pseudomonas fluorescens* und/oder *Streptomyces diastatochromogenes* vorteilhaft. Besonders bevorzugt wird als Enzym rekombinante Schweineleberesterase eingesetzt.

Beispielsweise wird bei dieser Ausführunsgform ein Ester der allgemeinen Formel IV in einem Puffer gelöst oder suspendiert, bevorzugt einem Phosphat- oder Carbonatpuffer; und die Hydrolase, bevorzugt eine Lipase oder Esterase, auch in immobilisierter oder anderweitig zubereiteter Form, zugesetzt. Bei größeren Ansätzen kann eine Pufferung bevorzugt durch pH-kontrollierten Zusatz von Base erreicht werden. Der pH-Wert richtet sich nach den bekannten oder bestimmten Optimum- und Grenzwerten des Enzyms, wobei ein pH-Wert zwischen diesen Werten der eine optimale Reaktionsführung erlaubt ideal ist. Die Reaktion erfolgt bevorzugt zwischen 10-70°C, besonders bevorzugt beim enzymatischen Optimum oder in einem plus/minus 10°C um das enzymatische Optimum, bis die gewünschte Umsetzung erfolgt ist, ggf. unter Agitation, bevorzugt durch scherfreies Rühren oder Schütteln. Die Reaktion wird bei einem Umsatz um 50% (bezogen auf Racemat) durchgeführt, ggf. auch etwas darunter oder seltener darüber, wenn

dies einer höheren optischen Reinheit des Zielproduktes (Alkohol bzw. Ester) dienlich ist. Die Aufarbeitung erfolgt durch Extraktion mit geeigneten nicht-wassermischbaren organischen Lösemitteln (Ester, Ether, Aromaten, bei Produktlöslichkeit auch einfache Alkane), bevorzugt mit Ethylacetat oder Toluol. In einigen Fällen läßt sich die Extraktionsausbeute durch den vorherigen Zusatz geringer Mengen Aceton oder anderer Lösevermittler steigern, oder durch das vorherige Abzentrifugieren des Enzyms. Die Aufarbeitung und Reinigung folgt den üblichen auch vorstehend beschriebenen Prozessen.

Die vorstehend beschriebene enzymkatalysierte racematspaltende Hydrolyse bzw. Veresterung findet vorzugsweise in einem Temperaturbereich von 10°C bis 70°C statt. Insbesondere die Lipase CAL-B hat ein Temperaturoptimum von 60°C und kann ohne weiteres auch bei Temperaturen bis zu 70°C eingesetzt werden. Ein weiterer möglicher bevorzugter Temperaturbereich für die bei den erfindungsgemäßen Verfahren geeigneten Temperaturen ist z.B. 20°C - 45°C.

Die enzymatischen Optima bzw. Temperaturoptima, insbesondere der vorstehend genannten Enzyme sind dem Fachmann bekannt oder können durch einfache Versuche ermittelt werden.

Bei sämtlichen vorstehend beschriebenen erfindungsgemäßen Verfahren ist es vorteilhaft, wenn zwischen den jeweiligen Syntheseschritten bzw. vor dem Einsatz als Synthesebaustein beispielsweise in der Epothilon-Synthese eine Diastereomerentrennung von Verbindungen der allgemeinen Formeln I, II, III, IV und/oder V, deren $\alpha$-Hydroxygruppe durch eine nicht-racemische chirale Acylgruppe ($R^3 = R^*$) geschützt ist, durchgeführt wird. Die Diastereomerentrennung kann beispielsweise durch Kristallisation, Cokristallisation, Destillation, sequentielle Extraktion, z. B. HSCCC (high speed counter current chromatography) und/oder chromatographische Trennung, vorzugsweise durch chromatographische Trennung, besonders bevorzugt durch flüssigchromatographische Verfahren an achiralen Festphasen wie Kieselgel erfolgen.

Bei einer weiteren bevorzugten Ausführungsform werden die Verbindungen der allgemeinen Formel IV zu Verbindungen der allgemeinen Formel V

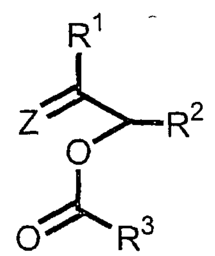

**V**

umgesetzt,
wobei Z ausgewählt wird aus der Gruppe bestehend aus =O, =N-Nu, =CH-Hetaryl, =CH-Aryl und =$PR_3$; und vorzugsweise =CH-Hetaryl ist; und besonders bevorzugt ausgewählt wird aus der Gruppe bestehend aus (*E*)-(2-Methylthiazol-4-yl)-CH= und (*E*)-(2-Methyloxazol-4-yl)-CH=; wobei alle Gruppen Z in (*E*)-form, (*Z*)-Form oder als (*E*/*Z*)-Gemisch vorliegen können.

Vorzugsweise erfolgt die Umsetzung durch dem Fachmann bekannte Wittigtyp-Reaktionen. Besonders bevorzugt erfolgt die Umsetzung zu einer Verbindung mit der allgemeinen Formel

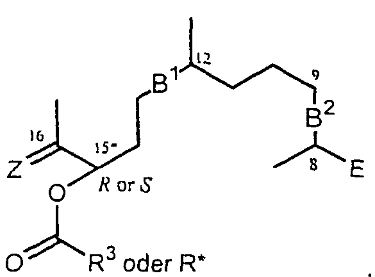

Auf diese Weise werden für die Epothilon-Synthese besonders geeignete Bausteine zur Verfügung gestellt.

Sämtliche vorstehend beschriebenen Umsetzungen können an getrennten Diastereomeren und/oder am Diastereomerengemisch erfolgen.

Sämtliche vorstehend beschriebenen Synthesebausteine und Verfahrensschritte können bei der Bereitstellung von Verbindungen mit der allgemeinen Formel I, vorzugsweise in nicht-racemischer Form an der $\alpha$-Hydroxyposition, eingesetzt werden.

Bei einer weiteren alternativen Ausführungsform des erfindungsgemäßen Verfahrens werden die Verbindungen der allgemeinen Formeln I, II, IV und/oder V unter wasserfreien, stark basischen Bedingungen, vorzugsweise mit den Basen Trialkylamin, DBU, DBN und/oder polymeren starken Basen racemisiert. Auf diese Weise wird eine Rückführung in die Racematspaltung ermöglicht.

Dazu wird beispielsweise das freie oder geschützte Acyloin in einem wasserfreien organischen Lösemittel, bevorzugt einem Alkan, Ether und/oder Aromaten, gelöst und mit der Base versetzt. Die Temperatur, vorzugsweise 0˚C bis 65˚C, besonders bevorzugt 25˚C bis 40˚C, kann zwecks schnellerer Isomerisierung bis zum Siedepunkt des Lösemittels erhöht werden, sofern es nicht zu starken Nebenreaktionen kommt. Nach Beendigung der Isomerisierung wird durch Kieselgel filtriert oder mit Säure ausgewaschen oder mit Scavenger-Harz gerührt um den basischen Katalysator zu entfernen.

Ein weiterer Gegenstand der vorliegenden Erfindung ist die Verwendung der erfindungsgemäßen Verbindungen als Baustein, insbesondere als $C_{15}$-$C_{16}$-haltiger Baustein für die Herstellung von Epothilonen und deren Derivaten.

Schließlich ist ein weiterer Gegenstand der vorliegenden Erfindung die Verwendung der erfindungsgemäßen Verfahren bei der Herstellung von Epothilonen und deren Derivaten.

Erfindungsgemäß hergestellte Synthesebausteine weisen die allgemeine Strukturformel I, II, III, IV und V, vorzugsweise die Formel I, IV oder V mit $R^1$ = Me und $R^2$ = A auf, und können in racemischer oder nichtracemischer Form oder als einzelne Diastereomere oder als Diastereomerengemisch vorliegen. Die Strukturelemente mit den allgemeinen Formeln I und V können vorzugsweise als Produkte oder als Zwischenprodukte in der Synthese von Wirkstoffen verwendet werden. Außerdem können die erfindungsgemäßen Strukturelemente mit den allgemeinen Formeln I bis V verwendet werden für die Synthese von Polyketid- und Terpenoid-Naturstoffen oder Derivaten von Polyketid- und Terpenoid-Naturstoffen, vorzugsweise, wie auch Strukturelemente der allgemeinen Formel V, für die Synthese von makrocylischen Wirkstoffe wie Epothilonen und deren Derivaten einschließlich Stereoisomeren und/oder Homologen, Nor-Verbindungen, und/oder als ganz oder teilweise invertierte Elemente, bei denen sie vorzugsweise als C15/C16-Bausteine, als C12-C16-Bausteine, als C11-C16- und C7-C16-Bausteine oder besonders bevorzugt als C7-C16(Me)-Bausteine des Ringes dienen können, die zusätzlich bereits vorgebildete Elemente oder auch die komplette Seitenkette an C15 oder C16 des Ringes tragen können. Die Synthesebausteine sind vorzugsweise angereichert in einer enantiomeren und/oder diastereomeren Form, besonders bevorzugt der den natürlichen Epothilonen entsprechenden absoluten Konfiguration. Ferner werden erfindungsgemäße Verbindungen mit der allgemeinen Strukturformel I, IV und V bereitgestellt, bei denen die funktionellen Gruppen ganz oder teilweise durch PG geschützt wurden.

Die erfindungsgemäßen Verbindungen mit den allgemeinen Formeln I; II, III, IV und V werden an C15 (oder bei Shift-Acyloinen an C16) in optisch aktiver Form erfindungsgemäß u.a. bereitgestellt durch asymmetrische Synthese mit Alkoxyacetylverbindungen verknüpft an Evanstyp-Auxiliare, durch Diastereomerenbildung und anschließende Trennung mit optisch aktiven Säuren und deren Derivaten, und durch Racematspaltung mittels enzymatischer Hydrolyse oder Veresterung; bevorzugt durch eines der letzteren beiden Verfahren, besonders bevorzugt durch enzymatische Verfahren.

Die erfindungsgemäßen Verbindungen mit den allgemeinen Formeln I, II, III, IV und V. weisen üblicherweise in der niedrigsten C-Position wenigstens einen Substituenten auf, der geeignet ist, Verknüpfungen mit anderen Epothilonbausteinen zu ermöglichen, z. B. an C7 besonders mit C1-C6-Bausteinen, wie sie z. B. in der deutschen Patentanmeldung DE 197 01 758.4 aufgeführt sind.

Im Zusammenhang mit den im Rahmen der vorliegenden Erfindung beschriebenen $\alpha$-Hydroxyketon-Verbindungen und gegebenenfalls auch im Zusammmenhang mit den weiteren nachfolgend beschriebenen erfindungsgemäßen Epothilon-Synthesebausteinen VII bis XVI umfassen die Reste in den vorstehend angegebenen allgemeinen Formeln die folgenden Substituenten:

$R^1$: H, Methyl, $CH_nF_{n-3}$ (n=0-3), Ethyl oder Propyl

$R^2$ Allylderivate vom Typ A (Anknüpfung bei X)

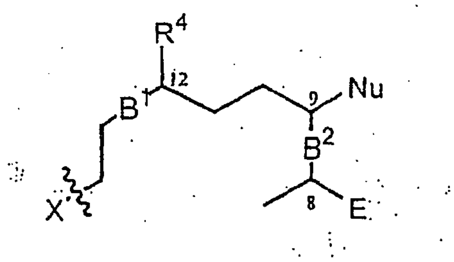

A

wobei

$B^1$, $B^2$: Einfach- Doppelbindung als *E*-(*trans*)-Form, *Z*-(*cis*)-Form oder *E*/*Z*-Gemisch; auch Epoxid als *E*-(*trans*)-Form, *Z*-(*cis*)-Form oder *E*/*Z*-Gemisch; bevorzugt Einfach-und Doppelbindungen; besonders bevorzugt mit $B^1$ als Z-Doppelbindung oder Epoxid und mit $B^2$ als Einfachbindung; und/oder Kombinationen davon;

$R^4$: Methyl, Ethyl, $CH_nF_{3-n}$ (n = 0-3), Halogen, oder Kombinationen davon

E: $CH_3$, $CH_2OH$, $CH_2OPG$, CH=O, $CO_2R$, $CO_2PG$, vorzugsweise $CH_3$, $CO_2R$, $CO_2PG$, besonders bevorzugt $CH_2OH$, $CH_2OPG$, CH=O;

X: Anknüpfungspunkt (als Formelteil) oder: H; OH; oder Halogen oder andere übliche Abgangsgruppen und deren Kombinationen; bevorzugt Cl, Br, I, O-Tosyl, Methylsulfonat, Trifluormethylsulfonat, Alkanoat und Arylcarboxylate; besonders bevorzugt H, Cl, Br;

Nu: H oder Alkyl.

$R^3$: Alkyl, Benzyl, Phenyl, bevorzugt Methyl, Ethyl oder Propyl,

R*: chirale nichtracemische Reste, insbesondere von solchen optisch aktiven Carbonsäuren und Derivaten (Estern, Halogeniden, Anhydriden) R*-CO-X, die käuflich sind oder üblicherweise für Diastereomerenbildung eingesetzt werden, z.B. Mandelsäure, O-geschützte Mandelsäure, Milchsäure, O-geschützte Milchsäure, N-geschützte Aminosäuren, Weinsäurederivate, Camphersäure etc., bevorzugt $\alpha$-chirale N-geschützte Amino- und O-geschützte Hydroxycarbonsäuren, besonders bevorzugt O-Alkylmandelsäure, ganz besonders mit R* = CH(OMe)Ph.

Z: =O, =N-Nu, =CH-Hetaryl, =CH-Aryl, $=PR_3$; wobei alle vorgenannten doppelgebundenen Gruppen Z in (*E*)-form, (*Z*)-Form oder als (*E*/*Z*)-Gemisch vorliegen, bevorzugt =CH-Hetaryl; besonders bevorzugt =O, (*E*)-(2-Methylthiazol-4-yl)-CH=, (*E*)-(2-Methyloxazol-4-yl)-CH=.

EWG: übliche elektronenziehende funktionelle Gruppen-wie $CO_2PG$

N-Aux*: übliche Evans-Typ Auxiliare vom Oxazolidinon - (Evans et al. *Pure & Appl. Chem.* **1981**, 53, 1109-1127) oder Imidazolidinon-Typ (Close, W. J. *J. Org. Chem.,* **1950**, *15,* 1131), abgeleitet von nicht-racemischen Aminoalkoholen oder Diaminen, kommerziell erhältlich oder zugänglich aus Aminosäuren, Ephedrinen etc. durch literaturbekannte Verfahren, besonders bevorzugt vom Ephedrin abgeleitete nicht-racemische Imidazolidinone.

Alkyl bezeichnet Kohlenwasserstoffreste, auch verzweigte Isomere, mit vorzugsweise 1 bis 8 Kohlenstoffatomen.

Aryl bezeichnet Phenyl, Naphthyl, Benzyl und deren Derivate mit bis zu fünf Alkyl-, Alkoxy- oder Halogensubstituenten, bevorzugt jedoch solche mit bis zu drei Substituenten, besonders bevorzugt mit bis zu einem Substituenten; bevorzugt sind entsprechende Phenyl- und Benzyl-Derivate; und Kombinationen davon.

Hetaryl oder Heteroaryl bezeichnet fünf- und sechsgliedrige Heteroaromaten mit einem oder mehreren O, S, und N-Atomen; und deren Derivate mit bis zu vier Alkyl-, Alkoxy- oder Halogensubstituenten, bevorzugt jedoch solche mit bis zu zwei Substituenten, besonders bevorzugt mit bis zu einem Substituenten; bevorzugt sind entsprechende Oxazol-, Thiazol- und Pyrimidin-Derivate; besonders bevorzugt Alkylthiazol- und Oxazolderivate; und Kombinationen davon.

PG bezeichnet für das angegebene Verknüpfungsatom oder die angegebene funktionelle Gruppe übliche Schutzgruppen, z. B. entsprechend dem Buch GREENE/WUTS 1991 (Protective Groups in Organic Synthesis, ISBN 0-471-62301-6), wie Allyl, t-Butyl, Methyl, Benzyl, Silyl-, Acyl- oder aktivierte Methylenderivate wie Methoxymethyl, Alkoxyalkyl oder 2-Oxacycloalkyl-Schutzgruppen; bevorzugt - überwiegend für Alkohol und Aminfunktionen - Trimethylsilyl, Triethylsilyl, Dimethyl-tert-butylsilyl, Acetyl, Propionyl, Benzoyl, Tetrahydropyranyl.
PG' sind dabei solche Gruppen, die bei üblicher Enolisierung der auxiliarmodifizierten Zwischenstufen (z. B. mit LDA) nicht reagieren, wie Silyl, Benzyl oder Oxymethylderivate entsprechend obiger Literatur.

Act sind übliche Aktivatoren die für den enzymatischen Acyltransfer geeignet sind. Dies sind üblicherweise Anhydride oder Aktivester wie Alkenylester (Act = O-vinyl, O-isopropenyl etc.), Trifluorethylester, Oximester oder Thioester, bevorzugt Alkenylester, besonders bevorzugt O-Vinyl und O-isopropenyl.

Ein weiterer wesentlicher Aspekt der vorliegenden Erfindung betrifft Epothilon-Synthesebausteine für die Nord- und Südhälfte von Epothilonen sowie Verfahren zu deren Herstellung und Verknüpfung.
Insbesondere sind die durch die vorliegenden Erfindung bereitgestellten Verbindungen geeignet als Baustein für die Synthese von Polyketiden, Terpenoiden, Epothilonen und/oder deren Derivaten. Beispiele für erfindungsgemäße Epothilon-Synthesebausteine sind ausgewählt aus der Gruppe bestehend aus Verbindungen mit den allgemeinen Formeln VII , X, Xa, XI, XIa, XII, XIII, XVa und XVb.

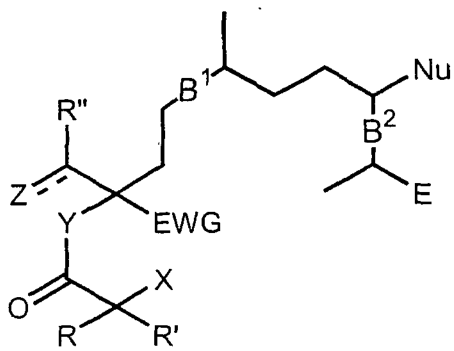

**VII**

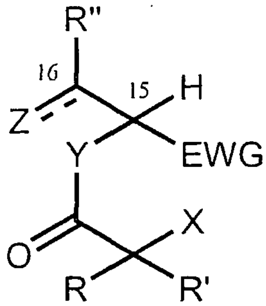

**X**

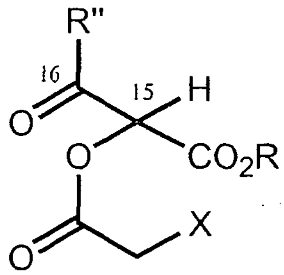

**Xa**

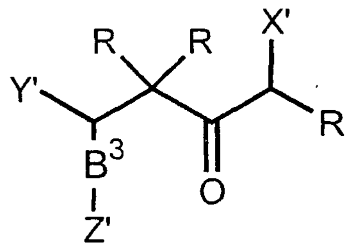

**XI**

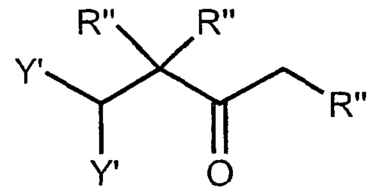

**XIa**

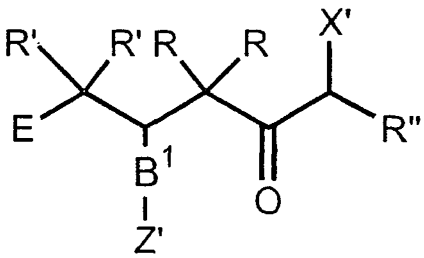

**XII**

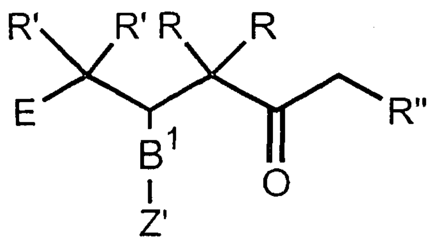

**XIII**

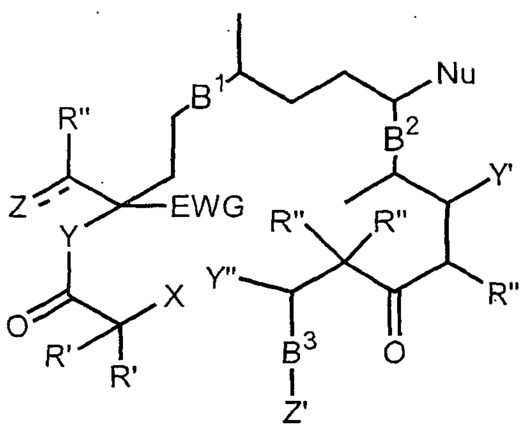

**XVa**

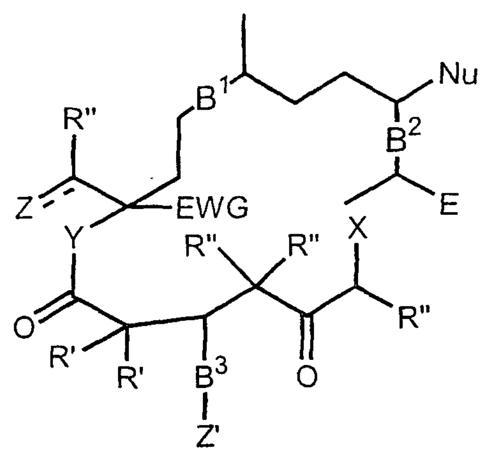

**XVb**

wobei

$B^1$, $B^2$, $B^3$ ausgewählt werden aus der Gruppe bestehend aus Einfachbindungen; Doppelbindungen als *E*-(*trans*)-Form, *Z*-(*cis*)-Form oder *E*/*Z*-Gemisch; Epoxidringe als *E*-(*trans*)-Form, *Z*-(*cis*)-Form oder *E*/*Z*-Gemisch; und Cyclopropanringe als *E*-(*trans*)-Form, *Z*-(*cis*)-Form oder *E*/*Z*-Gemisch; und/oder Kombinationen davon; vorzugsweise ausgewählt werden aus der Gruppe bestehend aus Einfach- und Doppelbindungen; und besonders bevorzugt $B^1$ eine Z-Doppelbindung oder ein Epoxid ist und $B^2$ sowie $B^3$ Einfachbindungen sind;

R ausgewählt wird aus der Gruppe bestehend aus H; Alkyl; Aryl; Alkyl-Aryl wie $CH_2$-Aryl, $C_2H_4$-Aryl und dergleichen; Vinyl; Cycloalkyl, insbesondere ein (3- bis 7-gliedriges Cycloalkyl; $CH_nF_{3-n}$ (n = 0-3); Oxacycloalkyl, insbesondere ein

3- bis 7-gliedriges Oxacycloalkyl und/oder Kombinationen davon; vorzugsweise ausgewählt wird aus der Gruppe bestehend aus H, Methyl, Ethyl, Phenyl und Benzyl; und besonders bevorzugt H, Methyl, Ethyl und/oder Kombinationen davon ist;

R' aus derselben Gruppe ausgewählt wird wie R und besonders bevorzugt H ist;

R" aus derselben Gruppe ausgewählt wird wie R und besonders bevorzugt Methyl ist;

E ausgewählt wird aus der Gruppe bestehend aus $CH_3$, $CH_2OH$, $CH_2OPG$, CH=O, $CO_2R$, $CO_2PG$, $CH_2X$, $CONR_2$, $CON(PG)_2$, CON(OMe)(Me) und CN; vorzugsweise ausgewählt wird aus der Gruppe bestehend aus $CH_3$, $CH_2X$, $CO_2R$, und $CO_2PG$; und besonders bevorzugt ausgewählt wird aus der Gruppe bestehend aus $CH_2OH$, $CH_2OPG$ und CH=O;

X ausgewählt wird aus der Gruppe bestehend aus H; OH; Halogen; anderen üblichen Abgangsgruppen und/oder deren Kombinationen; vorzugsweise ausgewählt wird aus der Gruppe bestehend aus Cl, Br, I, O-Tosyl, Methylsulfonat, Trifluormethylsulfonat, Alkanoaten und Arylcarboxylaten; und besonders bevorzugt ausgewählt wird aus der Gruppe bestehend aus H, Cl und Br;

X' ausgewählt wird aus der Gruppe bestehend aus OH; Halogen, anderen üblichen Abgangsgruppen und/oder deren Kombinationen; vorzugsweise ausgewählt wird aus der Gruppe bestehend aus Cl, Br, I, O-Tosyl, Methylsulfonat, Trifluormethylsulfonat, Alkanoaten und Arylcarboxylaten, und besonders bevorzugt Cl und/oder Br ist;

Y ausgewählt wird aus der Gruppe bestehend aus S, NH, N-PG, NR, O; und vorzugsweise ausgewählt wird aus der Gruppe bestehend aus NH, N-PG, NR und O; und besonders bevorzugt O ist;

Y' ausgewählt wird aus der Gruppe bestehend aus H, OH, OR, O-PG, $NH_2$, $NR_2$, $N(PG)_2$, SR und SH; und vorzugsweise O-PG und/oder OR ist;

Y" ausgewählt wird aus der Gruppe bestehend aus H, OH, OR, O-PG, $NH_2$, $NR_2$, $N(PG)_2$, SR, SH, Cl, Br und $CR'_2$-EWG; vorzugsweise O-PG und/oder OR ist; und besonders bevorzugt H, $CH_2CO_2R$ und/oder $CH_2COR^*$ ist;

Z ausgewählt wird aus der Gruppe bestehend aus -OH, -O-PG, -OR, =O, =N-Nu, =CH-Hetaryl, =CH-Aryl und $=PR_3$; wobei alle vorgenannten doppelgebunden Gruppen Z in (*E*)-form, (*Z*)-Form oder als (E/Z)-Gemisch vorliegen können, vorzugsweise =CH-Hetaryl ist; und besonders bevorzugt ausgewählt wird aus der Gruppe bestehend aus =O, (*E*)-(2-Methylthiazol-4-yl)-CH= und (*E*)-(2-Methyloxazol-4-yl)-CH=;

Z' ausgewählt ist aus der Gruppe bestehend aus O, OH, OR, O-PG, $N(H)_{1-2}$, $N(R)_{1-2}$, $N(PG)_{1-2}$, SR, S-PG und R; vorzugsweise O, O-PG und/oder OR ist;

Nu ausgewählt wird aus der Gruppe bestehend aus R, O-PG, OR, $N(PG)_2$, $NR_2$, S-PG, SR, SeR, CN, $N_3$, Aryl und Heteroaryl; vorzugsweise ausgewählt wird aus der Gruppe bestehend aus R, O-PG, OR, $N(PG)_2$ und $NR_2$; und besonders bevorzugt H und/oder Alkyl ist;

EWG ausgewählt wird aus der Gruppe bestehend aus E, CN, CO-R, Dialkylphosphonat, $SO_2$-R, $SO_2OR$, $CF_3$, $CCl_3$ und $NO_2$; vorzugsweise ausgewählt wird aus der Gruppe bestehend aus CN, CO-R und CO-$NR_2$; und besonders bevorzugt $CO_2R$, $CO_2PG$ ist, wobei PG vorzugsweise *tert*-Butyl ist;

Alkyl ausgewählt wird aus Kohlenwasserstoffen, auch verzweigten Isomeren, beispielsweise mit $C_{1-20}$ und vorzugsweise mit 1 bis 8 Kohlenstoffatomen;

Aryl ausgewählt wird aus der Gruppe bestehend aus Phenyl, Naphthyl, Benzyl und deren Derivaten; vorzugsweise mit bis zu fünf Alkyl-, Alkoxy- oder Halogensubstituenten, besonders bevorzugt solche mit bis zu drei Substituenten, am meisten bevorzugt solche mit bis zu einem Substituenten; und vorzugsweise ausgewählt wird aus der Gruppe bestehend aus Phenyl- und Benzyl-Derivaten und Kombinationen davon;

Hetaryl/Heteroaryl ausgewählt wird aus der Gruppe bestehend aus fünf- und sechsgliedrigen Heteroaromaten mit einem oder mehreren O-, S- und N-Atomen; und deren Derivaten mit bis zu vier Alkyl-, Alkoxy- und/oder Halogensubstituenten; vorzugsweise solche mit bis zu zwei Substituenten, besonders bevorzugt mit bis zu einem Substituenten; vorzugsweise ausgewählt wird aus der Gruppe bestehend aus Oxazol-, Thiazol- und Pyrimidin-Derivaten; und besonders bevorzugt ein Alkylthiazolderivat ist; und Kombinationen davon;

PG eine Schutzgruppe ist, vorzugsweise ausgewählt aus der Gruppe bestehend aus Allyl, Methyl, t-Butyl (bevorzugt bei EWG), Benzyl, Silyl, Acyl und aktivierten Methylenderivaten wie Methoxymethyl, Alkoxyalkyl und 2-Oxacycloalkyl; vorzugsweise - überwiegend für Alkohol und Aminfunktionen - ausgewählt aus der Gruppe bestehend aus Trimethylsilyl, Triethylsilyl, Dimethyl-tert-butylsilyl, Acetyl, Propionyl, Benzoyl, Tetrahydropyranyl; sowie aus Schutzgruppen, die benachbarte oder zweiwertige Gruppen ($PG_2$) unter Bildung 5-7-gliedriger Ringe gleichzeitig schützen, wie Succinyl, Phthalyl, Methylen, Acetonid und/oder Kombinationen aller vorgenannten Schutzgruppen.

Vorzugsweise ist E $CH_2X$, $CO_2PG$ und/oder CHO und besonders bevorzugt CHO.

Ferner ist es bevorzugt, daß EWG = H ist; und X H und/oder Halogen. Besonders bevorzugt ist EWG ausgewählt aus der Gruppe bestehend aus Cl, Br und I.

Des weiteren ist es bevorzugt, daß der Substituent Y-CO-CRR'X durch eine OH- oder $NH_2$-Gruppe, vorzugsweise durch OH, ersetzt ist.

Bei den Verbindungen mit der allgemeinen Formel X, insbesondere bei denen mit der allgemeinen Formel Xa, ist R = tert-Butyl und X = H, Cl und/oder Br.

Bevorzugte Ausführungsformen für Verbindungen mit den allgemeinen Formeln XI und/oder XIa sind solche,

bei denen $B^3$ eine Einfach- oder Doppelbindung als E-(*trans*)-Form, *Z*-(*cis*)-Form oder *E*/*Z*-Gemisch ist; vorzugsweise eine Einfach- oder Doppelbindung zu Heteroatomen wie O, S oder N, besonders bevorzugt eine Einfachbindung zu O-PG oder OH; R ausgewählt wird aus der Gruppe bestehend aus H, Methyl, Ethyl, Propyl, Phenyl, Benzyl; vorzugsweise ausgewählt wird aus der Gruppe bestehend aus H, Methyl, Ethyl und Kombinationen davon; R" aus derselben Gruppe ausgewählt wird wie R und besonders bevorzugt Methyl ist.

Bevorzugte Ausführungsformen für Verbindungen mit der allgemeinen Formel XII und/oder mit der allgemeinen Formel XIII, sind solche, bei denen $B^1$ ausgewählt ist aus der Gruppe bestehend aus Einfachbindungen, Doppelbindungen als *E*-(*trans*)-Form, *Z*-(*cis*)-Form oder *E*/*Z*-Gemisch; R ausgewählt ist aus der Gruppe bestehend aus H; Alkyl; Aryl; Alkyl-Aryl wie $CH_2$-Aryl, $C_2H_4$-Aryl und dergleichen; Vinyl; Cycloalkyl, insbesondere ein 3- bis 7-gliedriges Cycloalkyl; $CH_nF_{3-n}$ mit n = 0 bis 3;, Oxacycloalkyl, insbesondere ein 3- bis 7-gliedriges Oxacycloalykl; und/oder Kombinationen davon; vorzugsweise ausgewählt ist aus der Gruppe bestehend aus H, Methyl, Ethyl, Phenyl und Benzyl; und besonders bevorzugt ausgewählt ist aus der Gruppe bestehend aus H, Methyl, Ethyl und Kombinationen davon; R' aus derselben Gruppe ausgewählt ist wie R und besonders bevorzugt H ist; R" aus derselben Gruppe ausgewählt ist wie R und besonders bevorzugt Methyl ist; X' ausgewählt ist aus der Gruppe bestehend aus OH; Halogen; anderen üblichen Abgangsgruppen und/oder deren Kombinationen; vorzugsweise ausgewählt ist aus der Gruppe bestehend aus Cl, Br, I, O-Tosyl, Methylsulfonat, Trifluormethylsulfonat, Alkanoaten und Arylcarboxylaten; und besonders bevorzugt ausgewählt ist aus der Gruppe bestehend aus H, Cl und Br; Z' ausgewählt ist aus der Gruppe bestehend aus O, OH, OR, O-PG, $N(H)_{1-2}$, $N(R)_{1-2}$, $N(PG)_{1-2}$, SR, S-PG und R; vorzugsweise O, O-PG und/oder OR ist; E ausgewählt ist aus der Gruppe bestehend aus $CH_2OH$, $CH_2OPG$, $CH_2OR$, CH=O, CR=O, $CH(OR)_2$, $CH(OPG)_2$, CH=NPG und Kombinationen davon; vorzugsweise ausgewählt ist aus der Gruppe bestehend aus COEt, $CO_2R$, COR* und $CH(OPG)_2$; und besonders bevorzugt ausgewählt ist aus der Gruppe bestehend aus $CO_2R$, $CO_2PG$, $CO_2H$ und COR*; R* ausgewählt ist aus der Gruppe bestehend aus chiralen Resten und Auxiliaren und bei Acylderivaten wie Estern, Amiden und Imiden vorzugsweise ausgewählt ist aus der Gruppe bestehend aus 1-Alkylbenzylamino; 1-Alkylbenzyloxy-; Lactat- und Mandelatderivaten; Monoterpenderivaten, z.B. Oppolzer-Sultam, 8-Arylmenthyloxy und dergleichen; und *N*-gebundenen Oxazolidinonen (Evans-Typ-Auxiliaren); besonders bevorzugt ausgewählt ist aus der Gruppe bestehend aus 4-substituierten Oxazolidinonen; jeweils in enantiomerenreiner, enantiomerenangereicherter, racemischer Form und/oder Mischungen; Alkyl für Kohlenwasserstoffe, auch verzweigte Isomere, steht, vorzugsweise mit $C_{1-20}$, und besonders bevorzugt mit 1 bis 8 Kohlenstoffatomen; Aryl ausgewählt ist aus der Gruppe bestehend aus Phenyl, Naphthyl, Benzyl und deren Derivaten, vorzugsweise mit bis zu fünf Alkyl-, Alkoxy- und/oder Halogensubstituenten, besonders bevorzugt mit bis zu drei Substituenten, am meisten bevorzugt mit bis zu einem Substituenten; und vorzugsweise ausgewählt ist aus der Gruppe bestehend aus entsprechenden Phenyl- und Benzyl-Derivaten und Kombinationen davon; PG eine Schutzgruppe ist und vorzugsweise ausgewählt ist aus der Gruppe bestehend aus Allyl, Methyl, t-Butyl (bevorzugt bei EWG), Benzyl, Silyl, Acyl und aktivierten Methylenderivaten wie Methoxymethyl, Alkoxyalkyl und 2-Oxacycloalkyl-Schutzgruppen; vorzugsweise - überwiegend für Alkohol und Aminfunktionen - ausgewählt ist aus der Gruppe bestehend aus Trimethylsilyl, Triethylsilyl, Dimethyl-tert-butylsilyl, Acetyl, Propionyl, Benzoyl, Tetrahydropyranyl; sowie aus Schutzgruppen, die benachbarte oder zweiwertige Gruppen ($PG_2$) unter Bildung 5-7-gliedriger Ringe gleichzeitig schützen, wie Succinyl, Phthalyl, Methylen, Acetonid; und/oder Kombinationen aller vorgenannten Schutzgruppen; wobei besonders bevorzugt X' = Cl und/oder Br; R' = H; R = Me; E = $CO_2R$, $CO_2PG$, $CO_2H$ und/oder COR*, und/oder $B^1$-Z' = O-PG ist.

Bevorzugte Ausftihrungsformen für Verbindungen mit der allgemeinen Formel XVa und/oder XVb, sind solche, bei denen $B^3$ eine Einfach- oder Doppelbindung als E-(*trans*)-Form, *Z*-(*cis*)-Form oder *E*/*Z*-Gemisch ist; vorzugsweise eine Einfach- oder Doppelbindung zu Heteroatomen wie O, S, N; besonders bevorzugt eine einfache Bindung zu O-PG oder eine Doppelbindung zu O; EWG mit $X \neq H$ besonders bevorzugt H ist; wobei besonders bevorzugt $X \neq H$; EWG = H; Z =O, (E)-(2-Methylthiazol-4-yl)-CH= und/oder (E)-(2-Methyloxazol-4-yl)-CH=; R'= H; R"= Me; und/oder Y" = $CH_2COOH$, OH; Y', Z' = O-PG und/oder OH ist.

Im folgenden werden besondere Ausführungsformen von erfindungsgemäßen Synthesebausteinen für die Nord- bzw. Südhälfte von Epothilonen dargestellt.

<u>Die Nordhälfte:</u>

Es wurde überraschenderweise gefunden, daß natürliche, kommerzielle oder literaturbekannte Prenylderivate, insbesondere Geranyl-, Neryl-, Linaloyl- und Farnesylderivate mit den allgemeinen Formeln VIII und IX, vorzugsweise ausgehend von deren Alkoholen, Acetaten oder Halogeniden, in elektrophile Synthesebausteine des Typs VIII für Epothilone und deren Derivate umgewandelt werden können bzw. als solche genutzt werden können, um durch einfache nukleophile Substitution mit einem C15-C16-Strukturelement mit der allgemeinen Formel X- insbesondere des Acetoacetat-Typs, ganz besonders mit der Formel Xa - verknüpft zu werden, welches an C15 bereits über die geeignete Oxidationsstufe bzw. Substitution Y verfügt.

Der Begriff Derivate beinhaltet homologe, analoge und Nor-Verbindungen, vorzugsweise auch Varianten mit weiteren Substituenten an der Hauptkette oder im C15-C16-Element. Dabei ist je nach Ausgangssubstanz für die

Bereiche C7-C10 und/oder C11-C14 (eine Prenyleinheit), insbesondere jedoch für C7-C14 (zwei Prenyleinheiten, z. B. aus Nerol, Geraniol und Linalool, Y = OH) keine weitere C-C-Verknüpfung erforderlich. Durch die Wahl des Startmaterials (E- oder Z-Prenylverbindung) läßt sich die Stereochemie an C12-C13 vorbestimmen.

Im folgenden sind spezielle Beispiele für Verbindungen mit der allgemeinen Formel VII angegeben.

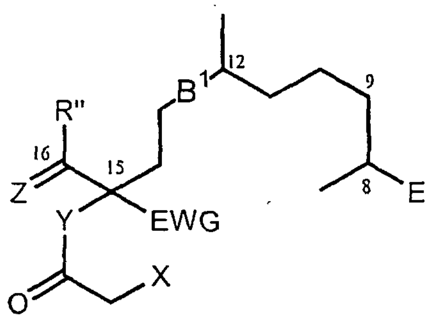

**VIIa**

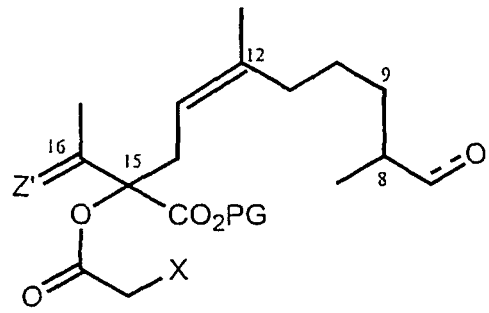

**VIIb**

In Verbindungen VIIb ist Z' vorzugsweise =O, 2-Methylthiazol-4-ylmethylen, 2-Methyloxazol-4-ylmethylen, X = Cl, Br; und besonders bevorzugt H; PG = t-Bu

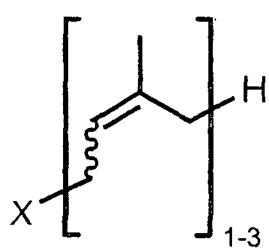

**VIII**

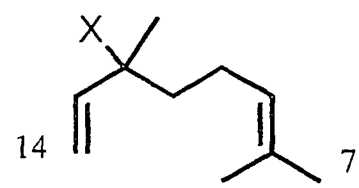

**IX**

In den Verbindungen VIII und IX ist X vorzugsweise OH, OAc, OTs, Cl und/oder Br. Als elektrophile Bausteine besonders bevorzugt sind OTs, Cl, Br.

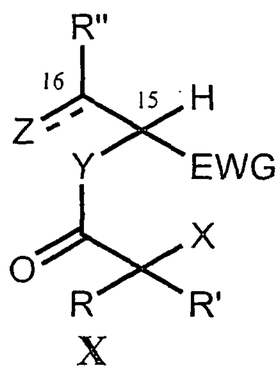

**X**

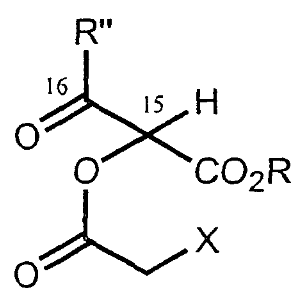

**Xa**

Bei dem C15-16-Baustein mit der Formel IV ist besonders bevorzugt X=H, R=t-Bu und/oder R"=Me.

Erfindungsgemäß hergestellte Synthesebausteine weisen unter anderem die allgemeine Strukturformel VII, vorzugsweise die Formel VIIa, besonders bevorzugt die Formel VIIb auf, und können in racemischer oder nichtracemischer Form oder als einzelne Diastereomere oder als Diastereomerengemisch vorliegen.

Die Strukturelemente VII und X können vorzugsweise als Produkte oder als Zwischenprodukte in der Synthese

von Wirkstoffen verwendet werden. Außerdem können die erfindungsgemäßen Strukturelemente VII für die Synthese von Polyketid- und Terpenoid-Naturstoffen oder Derivaten von Polyketid- und Terpenoid-Naturstoffen verwendet werden, vorzugsweise für makrocylische Wirkstoffe wie Epothilone und deren Derivate einschließlich Stereoisomeren und/oder Homologen, Nor-Verbindungen, und/oder als ganz oder teilweise invertierte Elemente, bei denen sie vorzugsweise als C7-C15- und C7-C16- oder besonders bevorzugt als C7-C14-Bausteine des Ringes dienen können, die gegebenenfalls zusätzlich bereits vorgebildete Elemente oder auch die komplette Seitenkette an C15 des Ringes tragen können.

Diese Bausteine, nachfolgend auch als Synthesebausteine bezeichnet, sind vorzugsweise angereichert an einer enantiomeren und/oder diastereomeren Form, besonders bevorzugt in den den natürlichen Epothilonen entsprechenden Formen.

Ferner werden erfindungsgemäße Verbindungen mit den allgemeinen Strukturformeln VII und X bereitgestellt, bei denen die funktionellen Gruppen ganz oder teilweise durch PG geschützt wurden.

Die erfindungsgemäßen Verbindungen des Typs VII sowie deren Stereoisomere können beispielsweise ausgehend von kommerziell erhältlichen oder nach bekannten Verfahren darstellbaren Ausgangsstoffen VIII und/oder IX durch C-C-Verknüpfung mit 2-oxy-substituierten-1,3-diaktivierten Methylenverbindungen mit der allgemeinen Formel X, vorzugsweise mit 2-Acyloxy-1,3-dicarbonylverbindungen, besonders bevorzugt mit 2-Acyloxy-acetoacetaten, speziell bevorzugt mit Verbindungen der Formel Xa, erhalten werden, wobei insbesondere basische Reaktionsbedingungen vorteilhaft sind.

Es wurde ferner gefunden, daß sich Modifikationen an C7, C8 und C9 der vorstehend genannten Verbindungen durchführen lassen, insbesondere durch Oxidation beispielsweise an C7; Reduktion von beispielsweise C8-9; nukleophile Addition an beispielsweise C9 mit E = EWG; Substitution beispielsweise an C7; Modifikationen an C12-13, beispielsweise Epoxidierung; Modifikationen an C16, beispielsweise Alkenylierung, z.B. Wittig-Typ-Reaktion mit Z = O; Modifikationen an C15 wie Entfernen von EWG, beispielsweise Decarboxylierung; und/oder Umesterung oder Verseifung/Veresterung von Y.

Die vorgenannten Substituenten, Schutzgruppen, Bindungsarten B und/oder Stereoisomeren sowie die Reihenfolge der Verknüpfung oder Modifikation können, soweit es chemisch sinnvoll ist, beliebig geändert und kombiniert werden.

Die erfindungsgemäßen Verbindungen des Typs VII, aber auch des Typs VII mit EWG = H weisen üblicherweise in den Positionen 7, 15 und/oder 16 wenigstens einen Substituenten auf, der nicht Wasserstoff oder R ist, vorzugsweise solche Substituenten, die geeignet sind, Verknüpfungen mit C17-C20-Seitenkettenbausteinen und insbesondere mit C1-C6-Bausteinen oder deren Ausgangsmaterialien herbeizuführen, wie sie z.B. in der deutschen Offenlegungsschrift 197 01 758.4 aufgeführt sind, besonders bevorzugt durch Ester- oder Lactonbildung eines Epothilon-C1-Bausteines mit C15 und/oder Aldol- oder Reformatsky-Typ-Reaktion eines Epothilon-C6-Bausteines mit C7 und/oder Verknüpfung eines C17-C20-Seitenkettenbausteines mit C16, sofern letzterer nicht schon in einem früheren Stadium angeknüpft wurde.

Verbindungen des Strukturelementes des Typs I mit E = $CH_2OH$, CHO, $CO_2R$ lassen sich beispielsweise darstellen, indem Verbindungen VII, vorzugsweise VIIa, besonders bevorzugt Nerylderivate, oxidiert werden, vorzugsweise in 7-Stellung. Dazu können empfindliche Positionen, die nicht oxidiert werden sollen, in üblicher und bekannter Weise (siehe unten) geschützt werden. So werden Alkohole vorzugsweise als Silylether oder Alkanoate und Carbonsäuregruppen vorzugsweise als Ester geschützut. Die Oxidation erfolgt entsprechend der Vorschriften, wie sie z. B. in HUDLICKY 1990 (Oxidations in Organic Chemistry, 0-8412-1781-5/90) aufgeführt sind. Dabei wird vorzugsweise die 7-Position oxidiert, vorzugsweise mit Selenreagenzien, besonders vorzugsweise katalytisch mit Selendioxid bzw. Peroxiden.

In diesem Zusammenhang wurde erfindungsgemäß gefunden, daß je nach Verfahren und der gewählten Menge Selendioxid zunächst Alkohole (E = $CH_2OH$) oder Aldehyde (E = CHO) gebildet werden.

Weitere Oxidation der Alkohole zum Aldehyd vorzugsweise mit aktiviertem DMSO, aber auch Reduktion und Substitution nach üblichen Verfahren machen weitere Substituenten E zugänglich. Weitere Oxidation der Aldehyde, z. B. mit $NaClO_2$ oder Luft/Katalysator, liefert Carbonsäuren.

Die aus den kommerziellen Prenylalkoholen oder nach obigen Verfahren gewonnen Allylalkohole können nach bekannten Verfahren in eine aktivierte Form überführt werden, vorzugsweise in Allylhalogenide, -sulfonate oder -carboxylate, besonders bevorzugt in C7- und C14-Halogenide der Verbindungen VII oder VIII und geschützte Derivate davon.

Weitere Modifikationen können an den Doppelbindungen erfolgen. So können diese nach literaturbekannten Verfahren in Einfachbindungen, Epoxide und Cyclopropane überführt werden. Die Reduktion gelingt nach bekannten Verfahren, vorzugsweise finden Hydriddonoren und besonders bevorzugt katalytische Verfahren, auch asymmetrische Varianten Verwendung. Eine Differenzierung der Doppelbindungen, soweit sie nicht bereits durch Substitutionsmuster der Ausgangsstoffe besteht, kann elektronisch, z. B. durch geeignet gewählte benachbarte Schutzgruppen (elektronenziehend oder-schiebend), oder vorzugsweise sterisch, z.B. durch die Wahl einer geeigneten Reihenfolge der im vorstehend beschriebenen Prenylmodifikationen, erfolgen.

So wurde insbesondere gefunden, das mit geeigneten Gruppen EWG, insbesondere *tert*-Butylcarboxylaten ($CO_2$t-Bu) die C8-C9-Doppelbindung selektiv reduziert werden kann. Eine verbleibende C12-13-Doppelbindung kann z.B. mit Persäuren selektiv epoxidiert werden. Ist die C8-9-Doppelbindung elektronenziehend substituiert (E = Oxomethylderivate, bevorzugt $CO_2R$, CHO), lassen sich selektiv Nucleophile durch Michael-Reaktion in Position C9 einführen, bevorzugt einfache Alkylcuprate, Alkohole und Amine.

Die Reduktionen, Nukleophilen Additionen, Epoxidierungen und Cyclopropanierungen der Doppelbindungen, oder Kombinationen davon, können an geeignet geschützten Prenylderivaten VII nach literaturbekannten Verfahren durchgeführt werden. Soweit chemisch sinnvoll, können einige diese Modifikationen auch noch bei einem einfach oder zweifach an Verbindungen der Formel I (auch mit EWG = H) angeknüpften C1-C6-Baustein erfolgen. Bevorzugt ist auch die Epoxidierung der C12-C13-Doppelbindung.

Ferner wurde erfindungsgemäß gefunden, daß sich Verbindungen des Typs VII, bevorzugt VIIa in Verbindungen des Typs VII mit EWG = H überführen lassen. Besonders bevorzugt verwendet man Verbindungen der Formel VII mit PG=t-Butyl, speziell solche der Formel VIIb, die unter saurer Katalyse in einem Schritt dealkylcarboxylieren zu entsprechenden Verbindungen VII mit EWG = H. Letztere lassen sich ebenfalls mit C1-C6-Bausteinen verknüpfen.

Bei den erfindungsgemäßen Verfahren zur Herstellung der vorstehend genannten Verbindungen ist es bevorzugt, daß die funktionellen Gruppen der Verbindungen des Typs VII-X, vorzugsweise des Typs VIIa, sowie von Intermediaten, in ihre geschützte Form übergeführt werden (M = PG). Geeignete Schutzgruppen sind: Allyl, Benzyl, Methyl, Ethyl, t-Butyl, aktivierte Methylenderivate wie Methoxymethyl, 1-Oxacycloalkyl, Silyl, insbesondere Trialkylsilyl; und - überwiegend für Alkoholfunktionen - auch Acylschutzgruppen, vorzugsweise Acetyl, Propionyl und Benzoyl und deren Derivate. Ebenfalls sind solche Schutzgruppen bevorzugt, die benachbarte Gruppen Y gleichzeitig schützenwie beispielsweise Acetonide, Methylen, Cyclodiacyl und solche, die Carbonylgruppen schützen, insbesondere Acetale und cyclische Acetale (O und S). Weitere für die erfindungsgemäßen Verfahren geeignete Schutzgruppen sind in GREENE/ WUTS 1991 (Protective Groups in Organic Synthesis) beschrieben, worauf ausdrücklich Bezug genommen wird. Auch Kombinationen der genannten Schutzgruppen sind denkbar und je nach Art Vorgehensweise vorteilhaft.

Die Südhälfte

Für die Synthese der Südhälfte von Epothilonen kommt erfindungsgemäß insbesondere die Verwendung der Verbindungen des Typs XII und XIII in Frage.

Es wurde erfindungsgemäß gefunden, daß auch Verbindungen des Typs XI geeignete C3-C6-Bausteine sind, und daß sich diese beispielsweise durch Reaktion von 2-Haloacylhalogeniden mit dem Enamin des Isobutyraldehyds und anschließende Hydrolyse und Acetalisierung darstellen lassen.

Es wurde ferner erfindungsgemäß gefunden, daß in C6-Position aktivierte Verbindungen des Typs XII bzw. XIII, leicht aus Verbindungen des Typs XIII und XIa durch Oxidation, insbesondere durch elektrophile Halogenierung, besonders bevorzugt mit tertiären oder quartären Ammoniumperhalogeniden, ausgesprochen leicht und in guter Ausbeute erhältlich sind.

Es wurde ferner erfindungsgemäß gefunden, daß sich die vorstehend genannten Verbindungen XI bis XIII gut an C7 -etc.-Bausteine, z.B. des Typs VII anknüpfen lassen, bevorzugt vermittelt durch niedervalente Metallionen oder Metalle in Reformatsky-Typ-Reaktionen wie sie beispielsweise in der deutschen Offenlegungsschrift Nr. 197 01 758.4 erläutert sind.

Ebenfalls wurde erfindungsgemäß gefunden, daß die Reaktion, d.h die Umsetzung Mit Verbindungen der allgemeinen Formel VII in außergewöhnlich hohem Maße *syn*-selektiv und aldehydselektiv verläuft, was es ermöglicht, Stoffe vom Typ VII effizient umzusetzen, insbesondere auch solche, bei denen Z ein Keton oder eine Hetarylalkylidenseitenkette ist. Ein besonderer Vorteil des erfindungsgemäßen Verfahrens ist es, daß die Funktionalitäten in großem Ausmaß ungeschützt verwendbar sind. So kann z.B. eine Estergruppe als C1 und eine Ketogruppe an C16 in freier Form eingesetzt werden.

Verknüpfungen von Nord- und Südhälfte

Ein wesentlicher Aspekt der vorliegenden Erfindung betrifft die Verknüpfung von Nord- und Südhälfte. Dabei wurde insbesondere gefunden, das Nord- und Südhälfte mittels der vorstehend genannten Verfahren, die im folgenden und insbesondere in den Beispielen detailliert beschrieben werden, durch Aldolreaktionen, bevorzugt durch solche vom Reformatsky-Typ, besonders bevorzugt mit Chrom(II)salzen, zwischen C6-C7 oder C2-C3, oder sukzessive an beiden Stellen, mitander verknüpft oder - bei vorveresterten Bausteinen - zyklisiert werden können.

Diese völlig neue Methode ermöglicht die Cyclisierung mit hoher syn-Selektivität und Chemoselektivität. Offenkettige Verbindungen können auch durch Macrolactonisierung nach bekannten Verfahren cyclisiert werden.

Auf den verschieden Etappen während der Bausteinsynthese als auch der Macrocyclensynthese lassen sich Modifikationen und Substitutionen erreichen. So wurde beispielsweise gefunden, daß sich Modifikationen an C7, C8

und C9 der vorstehend beschriebenen Bausteine und an geeigneten Zwischenverbindungen auf dem Weg zum Macrocyclus durchführen lassen, insbesondere durch Oxidation, bevorzugt an C7; Reduktion, bevorzugt an C8-9; nukleophile Addition, bevorzugt an C9 mit E = EWG; Substitution, bevorzugt an C7; Modifikationen an C12-13, bevorzugt Epoxidierung; Modifikationen an C16, bevorzugt an Alkenylierung, besonders bevorzugt Wittig-Typ-Reaktion mit Z = O; Modifikationen an C15, bevorzugt Entfernen von EWG, besonders bevorzugt durch Decarboxylierung; sowie Umesterung oder VerseifunglVeresterung von Y. Die vorgenannten Substituenten, Schutzgruppen, Bindungsarten B und/oder Stereoisomeren, sowie die Reihenfolge der Verknüpfung oder Modifikation können, soweit es chemisch sinnvoll ist, beliebig geändert und kombiniert werden.

Im folgenden werden eine Reihe von bevorzugten Ausführungsformen für erfindungsgemäße Verfahren zur Synthese von erfindungsgemäßen Synthesebausteinen VII bis XVI beschrieben:

Bei einer Ausführungsform der erfindungsgemäßen Verfahren werden die Verbindungen der allgemeinen Formeln VII, VIII, X und/oder XI unter wasserfreien, stark basischen Bedingungen, vorzugsweise mit den Basen Trialkylamin, DBU, DBN und/oder polymeren starken Basen racemisiert.

Eine weitere Ausführunsgform der erfindungsgemäßen Verfahren zur Herstellung von Verbindungen der Formel VII umfaßt die Umsetzung von in α- und/oder ω-Stellung elektrophil aktivierten Prenylverbindungen bestehend aus 1-4 Prenyleinheiten mit Verbindungen gemäß der allgemeinen Formel X.

Die in α- und/oder ω-Stellung elektrophil aktivierten Prenylverbindungen werden ausgewählt aus der Gruppe bestehend aus Prenylalkoholen, -acetaten, -halogeniden, Verbindungen der allgemeinen Formel VIII, Verbindungen der allgemeinen Formel IX, und funktionalisierten und/oder geschützten Prenylderivaten der allgemeinen Formel XIV,

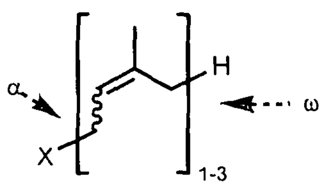

VIII

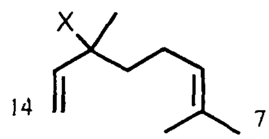

IX

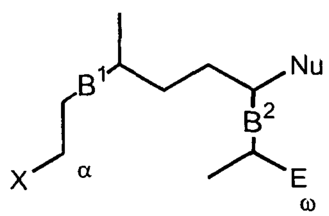

XIV

worin

$B^1$, $B^2$ ausgewählt sind aus der Gruppe bestehend aus Einfach- und Doppelbindungen als *E*-(*trans*)-Form, *Z*-(*cis*)-Form oder *E*/*Z*-Gemisch; Epoxidringen als *E*-(*trans*)-Form, *Z*-(*cis*)-Form oder *E*/*Z*-Gemisch; Cyclopropanringen, als *E*-(*trans*)-Form, *Z*-(*cis*)-Form oder *E*/*Z*-Gemisch; und/oder Kombinationen davon; vorzugsweise ausgewählt sind aus der Gruppe bestehend aus Einfach- und Doppelbindungen; und besonders bevorzugt $B^1$ eine Z-Doppelbindung oder ein Epoxid ist und $B^2$ eine Einfachbindung ist;

E ausgewählt ist aus der Gruppe bestehend aus $CH_3$, $CH_2OH$, $CH_2OPG$, CH=O, $CO_2R$, $CO_2PG$, $CH_2X$, $CONR_2$, $CON(PG)_2$, CON(OMe)(Me) und CN; vorzugsweise ausgewählt ist aus der Gruppe bestehend aus $CH_3$, $CH_2X$, $CO_2R$ und $CO_2PG$; und besonders bevorzugt ausgewählt ist aus der Gruppe bestehend aus $CH_2OH$, $CH_2OPG$ und CH=O;

X ausgewählt ist aus der Gruppe bestehend aus H; OH; Halogen; anderen üblichen Abgangsgruppen und deren Kombinationen; vorzugsweise ausgewählt ist aus der Gruppe bestehend aus Cl, Br, I, O-Tosyl, Methylsulfonat, Trifluormethylsulfonat, Alkanoaten und Arylcarboxylaten; und besonders bevorzugt ausgewählt ist aus der Gruppe bestehend aus

H, Cl und Br;
Nu ausgewählt ist aus der Gruppe bestehend aus R, O-PG, OR, $N(PG)_2$, $NR_2$, S-PG, SR, SeR, CN, $N_3$, Aryl und Heteroaryl; vorzugsweise ausgewählt ist aus der Gruppe bestehend aus R, O-PG, OR, $N(PG)_2$ und $NR_2$; und besonders bevorzugt ausgewählt ist aus der Gruppe bestehend aus H und Alkyl;
R ausgewählt ist aus der Gruppe bestehend aus H; Alkyl; Aryl; Alkyl-Aryl wie $CH_2$-Aryl, $C_2H_4$-Aryl und dergleichen; Vinyl; Cycloalkyl, insbesondere ein 3- bis 7- gliedriges Cycloalkyl; $CH_nF_{3-n}$ (n = 0-3); Oxacycloalkyl, insbesondere ein 3- bis 7- gliedriges Oxacycloalkyl; und Kombinationen davon, vorzugsweise ausgewählt ist aus der Gruppe bestehend aus H, Methyl, Ethyl, Phenyl und Benzyl; und besonders bevorzugt ausgewählt ist aus der Gruppe bestehend aus H, Methyl, Ethyl und Kombinationen davon;
Alkyl ausgewählt ist aus Kohlenwasserstoffen, auch verzweigten Isomeren, beispielsweise mit $C_{1-20}$ und vorzugsweise mit 1 bis 8 Kohlenstoffatomen; Aryl ausgewählt ist aus der Gruppe bestehend aus Phenyl, Naphthyl, Benzyl und deren Derivaten, vorzugsweise mit bis zu fünf Alkyl-, Alkoxy- oder Halogensubstituenten, besonders bevorzugt solche mit bis zu drei Substituenten, am meisten bevorzugt solche mit bis zu einem Substituenten; und vorzugsweise ausgewählt ist aus der Gruppe bestehend aus Phenyl- und Benzyl-Derivaten und Kombinationen davon;
PG eine Schutzgruppe ist, vorzugsweise ausgewählt aus der Gruppe bestehend aus Allyl, Methyl, t-Butyl (bevorzugt bei EWG), Benzyl, Silyl, Acyl und aktivierten Methylenderivaten wie Methoxymethyl, Alkoxyalkyl oder 2-Oxacycloalkyl; vorzugsweise - überwiegend für Alkohol und Aminfunktionen - ausgewählt aus der Gruppe bestehend aus Trimethylsilyl, Triethylsilyl, Dimethyl-tert-butylsilyl, Acetyl, Propionyl, Benzoyl, Tetrahydropyranyl; sowie aus Schutzgruppen, die benachbarte oder zweiwertige Gruppen ($PG_2$) unter Bildung 5-7-gliedriger Ringe gleichzeitig schützen, wie Succinyl, Phthalyl, Methylen, Acetonid; und/oder Kombinationen aller vorgenannten Schutzgruppen;
wobei besonders bevorzugt $B^1$ eine Doppelbindung ist; Nu H ist; E ausgewählt ist aus der Gruppe bestehend aus $CH_2OH$, CHO und $CO_2R$; und/oder X ausgewählt ist aus der Gruppe bestehend aus Cl, Br und OTs.

Insbesondere werden die Prenylverbindungen, vorzugsweise die Verbindungen der Formeln VIII, IX und/oder XIV durch C-C-Verknüpfung mittels nucleophiler Substitution mit 2-oxy-substituierten-1,3-di-aktivierten Methylenverbindungen der Formel X, bevorzugt 2-Acyloxy-1,3-dicarbonylverbindungen, besonders bevorzugt mit 2-Acyloxy-acetoacetaten, am meisten bevorzugt mit Verbindungen der Formel Xa, vorzugsweise in Gegenwart einer oder mehrerer Basen, gegebenenfalls unter Zusatz eines Lösemittels und/oder in Gegenwart von Modifikatoren, umgesetzt.

Die Reaktionstemperatur beträgt üblicherweise von -80˚C bis +180˚C, vorzugsweise von -30˚C bis +100˚C, und besonders bevorzugt von -5˚C bis +80˚C.

Geeignete Basen werden üblicherweise ausgewählt aus der Gruppe umfassend Alkalimetalle, hydridische Basen, Stickstoffbasen in neutraler oder negativ geladener Form, Alkoholate, Hydroxide, Carbonate, Hydrogencarbonate und Carbanionen; vorzugsweise umfassend hydridische Basen, Stickstoffbasen, negativ geladene Stickstoffbasen, Carbonate, Hydrogencarbonate; und besonders bevorzugt umfassend hydridische Basen, Carbonate und/oder Hydroxide der Alkali- und Erdalkalimetalle wie NaH, KH und LiH.

Geeignete Lösemittel werden z.B. ausgewählt aus der Gruppe umfassend Kohlenwasserstoffe, Alkane, Benzol und alkylierte Derivate davon, Ether, Dichlormethan, Chloroform, Tetrachlorkohlenstoff, chlorierte Aromaten, Alkohole, Ketone, Sulfoxide wie DMSO und Sulfolan, Carbonsäureamide, alkylierte Carbonsäureamide, Sulfolan, DMPU, Glymes, Alkyl- und Arylnitrile, Carbonsäureester, tertiäre Amine und/oder Mischungen davon; vorzugsweise umfassend Carbonsäureamide und alkylierte Carbonsäureamide, Ketone, Sulfoxide wie DMSO und Sulfolan, Alkohole, Alkyl- und/oder Arylnitrile; und besonders bevorzugt umfassend Carbonsäureamide, alkylierte Carbonsäureamide, Ketone, Sulfoxide, DMSO, Sulfolan und/oder Alkohole.

Ein weiterer Gegenstand der vorliegenden Erfindung sind Verfahren zur Synthese von Verbindungen mit der allgemeinen Formel VII mit EWG = H. Derartige Verfahren umfassen die Alkyldecarboxylierung oder Verseifung/Decarboxylierung von Verbindungen mit der allgemeinen Formel VII mit EWG = $CO_2R$ oder $CO_2PG$, bevorzugt nach der Krapcho-Methode, noch bevorzugter in Gegenwart von LiCl und DMSO beispielsweise bei erhöhter Temperatur, und besonders bevorzugt ausgehend von Verbindungen mit der allgemeinen Formel VII mit EWG = $CO_2tBu$ in Gegenwart geeignet starker Säuren, vorzugsweise flüchtiger und wasserfreier Säuren, besonders bevorzugt in Gegenwart von Trifluoressigsäure.

Ein weiterer Gegenstand der vorliegenden Erfindung sind Verfahren zur Synthese von Verbindungen mit der allgemeinen Formel XI. Derartige Verfahren umfassen die Umsetzung einer Verbindung mit der allgemeinen Formel XI mit X' = H mit einem Reagenz ausgewählt aus der Gruppe bestehend aus Halogenierungsreagenzien und elektrophilen Sauerstoffreagenzien in einem geeigneten Lösemittel gegebenenfalls unter Zusatz von sauren oder basischen Modifikatoren.

Vorzugsweise wird als Verbindung mit der allgemeinen Formel XI eine Verbindung mit der allgemeinen Formel XIa mit Y' = OMe eingesetzt.

Ein weiterer Gegenstand der vorliegenden Erfindung sind Verfahren zur Synthese von Verbindungen mit der allgemeinen Formel XII. Derartige Verfahren umfassen die Umsetzung einer Verbindung mit der allgemeinen Formel XIII mit einem Reagenz ausgewählt aus der Gruppe bestehend aus Halogenierungsreagenzien und elektrophilen Sauerstoffreagenzien in einem geeigneten Lösemittel gegebenenfalls unter Zusatz von sauren oder basischen Modifikatoren.

Geeignete Halogenierungsreagenzien sind beispielsweise elektrophile Halogenierungsmitiel, vorzugsweise ausgewählt aus der Gruppe bestehend aus elementaren Halogenen; Halogenimidderivaten; Schwefel(oxy)halogeniden wie Sulfurylchlorid; Perhaloalkanen und Ammoniumperhalogeniden; und besonders bevorzugt ausgewählt aus der Gruppe bestehend aus tertiären oder quarternären Ammoniumperhalogeniden wie Pyridiniumbromidperbromid, Trimethylphenylammoniumbromidperbromid und/oder polymergebundenen Varianten der vorgenannten Reagenzien.

Die Reaktionstemperatur bei den letztgenannten Verfahren beträgt üblicherweise von -80 bis +160˚C; vorzugsweise von -30 bis +65˚C; und besonders bevorzugt von -5 bis +10˚C beträgt.

Geeignete Lösemittel sind üblicherweise ausgewählt aus der Gruppe bestehend aus etherischen und halogenierten Lösemitteln; sind vorzugsweise ausgewählt aus der Gruppe bestehend aus Diethylether, THF, Chloroform, Dichlormethan, flüssigen Carbonsäureamiden und -estern; und sind besonders bevorzugt ausgewählt aus der Gruppe bestehend aus DMF, DMA, Essigester, flüssigen Sulfoxiden wie DMSO und Sulfolan.

Ein weiterer Gegenstand der vorliegenden Erfindung sind Verfahren zur Synthese von Verbindungen mit den allgemeinen Formeln XVa und/oder XVb. Derartige Verfahren umfassen die Umsetzung von Verbindungen VII mit Verbindungen des Typs XI bzw. XIa; XII; und/oder XIII. Solche Verbindungen XV sind bevorzugt, bei denen alle Kohlenstoffatome des Epothilon-Macrocyclus XVI vorhanden sind. Besonders bevorzugt sind solche Verbindungen XV, bei denen die Bindungen C2-3 wie in XVa; O15-C1 wie z. B. in XVa mit Y-CO-CR'$_2$X = OH oder Y" = $CH_2CO_2H$; und/oder C6-7 wie in XVb noch offen sind. Am meisten bevorzugt sind solche Verbindungen XV, die ein Halogen an C2 und/oder C6 tragen.

Ein weiterer Gegenstand der vorliegenden Erfindung sind Verfahren zur Synthese von Verbindungen mit der allgemeinen Formel XVa, umfassend die Erzeugung einer C6-C7-Verknüpfung durch Umsetzung von Verbindungen der Formel VII mit solchen der allgemeinen Formeln XI, XII und/oder XIII; vorzugsweise mit solchen der allgemeinen Formeln XI und/oder XII mit X' = Cl, Br oder I; in Gegenwart von Basen und/oder in Gegenwart eines oder mehrerer Metalle und/oder Metallsalze, gegebenenfalls unter Zusatz von Lösemitteln, Katalysatoren und/oder Modifikatoren.

Bei diesen Verfahren beträgt die Reaktionstemperatur üblicherweise von -80˚C bis +140˚C; vorzugsweise oberhalb von 0˚ bis 65˚C, und besonders bevorzugt von 15˚C bis 35˚C.

Geeignete Metalle werden üblicherweise ausgewählt aus der Gruppe bestehend aus Li, Mg, Zn, In, Mn, Fe (jeweils auch in aktivierter Form); und geeigneten Metallsalzen, vorzugsweise anorganischen oder organischen Salzen, Komplexen und/oder Organometallverbindungen der Metallionen Ti(II), Ti(III), Cr(II), Sm(II), Co(I), V(II) und Fe(II), besonders bevorzugt Halogeniden, Sulfaten, Sulfonaten, Alkanoaten, Cyclopentadienylaten und Phenylaten; festphasen- oder polymergebundenen Metallsalzen; und Kombinationen davon; und werden vorzugsweise ausgewählt aus der Gruppe bestehend aus Zink, Titan(II) und Cr(II)-Verbindungen, insbesondere als Chlorid, Bromid, Acetat, Sulfat, in situ erzeugt oder polymergebunden.

Geeignete Katalysatoren werden ausgewählt aus der Gruppe bestehend aus Iodiden; nicht reduzierbaren Lewissäuren, z.B. Lithiumsalzen, Aluminiumchlorid, Bortrifluorid, und/oder in situ bei der Bildung reduzierender Metallsalze mit $LiAlH_4$ entstehenden Lewissäuren; Nickel(II)salzen; nukleophilen und/oder redoxaktiven Metallkomplexen, z.B. Vitamin B12 und vergleichbaren synthetischen Co-Komplexen. Die geeigneten Katalysatoren liegen ferner bevorzugt in amidischen Lösungsmitteln und/oder Sulfolan vor; desweiteren bevorzugt bis zu 33 mol% des wasserfreien Katalysators, vorzugsweise 0.01 - 5 mol% reaktive Lewissäuren, gegebenenfalls mit dem geeigneten Meatllsalz, bevorzugt vor der Zugabe des Lösemittels, vermischt, zugesetzt werden.

Die Modifikatoren werden vorzugsweise ausgewählt aus der Gruppe bestehend aus Iodiden (Metalliodiden MI): nicht reduzierbaren gebräuchlichen Lewissäuren, z.B. Lithiumsalzen, Aluminiumchlorid, Bortrifluorid; komplexierenden Liganden, insbesondere auch chiralen Liganden, besonders bevorzugt bidentaten Liganden und anderen üblichen Liganden; und Kombinationen davon; und sind ebenfalls vorzugsweise im wasserfreien Zustand. Besonders bevorzugt werden die Modifikatoren aus der Gruppe bestehend aus wasserfreiem Lithiumiodid, Natriumiodid und Aluminimchlorid.

Geeignete Lösemittel werden üblicherweise ausgewählt aus der Gruppe bestehend aus Kohlenwasserstoffen wie Alkanen, Benzol und alkylierten Derivaten; Ethern; Dichlormethan; Chloroform; chlorierten Aromaten; sec./tert.-Alkoholen; Ketonen; Dimethylsulfoxid; Carbonsäureamiden; alkylierten Carbonsäureamiden; Sulfolan; DMPU; Glymes; Alkyl- und Arylnitrilen; Carbonsäureestern, tertiären Aminen und Mischungen davon; und werden vorzugsweise ausgewählt aus der Gruppe bestehend aus Methyl-tert.-butylether, Diethylether, Tetrahydrofuran, Glymes, Sulfolan, DMSO, Ketone bis C5, Dimethylformamid und -acetamid, Acetonitril und Mischungen davon; und sind besonders bevorzugt möglichst wasserfreie Lösemittel.

Vorzugsweise umfassen die erfindungsgemäßen Verfahren die Umsetzung von einem oder mehreren Metallsalzen durch Reduktion mit einem Reduktionsmittel in einer für das Verfahren verwendbaren Oxidationsstufe; wobei die Umsetzung vorzugsweise in situ oder der Synthese der Verbindung mit der allgemeinen Formel XVa vorhergehend durchgeführt wird.

Geeignete Reduktionsmittel bzw. -verfahren werden beispielsweise ausgewählt aus der Gruppe bestehend

aus elektrochemischen Verfahren, Lithiumaluminiumhydrid und vergleichbaren Hydriden, metallischem Eisen oder Mangan in ihren verschiedenen Formen, und werden vorzugsweise ausgewählt aus der Gruppe bestehend aus Lithiumaluminiumhydrid und Zink.

Ein weiterer Gegenstand der vorliegenden Erfindung betrifft Verfahren zur Synthese einer Verbindung mit der allgemeinen Formel XVb, umfassend die Erzeugung einer O15-C1-Verknüpfung durch Umsetzung von Verbindungen mit der allgemeinen Formel VII mit solchen der Formel XII oder XIII, besonders bevorzugt XII mit X' = Cl, Br oder I, in Gegenwart von Veresterungs-, Umesterungskatalysatoren, und/oder Kupplungsreagenzien, gegebenenfalls unter Zusatz von Lösemitteln.

Die Reaktionstemperatur bei diesem erfindungsgemäßen Verfahren beträgt üblicherweise von -50˚C bis +160˚C, vorzugsweise oberhalb von 0˚ bis 100˚C, und besonders bevorzugt von 15˚C bis 55˚C.

Veresterungs- und/oder Umesterungskatalysatoren werden z.B. ausgewählt aus der Gruppe bestehend aus für diese Zwecke üblichen Basen, Säuren und Metallalkoholaten wie Titantetraalkoholat.

Zur Veresterung freier Säuren werden insbesondere Kupplungsreagenzien, vorzugsweise ausgewählt aus der Gruppe umfassend EDCI, DCC/DMAP, und/oder Verfahren wie die Yamaguchi-Veresterung eingesetzt.

Geeignete Lösemittel werden bei diesem Verfahren üblicherweise ausgewählt aus der Gruppe bestehend aus Kohlenwasserstoffen wie Alkanen, Benzol und alkylierten Derivaten; Ethern; Dichlormethan; Chloroform; chlorierten Aromaten; Carbonsäureamiden; alkylierte Carbonsäureamiden; Sulfolan; DMPU; Glymes; Alkyl- und Arylnitrilen; Carbonsäureestern; tertiären Aminen; und Mischungen davon, und werden vorzugsweise ausgewählt aus der Gruppe bestehend aus Methyl-tert.-butylether, Diethylether, Tetrahydrofuran, Glymes, Sulfolan, Chloroform, Dichlormethan und/oder Mischungen davon; und werden besonders bevorzugt ausgewählt aus der Gruppe bestehend aus Chloroform und Dichlormethan.

Vorzugsweise sind die Lösemittel möglichst wasserfrei.

Ein weiterer Gegenstand der vorliegenden Erfindung betrifft Verfahren zur Synthese einer Verbindung mit der allgemeinen Formel VII mit $E \neq CH_3$, vorzugsweise mit E = $CN_2OH$, CHO und/oder $CO_2H$, bei denen man eine geschützte Verbindung mit der allgemeinen Formel VII mit E = $CH_3$, bevorzugt VIIa mit E = $CH_3$, in Allylstellung oxidiert und/oder vorhandene funktionelle Gruppen oxidiert. Die Oxidation erfolgt vorzugsweise mit Selendioxid.

Ein weiterer Gegenstand der vorliegenden Erfindung betrifft Verfahren zur Synthese einer Verbindung mit der allgemeinen Formel VIIa und/oder XVb, vorzugsweise mit E = $CH_2OH$, CHO und/oder $CO_2H$, bei denen man eine vorzugsweise geschützte Verbindung mit der allgemeinen Formel VII und/oder XVb, beispielsweise mit E = $CH_3$, oxidiert, vorzugsweise an Position C7 bzw. in Allylstellung bzw. an anderen funktionellen Gruppen, wobei als Oxidationsmittel vorzugsweise Selen-(IV)-Verbindungen, besonders bevorzugt Selendioxid, und/oder $C_6F_5SO_2H$, eingesetzt werden. Die Oxidationsmittel werden vorzugsweise katalytisch eingesetzt. Alternativ kann die Oxidation bakteriell oder enzymatisch mit Zellsystemen erfolgen, wobei die Oxidation mit $SeO_2$ besonders bevorzugt ist.

Ein weiterer Gegenstand der vorliegenden Erfindung betrifft Verfahren zur Synthese einer Verbindung mit den allgemeinen Formeln VII und/oder XV, umfassend die Addition eines Nucleophils oder Elektrophils an eine oder mehrere bzw. die Reduzierung, Epoxidierung oder Cyclopropanierung einer oder mehrerer Doppelbindungen einer Verbindung mit der allgemeinen Formel VII und/oder Formel XV.

Vorzugsweise umfaßt das erfindungsgemäßen Verfahren die Reduzierung der C8-C9-Doppelbindung; die Epoxidierung der C12-C13-Doppelbindung; und/oder die Addition eines Nucleophils an C9.

Ebenfalls bevorzugt umfaßt das erfindungsgemäße Verfahren die asymmetrische und/oder katalytische Hydrierung der C8-C9-Doppelbindung.

Ein weiterer Aspekt der vorliegenden Erfindung betrifft Verfahren zur Synthese von Epothilonmacrocyclen und Derivaten mit der allgemeinen Formel XVI

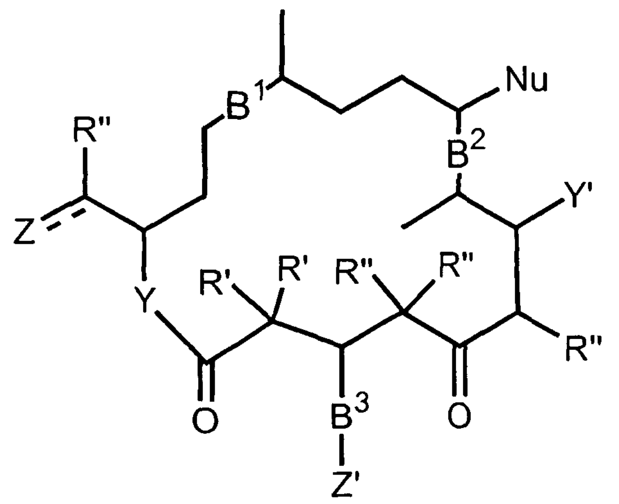

**XVI**

worin:

$B^1$, $B^2$, $B^3$ ausgewählt werden aus der Gruppe bestehend aus Einfachbindungen; Doppelbindungen als *E*-(*trans*)-Form, *Z*-(*cis*)-Form oder *E*/*Z*-Gemisch; Epoxidringen als *E*-(*trans*)-Form, *Z*-(*cis*)-Form oder *E*/*Z*-Gemisch; Cyclopropanringen als *E*-(*trans*)-Form, *Z*-(*cis*)-Form oder *E*/*Z*-Gemisch; und/oder Kombinationen davon; vorzugsweise ausgewählt werden aus der Gruppe bestehend aus Einfach- und

Doppelbindungen; und besonders bevorzugt ausgewählt werden aus der Gruppe bestehend aus $B^1$ als Z-Doppelbindung oder Epoxid und $B^2$ und $B^3$ als Einfachbindung;

R ausgewählt wird aus der Gruppe bestehend aus H; Alkyl; Aryl; Alkyl-Aryl wie $CH_2$-Aryl, $C_2H_4$-Aryl und dergleichen; Vinyl; Cycloalkyl, insbesondere ein 3- bis 7-gliedriges Cycloalkyl; $CH_nF_{3-n}$ mit n = 0 bis 3; Oxacycloalkyl, insbsondere ein 3-7-gliedriges Oxacycloalkyl; und/oder Kombinationen davon; vorzugsweise ausgewählt wird aus der Gruppe bestehend aus H, Methyl, Ethyl, Phenyl, Benzyl; und besonders bevorzugt ausgewählt wird aus der Gruppe bestehend aus H, Methyl, Ethyl und Kombinationen davon;

R' aus derselben Gruppe ausgewählt wird wie R und ganz besonders bevorzugt H ist; R'' aus derselben Gruppe ausgewählt wird wie R und ganz besonders bevorzugt Methyl ist;

Y ausgewählt wird aus der Gruppe bestehend aus S, NH, N-PG, NR und O; vorzugsweise ausgewählt wird aus der Gruppe bestehend aus NH, N-PG, NR und O, und besonders bevorzugt O ist;

Y' ausgewählt wird aus der Gruppe bestehend aus H, OH, OR, O-PG, $NH_2$, $NR_2$, $N(PG)_2$, SR und SH; vorzugsweise O-PG und/oder OH ist;

Nu ausgewählt wird aus der Gruppe bestehend aus R, O-PG, OR, $N(PG)_2$, $NR_2$, S-PG, SR, SeR, CN, $N_3$, Aryl und Heteroaryl; vorzugsweise ausgewählt wird aus der Gruppe bestehend aus R, O-PG, OR, $N(PG)_2$ und $NR_2$, und besonders bevorzugt H ist;

Z ausgewählt wird aus der Gruppe bestehend aus -OH, -O-PG, -OR, =O, =N-Nu, =CH-Hetaryl, =CH-Aryl und $=PR_3$, wobei alle vorgenannten doppelgebunden Gruppen Z in (*E*)-form, (*Z*)-Form oder als (*E*/*Z*)-Gemisch vorliegen können; vorzugsweise =CH-Hetaryl ist; und besonders bevorzugt ausgewählt wird aus der Gruppe bestehend aus =O, (*E*)-(2-Methylthiazol-4-yl)-CH= und (*E*)-(2-Methyloxazol-4-yl)-CH=;

Z' ausgewählt wird aus der Gruppe bestehend aus O, OH, OR, O-PG, $N(H)_{1-2}$, $N(R)_{1-2}$, $N(PG)_{1-2}$, SR, S-PG und R; vorzugsweise O, O-PG und/oder OR ist;

$B^3$ ausgewählt wird aus der Gruppe bestehend aus Einfach- und Doppelbindungen als *E*-(*trans*)-Form, *Z*-(*cis*)-Form oder *E*/*Z*-Gemisch; vorzugsweise ausgewählt wird aus der Gruppe bestehend aus Einfach- und Doppelbindungen zu Heteroatomen wie O, S und N; und besonders bevorzugt eine einfache Bindung zu O-PG und/oder OH ist. PG eine Schutzgruppe ist und vorzugsweise ausgewählt wird aus der Gruppe bestehend aus Allyl, Methyl, t-Butyl (bevorzugt bei EWG), Benzyl, Silyl, Acyl und aktivierten Methylenderivaten wie Methoxymethyl, Alkoxyalkyl oder 2-Oxacycloalkyl; vorzugsweise - überwiegend für Alkohol und Aminfunktionen - ausgewählt wird aus der Gruppe bestehend aus Trimethylsilyl, Triethylsilyl, Dimethyl-tert-butylsilyl, Acetyl, Propionyl, Benzoyl, Tetrahydropyranyl, sowie Schutzgruppen, die benachbarte oder zweiwertige Gruppen ($PG_2$) unter Bildung 5-bis 7-gliedriger Ringe gleichzeitig schützen, wie Succinyl, Phthalyl, Methylen, Ethylen, Propylen, 2,2-Dimethylpropa-1,3-diyl, Acetonid; und/oder Kombinationen aller vorgenannten Schutzgruppen.

Alkyl ausgewählt wird aus der Gruppe bestehend aus Kohlenwasserstoffen, auch verzweigten Isomeren, vorzugsweise mit $C_{1-20}$, besonders bevorzugt mit 1 bis 8 Kohlenstoffatomen;
Aryl ausgewählt wird aus der Gruppe bestehend aus Phenyl, Naphthyl, Benzyl und deren Derivaten, vorzugsweise mit bis zu fünf Alkyl-, Alkoxy- und/oder Halogensubstituenten, bevorzugt solche mit bis zu drei Substituenten, besonders bevorzugt mit bis zu einem Substituenten; vorzugsweise ausgewählt wird aus der Gruppe bestehend aus Phenyl- und Benzyl-Derivaten; und Kombinationen davon; Hetaryl/Heteroaryl ausgewählt wird aus der Gruppe bestehend aus fünf- und sechsgliedrigen Heteroaromaten mit einem oder mehreren O-, S- und N-Atomen und deren Derivaten mit bis zu vier Alkyl-, Alkoxy- und/oder Halogensubstituenten, bevorzugt solche mit bis zu zwei Substituenten, besonders bevorzugt mit bis zu einem Substituenten; vorzugsweise ausgewählt wird aus der Gruppe bestehend aus Oxazol-, Thiazol- und Pyrimidin-Derivaten; und besonders bevorzugt ein Alkylthiazolderivat ist; und Kombinationen davon;
worin besonders bevorzugt Z = O, (E)-(2-Methylthiazol-4-yl)-CH=, (E)-(2-Methyloxazol-4-yl)-CH=; R' = H; R" = Me; Y', Z' = O-PG, OH und/oder Y = O ist,

Die Synthese von Epothilonmacrocyclen und Derivaten mit der allgemeinen Formel XVI erfolgt insbesondere durch ringschließende Lactonisierung und/oder Reformatsky-Typ-Reaktionen und/oder Macroaldolisierungen der offenkettigen Verbindung mit der allgemeinen Formel XVa und/oder XVb, wobei Verknüpfungen an C2-C3 und/oder C6-C7 jeweils bevorzugt mit EWG = H; E = CHO, $CO_2R$, $CO_2PG$; Y", $B^3$-Z' = OH oder Y"+$B^3$-Z' = (=O), bevorzugt sind.

Die Alternative Y", $B^3$-Z' = OH stellt ein Acetal an Position C3 dar, während die Alternative Y"+$B^3$-Z' = (=O) für eine entsprechende Aldehydfunktion an Position C3 steht.

Als Reformatsky-Typ-Reaktionen werden im Rahmen der vorliegenden Erfindung Umsetzungen von Halogencarbonsäureestern bzw. Halogenketonen mit Aldehyden oder Ketonen in Gegenwart von Metallen bezeichnet. Analog zu Grignard-Verbindungen werden bei diesem Reaktionstyp metallorganische Reaktionsstufen durchlaufen, die eine C-C-Verknüpfung insbesondere an C2-C3 und/oder C6-C7 ermöglichen.

Bevorzugte Reformatsky-Typ-Reaktionen im Rahmen der vorliegenden Erfindung umfassen die Umsetzung von Verbindungen XVa und/oder XVb mit X = Cl, Br, I mit Chrom(II)salzen, Indium und/oder Zink und/oder deren Salzen.

Bei einer besonders bevorzugten Ausführungsform des erfindungsgemäßen Verfahrens werden Verbindungen XVa und/oder XVb in Verbindungen XVa und/oder XVb mit X = M überführt, wobei M ausgewählt wird aus der Gruppe bestehend aus ZnX, MgX, Li, $CrX_2$, $SmX_2$ und $InX_2$; und X vorzugsweise Halogenid ist. Anschließend erfolgt die Ringschließung insbesondere durch elektrophile C2- bzw. C7-Bausteine.

Eine speziellen Ausführungsform eines Verfahren zur Makrocyclensynthese umfaßt die folgenden Schritte:

a) Überführung von Verbindungen XI und XIa in Verbindungen XI mit X' = M überführt werden, wobei M ausgewählt wird aus der Gruppe bestehend aus ZnX, MgX, Li, $CrX_2$, $SmX_2$ und $InX_2$; und X vorzugsweise Halogenid ist; und
b) Umsetzung der Verbindungen aus Schritt a) mit elektrophilen C-7-Bausteinen, z.B. des Typs VII mit geeigneten Substituenten E, vorzugsweise mit E = CHO.

Selbstverständlich lassen sich die erfindungsgemäßen Synthesebausteine mit den allgemeinen Formeln VII bis XVI sowie davon abgeleitete Verbindungen auf analoge Weise wie die vorstehend beschriebenen erfindungsgemäßen $\alpha$-Hydroxyketone in nicht racemischer Form an der der $\alpha$-Hydroxyposition der Verbindung mit der allgemeinen Formel I entsprechenden Position darstellen. Die Übertragung der am Beispiel von Verbindungen mit den allgemeinen Formeln I bis VI dargestellten und in den Beispielen veranschaulichten Prinzipien zur Herstellung von entsprechend nicht racemischen Synthesebausteinen mit den allgemeinen Formeln VII bis XVI ist dem Fachmann ohne weiteres möglich.

Die Erfindung beschränkt sich in ihrer Ausführung nicht auf die vorstehend genannten bevorzugten Ausführungsbeispiele. Vielmehr ist eine Vielzahl von Varianten denkbar, welche von den dargestellten Lösungen auch bei grundsätzlich anders gearteten Ausführungen Gebrauch machen.

Die vorliegende Erfindung wird im folgenden anhand von weiteren Beispielen veranschaulicht, die jedoch keineswegs als einschränkend auszulegen sind.

## Beispiele

Durch in Formeln eingezeichnete Stereozentren wird keine Aussage bezüglich der absoluten Konfiguration oder optischer Reinheit begründet, es handelt sich in diesen Fällen lediglich um nicht-racemische (optisch aktive) Produkte.

Ferner werden in den Beispielen die Abkürzungen gemäß Tabelle 1 verwendet:

Tabelle 1

| | |
|---|---|
| Ac | Acetyl |

(fortgesetzt)

| | |
|---|---|
| AYS | Lipase aus *Candida rugosa* |
| BSDR | *Bacillus stearothermophilus* Diacetylreduktase |
| CAA/CAL-A | Lipase Typ A aus *Candida antartica* |
| CAB/CAL-B | Lipase Typ B aus *Candida antartica* |
| CD | Cyclodextrin |
| CPGC | Chiralphasengaschromatographie |
| DMF | *N,N*-Dimethylformamid |
| E | Enantioselektivität (Enantiomeric ratio) nach Chen, C. S.; Fujimoto, Y.; Girdaukas, G.; Sih, C. J. *J. Am. Chem. Soc.* **1982,** *104,* 7294-7299. |
| e.e. | Enantiomerenüberschuß (enantiomeric excess) |
| LDH | Lactatdehydrogenase |
| MJ | Lipase aus *Mucor javanicus* |
| MPA | Methoxyphenylessigsäure |
| NAD(H) | (Reduziertes) Nicotinamidadenindinukleotid |
| NADP(H) | (Reduziertes) Nicotinamidadenindinukleotid-phosphat |
| n.d. | nicht bestimmt (not determined) |
| NMR | Kernspinresonanaspektroskopie (Nuclear magnetic resonance spectroscopy) |
| O-PG | O-Schutzgruppe |
| PPL | Porcine pancreas lipase |
| PS | Lipase aus *Pseudomonas cepacia* |
| *p*TsOH | *para*-Toluolsulfonsäure |
| TBDMS | siehe TBS |
| TBS(O) | *tert*-Butyl-di-methyl-silyl(-ether) |
| *t*-Bu | *tert*-Butyl |
| TMS | Tetramethylsilan |

**Beispiel 1**

Teil A - Synthese von racemischen O-Acylacyloinen und deren Vorstufen

***tert*-Butyl-2-acetoxy-2-acetyl-4-hexenoat**

3,24 g (15,0 mmol) *tert*-Butyl-2-acetoxyacetoacetat werden zu einer Suspension aus 660 mg (16,5 mmol) Natriumhydrid in 30 ml DMF bei 0˚C gegeben. nach Beendigung der Gasentwicklung wurden 2,00 g (15,0 mmol) 1-Brom-2-butin bei 0˚C zugegeben. Die orange Lösung wurde 40 min gerührt und dann in 15 min auf Zimmertemperatur erwärmt. Nach der Zugabe von 120 ml Diethylether wurde einige Male mit 35 ml Wasser gewaschen und zuletzt mit konz. Kochsalzlösung. Die organische Phase wurde mit $Na_2SO_4$ getrocknet, filtriert und *in vacuo* vom Lösemittel befreit

| | |
|---|---|
| Ausbeute | 3,83 g (14,3 mmol, 95%) |
| $R_f$-Wert | ca. 0,57 mit Ethylacetat/Hexan = 1:4 |

**3-Acetoxy-5-heptin-2-on**

1,61 g (6,00 mmol) *tert*-Butyl-2-acetoxy-2-acetyl-4-hexenoat, 114 mg (0,60 mmol) *para*-Toluolsulfonsäuremonohydrat und 20 ml Benzol wurden bei 78˚C 2 h gerührt. Nach Ende der Reaktion (Ende der Gasentwicklung, Verfärbung) wurde das Rohprodukt auf Kieselgel gegeben mit Ethylacetat/Hexan = 1:4 eluiert. Die Fraktion mit dem gewünschten

Produkt wird *in vacuo* eingedampft.

**Ausbeute** 922 mg (5,48 mmol, 91 %)

**tert-Butyl-2-acetoxy-2-acetyl-4-hexenoat**

630 mg (2,3 mmol) *tert*-Butyl-2-acetoxy-2-acetyl-4-hexenoat werden zu einer Suspension von 120 mg Lindlar-Katalysator in 50 ml Ethylacetat gegeben und zweimal evakuiert und unter Wasserstoffatmosphäre mit leichtem Überdruck gesetzt. Nach 24 h Rühren wird durch Celite® mit Ethylacetat filtriert, mit gesättigter Natriumhydrogencarbonatlösung gewaschen, mit $Na_2SO_4$ getrocknet, gefiltert und *in vacuo* vom Lösungsmittel befreit.

**Ausbeute** 628 mg (2,32 mmol, 99%)

**3-Acetoxy-5-hepten-2-on**

433 mg (1,60 mmol) *tert*-Butyl-2-acetoxy-2-acetyl-4-hexenoat, 30 mg (0.16 mmol) *para*-Toluolsulfonsäuremonohydrat und 6 ml Benzol wurden bei 78˚C 4 h gerührt. Nach Ende der Reaktion (Ende der Gasentwicklung, Verfärbung) wurde das Rohprodukt auf Kieselgel gegeben und mit Ethylacetat/Hexan = 1:4 eluiert. Die Fraktion mit dem gewünschten Produkt wird *in vacuo* eingedampft.

**Ausbeute** 205 mg (1,20 mmol, 75%)
**$R_f$-Wert** ca. 0,50 mit Ethylacetat/Hexan = 1:4

**3-Acetoxy-6-methyl-heptan-2-on**

300 mg (1,63 mmol) 3-Acetoxy-5-hepten-2-on und 60 mg 5% Palladium auf Kohle wurden bei Raumtemperatur in trockenem Ethanol verrührt. Nach dreimaligem Evakuieren und Begasen mit Wasserstoff wird das Gas noch 25 min. langsam durch die Suspension geleitet. Nach Filtrieren durch Celite® und Trocknen über $Na_2SO_4$ wird filtriert und *in vacuo* das Lösemittel entfernt.

**Ausbeute** 302 mg (1,62 mmol, 99%)

<u>Teil B - Diastereoselektive Synthesen und Diastereomerentrennungen von Acyloinderivaten und Darstellung von nicht-racemischen Acyloinen, Acyloinderivaten und Epothilonnordhälftebausteinen.</u>

**(*4S*,*5R*,*2'-nicht-rac*)-1,5-Dimethyl-4-phenyl-3-(2'-benzyloxy-5',9'-dimethyldeca-4'(*Z*),8'-dienoyl)-imidazotidin-2-on:**

Zu einer Lösung von Diisopropylamin (2,65 ml, 1,90 g, 18,8 mmol) in THF (25 ml) wird bei 0˚C eine *n*-Butyllithium-Lösung (1,88 ml, 10 M in Hexan, 18,78 mmol) getropft. Die so erhaltene LDA-Lösung wurde bei -78˚C langsam zu einer Lösung von (*4S,5R*)-1,5-Dimethyl-4-phenyl-3-(benzyloxyacetyl)-imidazolidin-2-on (5,30 g, 15,7 mmol) in THF (25 ml) getropft. Nach einstündigem Rühren bei -78˚C wurde Nerylbromid (3,40 g, 15,7 mmol) tropfenweise zugegeben. Nach dreistündigem Rühren bei -78˚C wurde das Kühlbad entfernt und 15 Minuten bei Umgebungstemperatur gerührt. Nach Löschen mit einer gesättigten $NH_4Cl$ Lösung (25 ml) wurde das organische Lösemittel im Vakuum entfernt und die zurückbleibende wässrige Phase wurde mit demi. Wasser (25 ml) verdünnt. Es wurde dreimal mit 75 ml $Et_2O$ extrahiert und die vereinigten organischen Phasen wurden über $Na_2SO_4$ getrocknet. Danach wurde vom Trockenmittel abfiltriert und das Lösemittel im Vakuum entfernt.

Das Produkt wurde mittels Chromatographie an Kieselgel (Säulendimension: 30 x 3 cm, EtOAc/Petrolether = 1:2) gereinigt.

Ausbeute: 3,64 g (7,67 mmol, 49 %)

**Nicht-racemisches 2-Benzyloxy-*N*-methoxy-*N,5*,9-trimethyldeca-4(*Z*),8-dienoylamid:**

Trimethylaluminium Lösung (4,50 ml, 2 M in Toluol, 9,00 mmol) wurde bei 0˚C zu einer Suspension von *N,*

*O*-Dimethylhydroxylaminhydrochlorid (878 mg, 9,00 mmol) in $CH_2Cl_2$ (12 ml) langsam zugetropft. Das Gemisch wurde 15 Minuten bei Umgebungstemperatur gerührt und danach auf-10°C abgekühlt. Jetzt wurde eine Lösung von (*4S,5R, 2'-nicht-rac*)-1,5-dimethyl-4-phenyl-3-(2'-benzyloxy-5',9'-dimethyldeca-4'(*Z*),8'-dienoyl)imidazolidin-2-on (s.o., 1.42 g, 3.00 mmol) in $CH_2Cl_2$ (12 ml) langsam zugetropft. Es wurde eine Stunde bei -10°C, zwei Stunden bei 0°C und 30 Minuten bei Umgebungstemperatur gerührt. Die Reaktionsmischung wurde schließlich in ein Gemisch von 0.5 N HCl (100 ml) und $CH_2Cl_2$ (50 ml) geschüttet und für 5 Minuten intensiv geschüttelt. Die Phasen wurden getrennt und die wässrige Phase zweimal mit $CH_2Cl_2$ (2 x 25 ml) extrahiert. Die vereinigten organischen Phasen wurden mit 0.5 N HCl (60 ml) und 1 M Phosphatpuffer (60 ml, pH = 7,0) gewaschen und anschließend über $Na_2SO_4$ getrocknet. Danach wurde vom Trockenmittel abfiltriert und das Lösemittel im Vakuum entfernt.

Das Produkt wurde mittels Chromatographie an Kieselgel (Säulendimension: 30 x 3 cm, EtOAc/Petrolether = 1:2) gereinigt.

Ausbeute: 779 mg (2,25 mmol, 75 %)

**Nichtracemisches 3-Benzyloxy-6,10-dimethylundeca-5(*Z*),9-dien-2-on:**

Methyllithium-Lithiumbromid Komplex (1,87 ml, 1,5 M in THF, 2,80 mmol) wurde bei -78°C zu einer Lösung von nichtracemischen 2-Benzyloxy-N-methoxy-N,5,9-trimethyldeca-4(Z),8-dienoylamid (s.o., 345 mg, 1,00 mmol) in $CH_2Cl_2$ (15 ml) getropft. Es wurde 40 Minuten bei -78°C gerührt, anschließend wurde das Reaktionsgemisch über eine Edelstahlkanüle in eine auf 0°C gekühlte und intensiv gerührte Mischung aus gesättigter $NH_4Cl$ Lösung (20 ml), 14 ml Hexan und 7 ml $CH_2Cl_2$ gepumpt. Das Gemisch wurde nach Auftauen auf Umgebungstemperatur mit gesättigter NaCl-Lösung (50 ml) sowie mit einem 3:1 Hexan/$CH_2Cl_2$ Gemisch (50 ml) verdünnt. Es wurde nochmals intensiv geschüttelt und nach Phasentrennung wurde die wässrige Phase nochmals mit $CH_2Cl_2$ (30 ml) extrahiert. Die vereinigten organischen Phasen wurden mit gesättigter NaCl-Lösung (150 ml) gewaschen und über $Na_2SO_4$ getrocknet. Danach wurde vom Trockenmittel abfiltriert und das Lösemittel im Vakuum entfernt. Das Produkt konnte in hoher Reinheit erhalten werden.

Ausbeute: 308 mg (1,03 mmol, nahezu quantitativ)

$[\alpha]_D^{25}$ (c = 1, $CHCl_3$) = -18,0°.

**11-(*tert*-Butyl-dimethyl-silanyloxy)-3-hydroxy-6,10-dimethyl-undec-5-en-2-on**

Zu 1,94 g (5,05 mmol) 3-Acetoxy-11-(*tert*-butyl-dimethylsilyloxy)-6,10-dimethyl-5-undecen-2-on in 20.0 ml Methanol werden 400 µl gesättigte Kaliumcarbonatlösung gegeben. Nach Beendigung der Reaktion (genaue DC-Kontrolle, z. B. ca. 10 min. bei Raumtemperatur) werden 30 ml NaCl-Lösung zugesetzt und fünf mal mit 30 ml Diethylether extrahiert. Die vereinten organischen Phasen werden nochmals mit NaCl-Lösung gewaschen und über Natriumsulfat getrocknet, das Lösemittel dann im Vakuum entfernt, und der Rückstand an Kieselgel chromatographiert.

Ausbeute 1,68 g (4,92 mmol, 97%)
$R_f$- Wert 0,42
$C_{19}H_{38}O_3Si$ (342,59)

**(*R*)-Methoxy-phenyl-essigsäure [(1*R*)-1-acetyl-9-(*tert*-butyl-dimethyl-silanyloxy)-4,8-dimethyl-non-3-enyl] ester** und

**(*R*)-Methoxy-phenyl-essigsäure [(1*S*)-1-acetyl-9-(*tert*-butyl-dimethyl-silanyloxy)-4,8-dimethyl-non-3-enyl] ester**

Zu 50 mg (0,146 mmol) 11-(tert-Butyl-dimethyl-silanyloxy)-3-hydroxy-6,10-dimethyl-undec-5-en-2-on, 27 mg (0,161 mmol) (*R*)-1-Methoxy-phenyl-essigsäure und 4 mg (0,029 mmol) 4,4'-Dimethylaminopyridin (DMAP) in 1.0 ml $CH_2Cl_2$werden 56 mg (0,292 mmol) des Kupplungsreagenzes EDCI gegeben und 18 h bei Zimmertemperatur gerührt. Nach Zugabe von 5 ml Diethylether wird zweimal mit 2 ml Wasser 2 ml NaCl-Lösung extrahiert, die organische Phase über $Na_2SO_4$ getrocknet und Lösemittel *in vacuo* abdestilliert. Das O-Methylmandelat (102 mg) enthält zwei Diastereomere, die sich chromatographisch trennen lassen, an Kieselgel mit Säule 1,0×20,0 cm, Diethylether/Petrolether = 2:9.

Gesamtausbeute: 53 mg (0,108 mmol, 74%)
$C_{28}H_{46}O_5Si$ (490,75)

Fraktion I

18 mg (0,037 mmol, 25%)
$R_f$ - Wert 0.31 (Diethylether/Petrolether =1:4)
Drehwert = -19,1˚

Fraktion II

Ausbeute 23 mg (0,047 mmol, 32%)
$R_f$-Wert ca.0,22 (Diethylether/Petrolether = 1:4)
Drehwert = -23,9˚
$C_{28}H_{46}O_5Si$ (490,75)

**Optisch aktives 11-(*tert*-Butyl-dimethyl-silanyloxy)-3-hydroxy-6,10-dimethyl-undec-5-en-2-on**

Milde basische Hydrolyse (s.u.) eines der diastereomeren Methoxy-phenylessigsäure [(1-acetyl-9-(*tert*-butyl-dimethyl-silanyloxy)-4,8-dimethyl-non-3-enyl]ester liefert nichtracemisches Produkt.

**(R)-Methoxy-phenyl-essigsäure 9-(tert-butyl-dimethyl-silanyloxy)-4,8-dimethyl-1-[1-methyl-2-(2-methyl-thiazol-4-yl)-vinyl]-non-3-enylester**

Eine Lösung von 1022 mg (2,92 mmol) 2-methyl-4-(methyl-tributylphosphoniumbromid)-1,3-thiazol in 19.0 ml abs. THF wurde auf-65˚C gekühlt und 1,56 ml (3,12 mmol) einer 2,0 M Natrium-hexamethyldisilazid-Lösung in THF wurden langsam zugetropft. Nach zehnminütigem Rühren wurde eine Lösung von 1194 mg (2,43 mmol) (*R*)-Methoxy-phenyl-essigsäure (3*S*)-1-acetyl-9-(tert-butyl-dimethylsilanyloxy)-4,8-dimethyl-non-3-enylester in 8.0 ml abs. THF langsam zugetropft.

Nach 60 Minuten Rühren bei -65˚C wurden 45 ml gesättigte $NH_4Cl$-Lösung zugegeben. Die Phasen wurden geschieden und die wässrige Phase fünfmal mit 25 ml Diethylether extrahiert. Die organischen Phasen wurden vereinigt und dreimal mit 30 ml demineralisiertem Wasser sowie einmal mit 50 ml gesättigter NaCl-Lösung gewaschen. Schließlich wurde über $Na_2SO_4$ getrocknet, filtriert und im Vakuum eingeengt. Das zurückbleibende braune Öl wurde mittels Flashchromatographie an Kieselgel (Parameter der Chromatographiesäule: 2,0 x 20,0 cm, Ethylacetat/Petroleumether = 1: 5) gereinigt.

| | |
|---|---|
| Ausbeute | 1,3 g (2,3 mmol, ca. 95%) |
| $R_f$ - Wert | 0,37 (Ethylacetat/Petrolether = 1:5) |

**(*R*)-Methoxy-phenyl-essigsäure (*R*)-9-(tert-butyl-dimethyl-silanyloxy)-4,8-dimethyl-1-[1-methyl-2-(2-methyl-thiazol-4-yl)-vinyl]-non-3-enylester**

Eine Lösung von 1018 mg (2,91 mmol) 2-Methyl-4-(methyl-tributylphosphoniumbromid)-1,3-thiazol in 19,0 ml abs. THF wurde auf -65˚C gekühlt und 1,39 ml (2,79 mmol) einer 2.0M Natrium-hexamethyldisilazid-Lösung in THF wurden langsam zugetropft. Nach zehnminütigem Rühren wurde eine Lösung von 1190 mg (2,42 mmol) (*R*)-Methoxy-phenyl-essigsäure (3*R*)-1-acetyl-9-(tert-butyl-dimethylsilanyloxy)-4,8-dimethyl-non-3-enylester in 8,0 ml abs. THF langsam zugetropft.

Nach 60 Minuten Rühren bei -65˚C wurden 45 ml gesättigte $NH_4Cl$-Lösung zugegeben. Die Phasen wurden geschieden und die wässrige Phase fünfmal mit 25 ml Diethylether extrahiert. Die organischen Phasen wurden vereinigt und dreimal mit 30 ml demineralisiertem Wasser sowie einmal mit 50 ml gesättigter NaCl-Lösung gewaschen. Schließlich wurde über $Na_2SO_4$ getrocknet, filtriert und im Vakuum eingeengt. Das zurückbleibende braune Öl wurde mittels Flashchromatographie an Kieselgel (Parameter der Chromatographiesäule: 2,0 x 20,0 cm, Ethylacetat/Petroleumether= 1: 5) gereinigt.

| | |
|---|---|
| Ausbeute | 1,282 mg (2,19 mmol, 90%) |
| $R_f$ - Wert | 0.37 (Ethylacetat/Petrolether = 1:5) |
| $C_{33}H_{51}NO_4SSi$ | (585,91) |

Teil C - Hydrolysen von O-Acylacyloinen

Im folgenden werden die nachstehend beschriebenen Enzyme eingesetzt:

Lipasen: *Candida antarctica* (Novo lipase B~Chirazyme L2, CAL-B), *Candida antarctica* (Novo, lipase A, CAL-A), *Candida rugosa* (Amano AY, CRL), *Aspergillus niger* (Amano, ANL), *Pseudomonas cepacia* (Amano PS, PCL-PS). *Pseudomonas cepacia* (Amano AK, PCL-AK), *Pseudomonas* sp. (Chirazyme L6, PSL). AYS = *Candida rurgosa,* CAA= *Candida antartica* A, CAB = *Candida antartica* B, MJ = *Mucor javanicus,* PS = *Pseudomonas cepacia,* PPL = porcine pancreas lipase.

Schweineleberesterase, pig liver esterase (PLE, Sigma).

Produktion rekombinanter Esterasen aus *Pseudomonas fluorescens* (PFE, PFE-II), *Streptomyces diastatochromogenes* (SDE) durch Expression in *E. coli* gemäß Literatur (Krebsfänger, N.; Zocher, F.; Altenbuchner, J.; Bornscheuer, U. T. *Enzyme Microb. Technol.* **1998**, *21,* 641-646. Khalameyzer, V.; Bornscheuer, U. T. *Biotechnol. Lett.* **1999,** *21,* 101-104. Khalameyzer, V.; Fischer, I.; Bornscheuer, U. T.; Altenbuchner, J. *Appl. Environm. Microbiol.* **1999**, *65,* 477-482.)

Rekombinante Schweineleberesterase, pig liver esterases (rPLE) durch Expression in der Hefe *Pichia pastoris.* (Musidlowska, A.; Lange, S.; Bornscheuer, U. T. *Angew. Chem. Int. Ed. Engl.* **2001,** im Druck. Lange, S.; Musidlowska, A.; Schmidt-Dannert, C.; Schmitt, J.; Bornscheuer, U. T. *Chem. Bio. Chem.* **2001,** im Druck).

**Allgemeine Vorschrift I zur (racemischen) Verseifung von O-Acylacyloinen**

Der Ester wird in Methanol gelöst (z.B. 0,05 - 1 M, üblicherweise ca. 0,13 M) und dann langsam mit wässeriger Base, bevorzugt - und falls nicht gesondert erwähnt - in gesättigter wässeriger Kaliumcarbonatlösung versetzt (80 μl pro mmol Ester). Nach abgeschlossener Reaktion (z.B. 30 min.) wird mit ges. NaCl-Lösung (ca. 1,5 × des Methanolvolumens) und der fünffachen Menge Ether versetzt. Die organische Phase wird nochmals gründlich mit NaCl-Lösung gewaschen, mit Natriumsulfat getrocknet, filtriert, und in vacuo vom Lösemittel befreit. Weitere Reinigung kann z. B. durch die Chromatographie an Kieselgel erfolgen.

**Allgemeine Vorschrift II zur enzymatischen Racematspaltung von O-Acylacyloinen (kleine Mengen)**

In ein geeignetes Gefäß (hier z. B. ein Eppendorf® 1.5 ml-Gefäß) wird nacheinander bestückt mit Lipase oder Esterase (z.B. circa 20 mg), 0.7 ml Phosphatpuffer (0,1 M, pH 7,0) und dem Acyloinester (z.B. 5 μl). Die Mischung wird heftig aufgeschüttelt und weiterhin normal geschüttelt (Vortex, 300 strokes / Minute) bei 0 -60˚C je nach Enzym, normalerweise bei Zimmertemperatur. Die Reaktion wird durch Zugabe von Aceton beendet. Üblicherweise wird der Verlauf durch einen Titrator oder ein Polarimeter kontrolliert, oder über Zeit, wobei meist etwa 30 Minuten angesetzt werden. Ethylacetat (0,4 ml) wird zugegeben, ausgeschüttelt und zentrifugiert (bei 8000 g 5 min.). Die obere organische Schicht wird entfernt und der Rest erneut extrahiert. Die Extraktion kann schwieriger auch ohne Zentrifugieren erreicht werden. Nach dem Trocknen der vereinten organischen Phasen über $Na_2SO_4$ kann nochmals zentrifugiert werden (z. B. bei 8000 g 5 min).

In der folgenden Tabelle 2 sind Beispiele von Umsetzungen nach allgemeiner Vorschrift II mit Substrat **IV** $R^1$, $R^3$ = Me beschrieben (ee-Werte nach GC, 99+= 99-100%, [1]Die e.e.Werte der Substrate sind berechnete Werte, Alkohol/ Ester 1 und 2 bezeichnen das jeweils andere Enantiomer).

Tabelle 2

| **Substrat IV mit $R^2$ =** | **Enzym** | **e.e.** | **Alkohol im Überschuß** | **e.e.** | **Ester im Überschuß** | **E** |
|---|---|---|---|---|---|---|
| Prenyl | AYS | 2% | Alkohol 2 | 0% | Ester 2 | 1 |
| | CAA | 89% | Alkohol 2 | 19% | Ester 1 | 20 |
| | CAB | 94% | Alkohol 2 | 98% | Ester 1 | ≥ 156 |
| | MJ | 26% | Alkohol 2 | 0% | Ester 1 | 2 |
| | PS | 99% | Alkohol 2 | 99% | Ester 1 | > 200 |
| | PPL | 8% | Alkohol 1 | 1% | Ester 2 | 1 |
| Methylallyl (trans)* (trans-crotyl) | AYS | 7% | Alkohol 1 | 3% | Ester 2 | 1 |
| | CAA | 88% | Alkohol 2 | 76% | Ester 1 | 36 |
| | CAB | 94% | Alkohol 2 | 95% | Ester 1 | ≥ 100 |
| | MJ | 37% | Alkohol 2 | 2% | Ester 1 | 2 |
| | PS | 27% | Alkohol 2 | 100% | Ester 1 | 12 |
| | PPL | 27% | Alkohol 1 | 4% | Ester 2 | 2 |
| Methylallyl (cis)* (cis-crotyl) | AYS | 3% | Alkohol 1 | 0% | Ester 2 | 1 |
| | CAA | 69% | Alkohol 2 | 33% | Ester 1 | 8 |
| | CAB | 95% | Alkohol 2 | 99+% | Ester 1 | ≥ 200 |
| | MJ | 7% | Alkohol 2 | 1% | Ester 1 | 1 |
| | PS | 99+% | Alkohol 2 | 99+% | Ester 1 | 80096 |
| | PPL | 24% | Alkohol 2 | 1% | Ester 1 | 2 |

Fortsetzung der Tabelle 2

| Substrat | Enzyme | e.e. | Alkohol im Überschuß | e.e. | Ester im Überschuß | E |
|---|---|---|---|---|---|---|
| Methylallyl cis (cis-crotyl) | AYS | | | | | |
| | CAA | 63% | Alkohol 2 | 9% | Ester 1 | 5 |
| | CAB | 93% | Alkohol 2 | 99+% | Ester 1 | > 200 |
| | MJ | | | | | |
| | PS | 96% | Alkohol 2 | 99+% | Ester 1 | >200 |
| | PPL | | | | | |
| Hexyl | AYS | 68% | Alkohol 1 | 1% | Ester 2 | 5 |
| | CAA | 71% | Alkohol 2 | 19% | Ester 1 | 7 |
| | CAB | 93% | Alkohol 2 | 99% | Ester 1 | > 120 |
| | PS | 34% | Alkohol 2 | 99% | Ester 1 | 9 |
| | PPL | 32% | Alkohol 2 | 5% | Ester 1 | 2 |
| Benzyl | AYS | 5% | Alkohol 2 | 1% | Ester 1 | 1 |
| | CAA | 85% | Alkohol 2 | 15% | Ester 1 | 15 |
| | CAB | 84% | Alkohol 2 | 14% | Ester 1 | 13 |
| | MJ | 9% | Alkohol 2 | 0% | Ester 1 | 1 |
| | PS | 95% | Alkohol 2 | 97% | Ester 1 | > 150 |
| | PPL | 44% | Alkohol 1 | 2% | Ester 2 | 3 |
| Butyl | AYS | 42% | Alkohol 1 | 2% | Ester 2 | 3 |
| | CAA | 73% | Alkohol 2 | 19% | Ester 1 | 8 |
| | CAB | 87% | Alkohol 2 | 99+% | Ester 1 | > 150 |
| | MJ | 15% | Alkohol 1 | 1% | Ester 1 | 1 |
| | PS | 66% | Alkohol 2 | 99+% | Ester 1 | 30 |
| | PPL | 1% | Alkohol 1 | 2% | Ester 1 | 1 |

Fortsetzung der Tabelle 2

| | | | | | | |
|---|---|---|---|---|---|---|
| Butynyl | AYS | 61% | Alkohol 1 | 20%[1] | Ester 2 | 5 |
| | CAA | 66% | Alkohol 2 | 14%[1] | Ester 1 | 6 |
| | CAB | 91% | Alkohol 2 | 74-91%[1] | Ester 1 | 47-67 |
| | MJ | 17% | Alkohol 2 | 1%[1] | Ester 1 | 1 |
| | PS | 8% | Alkohol 2 | <5%[1] | Ester 1 | 4 |
| | PPL | 25% | Alkohol 1 | 3%[1] | Ester 2 | 2 |
| Neryl | AYS | 2% | Alkohol 2 | 1% | Ester 1 | 1 |
| | CAA | 78% | Alkohol 2 | 52% | Ester 1 | 14 |
| | CAB | 89% | Alkohol 2 | 55% | Ester 1 | 31 |
| | MJ | | nicht gemessen | | nicht gemessen | |
| | PS | 94% | Alkohol 2 | 95% | Ester 1 | > 100 |
| | PPL | | nicht gemessen | | nicht gemessen | |
| 2-Acetoxy-cyclopentanon C5(c) | AYS | 64% | Alkohol 1 | 15% | Ester 2 | 5 |
| | CAA | 47% | Alkohol 1 | 0% | Ester 2 | 3 |
| | MJ | 35% | Alkohol 1 | 1% | Ester 2 | 2 |
| | PS | 77% | Alkohol 1 | 95% | Ester 2 | 27 |
| | PPL | 60% | Alkohol 1 | 8% | Ester 2 | 4 |

* Gemessen aus einer etwa 9/1-Mischung der trans- und cis-Isomere.

**Allgemeine Vorschrift III zur enzymatischen Racematspaltung von O-Acylacyloinen**

Der Ester wurde in 0,1 M Phosphatpuffer pH 7,0 (Konzentration ca. 0,07 M) gelöst Lipase zugesetzt (z. B. bis 25 Gew. % des Acyloinesters). Während der Reaktion wurde bis zur Vollständigkeit geschüttelt (DC und GC-Kontrolle). Aceton wurde zugesetzt (1/2 des Puffervol.) und fünf mal mit Ethylacetat extrahiert (1,5 × Puffervol.). Die organischen Phasen werden zweimal mit NaCl-Lösung gewaschen, mit Natriumsulfat getrocknet und *in vacuo* vom Lösemittel befreit. Alkohol und Ester werden durch Chromatographie getrennt (z. B. an Kieselgel mit Ethylacetat/Petroleumether = 1:6)

**Allgemeine Vorschrift IV zur enzymatischen Racematspaltung von O-Acylacyloinen**

Kleine Ansätze: 10 mg Hydrolase (Lipase oder Esterase) werden in 1 ml Natriumphosphat-puffer (pH 7,5, 50 mM) in 2 ml Reaktionsgefäßen bei 37°C geschüttelt (z.B. mit Thermoshaker - Eppendorf, Hamburg, Deustchland). Dann werden 200 µl Substratlösung zugesetzt (10 mg/ml in Toluol). Nach 20 h wird die Reaktion durch Abzentrifugieren des Enzyms gestoppt, die Toluolschicht abgetrennt und analysiert (z. B. durch GC, Polarimetrie)

Größere Ansätze: Diese werden ähnlich durchgeführt, aber in 10 ml Phosphatpuffer mit 200 mg Hydrolase und 0,44 mmol (etwa 100 mg) Acyloinester in 3 ml Toluol. Zum Ende der Reaktion werden 10 ml Toluol zugesetzt, das Enzym durch Zentrifugieren entfernt und die organische Schicht abgetrennt. Die wässerige Phase wird noch zweimal mit 5 ml Toluol extrahiert, die vereinten organischen Phasen getrocknet ($Na_2SO_4$) und vom Lösemittel in vacuo befreit. Acyloin und der nicht reagierte Ester werden chromatographisch getrennt (z. B. an Kieselgel mit Petrolether:Ether, 4: 1). Enantiomerenüberschüsse können am besten mittels Chiralphasen-Gaschromatograpie (CP-GC), chemische Identität mittels NMR-Spektroskopie analysiert werden.

**Allgemeine Vorschrift V zu enzymkatalysierter Umesterung in organischen Lösemitteln (Enzymatische Veresterung freier Acyloine).**

Eine Lösung von 0,44 mmol des Acyloins in 3 ml Toluol, activiertes Molecularsieb (3 Å), 200 mg Lipase oder Esterase und Vinylacetat (1,2 Äquivalente) werden bei 37˚C zur Reaktion gebracht, bis die gewünschte Umsetzung erfolgt ist (ggf. Schütteln). Die Reaktion wird durch Zentrifugieren beendet, das Lösemittel abgenommen und durch Vakuumverdampfung entfernt. Ester und nicht reagiertes freies Acyloin können chromatographisch getrennt werden (z. B. an Kieselgel mit Petrolether:Ether, 4:1). Enantiomerenüberschüsse können am besten mittels Chiralphasen-GC, Chemische Identität mittels NMR-Spektroskopie analysiert werden.

Anstelle des Vinylacetates lassen sich auch andere Aktivester einsetzen.

**Tabelle 3**. Racematspaltung von Acyloin acetat UWE**1b** nach Vorschrift IV (klein)

| Hydrolase[a] | Umsatz [%][b] | Enantiomerenüberschuß (e.e.)[b] Acetat [%ee] | Acyloin [%ee] | E-Wert[c] |
|---|---|---|---|---|
| CAL-A | 99 | 0 | 0 | 0 |
| CAL-B | 30 | 31 | 80 | 12 |
| ANL | 7 | n.d.[d] | n.d. | n.d. |
| CRL | 30 | 18 | 13 | 3 |
| PCL | 51 | >99 | 93 | >100 |
| PFE | 56 | >99 | 92 | >100 |
| SDE | 56 | >99 | 85 | 64 |

[a] Abk. s.o., Haupttext oder Fachbücher; [b]gemäß GC; [c]berechnet nach Chen et al. ( *J. Am. Chem. Soc.* **1982,** *104,* 7294-7299); [d]n.d.,nicht bestimmt

**Tabelle 4.** Racematspaltung von Acyloinacetat UWE**3a** nach Vorschrift IV (klein).

| Hydrolase[a] | Umsatz [%][b] | Enantiomerenüberschuß[b] Acetat [%ee] | Acyloin [%ee] | E-Wert[c] |
|---|---|---|---|---|
| CAL-B | 5 | 5 | 96 | >50 |
| PSL | 50 | >99 | >99 | >100 |
| PCL-AK | 50 | >99 | >99 | >100 |
| PLE[d] | 80 | >99 | 20 | 6 |
| rPLE[d,e] | 25 | 33 | >98 | >50 |
| SDE | 50 | >99 | >99 | >100 |
| PFE | 11 | 11 | 86 | 15 |
| PFE-II | 75 | <3 | <1 | 1 |

[a] Abk. s. Verzeichnis oder Fachbücher; [b]gemäß GC; [c]berechnet nach Chen et al. (*J. Am. Chem. Soc.* **1982,** *104,* 7294-7299); [d]umgekehrte Stereopräferenz; [e]Rekombinante Schweineleberesterase (pig liver esterase)

Racemische freie Acyloine

**(R, *S*) 3-Hydroxy-5-hepten-2-on**

Gemäß allgemeiner Vorschrift 1 werden 48 mg (0,26 mmol) 3-Acetoxy-5-hepten-2-on 20 min. hydrolysiert, wobei die Lösung trübe und dann gelblich wird. Chromatographie (DiethyletherIPetrolether = 1:4) ergibt:

| | |
|---|---|
| Ausbeute | 28 mg (0,20 mmol, 77%) |
| $R_1$-Wert | ca. 0,46 mit Ethylacetat/Hexan = 1:4 |

**(*R*, *S*) 3-Hydroxy-nonan-2-on**

Gemäß allgemeiner Vorschrift I werden 60 mg (0,30 mmol) 3-Acetoxy-nonan-2-on 20 min hydrolysiert, wobei die Lösung trübe und dann gelblich wird.
Chromatographie ergibt:

| | |
|---|---|
| Ausbeute | 42 mg (0,27 mmol, 88%) |
| $R_f$-Wert | ca. 0,63 mit Ethylacetat/Hexan = 1:4 |

**(*R*, *S*) 3-Hydroxy-6-methyl-heptan-2-on**

Gemäß allgemeiner Vorschrift I werden 47 mg (0,25 mmol) 3-acetoxy-6-methylheptan-2-on 5 min. hydrolysiert, wobei die Lösung trübe und dann gelblich wird.

Ausbeute 32 mg (0,22 mmol, 88%)

**(*R*, *S*) 3-Hydroxy-5(Z)-hepten-2-on**

Gemäß allgemeiner Vorschrift I werden 43 mg (0,25 mmol) 3-acetoxy-5(Z)-hepten-2-on 5 min hydrolysiert, wobei die Lösung trübe und dann gelblich wird.

Ausbeute 31 mg (0.24 mmol, 96%)

**(*R, S*) (E) 3-Hydroxy-6,10-dimethyl-5,9-undecen-2-on**

Gemäß allgemeiner Vorschrift I werden 63 mg (0,25 mmol) (E) 3-Acetoxy-6,10-dimethyl-5,9-undecen-2-on 6 min hydrolysiert, wobei die Lösung trübe und dann gelblich wird.

| | |
|---|---|
| Ausbeute | 47 mg (0,22 mmol, 89%) |
| $R_f$-Wert | ca. 0.35 mit Ethylacetat/Hexan = 1:4 |

**(*R*, *S*) (Z) 3-Hydroxy-6,1 0-dimethyl-5,9-undecen-2-on**

Gemäß allgemeiner Vorschrift I werden 63 mg (0,25 mmol) (Z) 3-Acetoxy-6,10-dimethyl-5,9-undecen-2-on 6 min hydrolysiert, wobei die Lösung trübe und dann gelblich wird.

| | |
|---|---|
| Ausbeute | 47 mg (0,22 mmol, 89%) eines gelben Öls |
| $R_f$-Wert | 0,42 mit Ethylacetat/Hexan = 1:4 |

Optisch aktive nicht-racemische Alkohole und Ester

Das jeweilige Startmaterial ist nicht zusätzlich im Titel erwähnt.

**Optisch aktives 3-glydroxy-heptan-2-on**

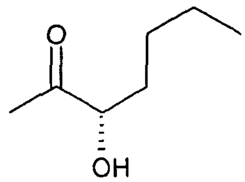

Gemäß allgemeiner Vorschrift III werden 100 mg (0,58 mmol) 3-Acetoxy-heptan-2-on mit 6 mg *Candida antartica* B Lipase in 10 ml Puffer umgesetzt und nach 3,75 h beendet. Nach Chromatographie erhält man:

| | | |
|---|---|---|
| Ausbeute | Alkohol: | 20 mg (0,15 mmol, 26%) |
| | Ester: | 35 mg (0,20 mmol, 35%) |
| $R_f$-Wert | Alkohol: | ca. 0,33 mit Ethylacetat/Hexan = 1:4 |
| | Ester: | ca. 0,48 mit Ethylacetat/Hexan = 1:4 |
| $[\alpha]_D^{25}$ (ca-Werte) | Alkohol: | +69,9° (c=0,410; $CHCl_3$; e.e. = 82%) |
| | Ester: | +9,6° (c=1,805; $CHCl_3$; e.e. = 78%) |

**Optisch aktives 3-Hydroxy-nonan-2-on**

Gemäß allgemeiner Vorschrift III werden 16 mg (0,58 mmol) 3-Acetoxy-nonan-2-on mit 7 mg *Candida antartica* B Lipase in 10 ml Puffer umgesetzt und nach 4,5 h beendet. Nach Chromatographie erhält man:

| | | |
|---|---|---|
| Ausbeute | Alkohol: | 10 mg (0,063 mmol, 11%) |
| | Ester: | 46 mg (0,23 mmol, 40%) |
| $R_f$-Wert | Alkohol: | ca. 0,44 mit Ethylacetat/Hexan = 1:4 |
| | Ester: | ca. 0,61 mit Ethylacetat/Hexan =1:4 |
| $[\alpha]_D^{25}$: | Ester: | +6,3° (c=1,615; $CHCl_3$) |

**Optisch aktives 3-Hydroxy-5(Z)-hepten-2-on**

Gemäß allgemeiner Vorschrift III werden 32 mg (0,19 mmol) 3-Acetoxy-5(Z)-hepten-2-on mit 20 mg *Amano PS* Lipase in 5 ml Puffer umgesetzt und nach 40 min beendet. Nach Chromatographie erhält man die Produkte:

| | | |
|---|---|---|
| **$R_f$-Wert** | Alkohol: | ca. 0,30 mit Ethylacetat/Hexan =1:4 |
| | Ester: | ca. 0,50 mit Ethylacetat/Hexan = 1:4 |

**Optisch aktives 3-Hydroxy-6-methyl-5-hepten-2-on**

Gemäß allgemeiner Vorschrift III werden 200 mg (1,09 mmol) 3-Acetoxy-6-methyl-5-hepten-2-on mit 63 mg Amano PS Lipase in 15 ml Puffer umgesetzt und nach 6 h beendet. Nach Chromatographie erhält man die Produkte:

| | | |
|---|---|---|
| Ausbeute | Alkohol: | 71 mg (0,50 mmol, 46%) eines klaren farblosen Öls |
| | Ester: | 103 mg (0,56 mmol, 52%) eines klaren farblosen Öls |
| $R_f$-Wert | Alkohol: | ca. 0,22 mit Ethylacetat/Hexan = 1:4 |
| | Ester: | ca. 0,50 mit Ethylacetat/Hexan = 1:4 |

(fortgesetzt)

| | | |
|---|---|---|
| $[\alpha]_D^{25}$: | Alkohol: | +77,3° (c=0,979; $CHCl_3$) |
| | Ester: | -15.5° (c=2,030; $CHCl_3$) |

**Optisch aktives 3-Hydroxy-4-phenylbutan-2-on**

Gemäß allgemeiner Vorschrift III werden 150 mg (0,73 mmol) 3-Acetoxy-4-phenylbutan-2-on mit 38 mg *Amano PS* Lipase in 15 ml Puffer umgesetzt und nach 3 h beendet. Nach Chromatographie erhält man die Produkte:

| | | |
|---|---|---|
| Ausbeute | Alkohol: | 51 mg (0,31 mmol, 43%) eines klaren farblosen Öls |
| | Ester: | 76 mg (0,37 mmol, 50%) eines klaren farblosen Öls. |
| $[\alpha]_D^{25}$: | Alkohol: | +64,1° (c=0,820; $CHCl_3$; e.e. = 89%) |
| | Ester: | -3.0° (c=2,325; $CHCl_3$; e.e. = 85%) |

**Optisch aktives 3-Hydroxy-6,10-dimethylundeca-5(Z),9-dien-2-on**

Gemäß allgemeiner Vorschrift III werden 200 mg (0,79 mmol) 3-Acetoxy-6,10-dimethylundeca-5(Z),9-dien-2-on mit 63 mg Amano PS Lipase in 15 ml Puffer umgesetzt und nach 6 h beendet. Nach Chromatographie erhält man die Produkte:

| | | |
|---|---|---|
| Ausbeute | Alkohol: | 70 mg (0,33 mmol, 42%) eines klaren farblosen Öls |
| | Ester: | 107 mg (0,42 mmol, 54%) eines klaren farblosen Öls. |
| $R_f$-Wert | Alkohol: | ca. 0,38 mit Ethylacetat/Hexan = 1:4 |
| | Ester: | ca. 0,52 mit Ethylacetat/Hexan = 1:4 |
| $[\alpha]_D^{25}$: | Alkohol: | +63,7° (c=2,115; $CHCl_3$; e.e. = 95%) |
| | Ester: | -19,7° (c=1,560; $CHCl_3$; e.e. = 97%) |

**Optisch aktives (*Z*)-3-Acetoxy-6,10-dimethyl-5,9-undecadien-2-on und (Z)-3-Hydroxy-6,10-dimethyl-5,9-undecadien-2-on:** Hydrolyse (größerer Ansatz) mit PCL nach allgemeiner Vorschrift IV gibt optisch aktives (*Z*)-3-Acetoxy-6,10-dimethyl-5,9-undecadien-2-on (40% Ausbeute, >99 %ee) und (*Z*)-3-Hydroxy-6,10-dimethyl-5,9-undecadien-2-on (43 % Ausbeute, 92 %ee), E»100.

**Optisch aktives (*E*)-3-Acetoxy-6,10-dimethyl-5,9-undecadien-2-on und (*E*)-3-Hydroxy-6,10-dimethyl-5,9-undecadien-2-on:** Hydrolyse (größerer Ansatz) mit PCL nach allgemeiner Vorschrift IV gibt optisch aktives (*E*)-3-Acetoxy-6,10-dimethyl-5,9-undecadien-2-on (40 % Ausbeute, 95 %ee) und (*E*)-3-Hydroxy-6,10-dimethyl-5,9-undecadien-2-on (32 % Ausbeute, >99 %ee), E»100.

**Optisch aktives (Z)-3-Hydroxy-6,10-dimethyl-5,9-undecadien-2-on und (Z)-3-Acetoxy-6,10-dimethyl-5,9-undecadien-2-on:** Acylierung mit PCL nach allgemeiner Vorschrift V gibt optisch aktives (*Z*)-3-Hydroxy-6,10-dimethyl-5,9-undecadien-2-on (40 % Ausbeute, 95 %ee) und (*Z*)-3-Acetoxy-6,10-dimethyl-5,9-undecadien-2-on (32 % Ausbeute, >99 %ee), E>>100.

**Optisch aktives (*E*)-3-Acetoxy-6,10-dimethyt-5-en-11-hydroxy-undecan-2-on und (*E*)-3-Hydroxy-6,10-dimethyl-5-en-11-hydroxy-undecan-2-on:** Hydrolyse (0,94 mmol) mit PCL nach allgemeiner Vorschrift IV gibt optisch aktives (*E*)-3-Acetoxy-6,10-dimethyl-5-en-11-hydroxy-undecan-2-on (28 % Ausbeute, 92 %ee) und Alkohol (*E*)-3-Hydroxy-6,10-dimethyl-5-en-11-hydroxy-undecan-2-on (28 % Ausbeute, >99 %ee), E»100.

**Optisch aktives (*E*)-3-Acetoxy-6,10-dimethyl-5-en-11-*O*-TBDMS-undecan-2-on und (*E*)-3-Hydroxy-6,10-dimethyl-5-en-11-*O*-TBDMS-undecan-2-on:** Hydrolyse (1,25 mmol) mit PCL (Chirazyme L6) nach allgemeiner Vorschrift IV ergibt optisch aktives (*E*)-3-Acetoxy-6,10-dimethyl-5-en-11-*O*-TBDMS-undecan-2-on (48 % y, 46 %ee) und Alkohol 3-Hydroxy-6,10-dimethyl-5-en-11-*O*-TBDMS-undecan-2-on (42 % Ausbeute, >99 %ee), E»100. Eine weitere Verbesserung der optischen Ausbeuten von (*E*)-3-Acetoxy-6,10-dimethyl-5-en-11-*O*-TBDMS-undecan-2-on wurde

durch eine zweite kinetische Racematspaltung mit PCL (Amano PS) erreicht. Ausbeute 35 % (*E*)-3-Acetoxy-6,10-dimethyl-5-en-11-*O*-TBDMS-undecan-2-on mit >98 %ee.

**Beispiel 2:**

Bei den angegebenen Verbindungen kann es sich, sofern nicht explizit anders angegeben, auch um Isomerengemische, Racemate, und/oder Diastereomere) handeln. Enantiomerenangereicherte Verbindungen sind gewöhnlich durch die Angabe eines Drehwertes angezeigt. Bei Bedarf wurden die erhaltenen Produkte durch im Stand der Technik bekannte Reinigungsverfahren gereinigt.

**1. *tert*-Butyl-2-bromacetoacetate**

Zu einer Lösung von *tert*-butyl acetoacetate (49.0 ml, 47.5 g, 300 mmol) in Aceton (30 ml) wurde *N*-bromsuccinimid (58.7 g, 330 mmol) portionsweise gegeben. Die erhaltene Suspension wurde eine Stunde bei Umgebungstemperatur gerührt und anschliessend filtriert. Das Filtrat wurde im Vakuuum eingeengt und der Rückstand in 300 ml Petroleumether aufgenommen. Nach dreimaligem Waschen mit je 100 ml Wasser wurde die Lösung über $Na_2SO_4$ getrocknet und filtriert. Anschließend wurde durch Einengen im Vakuum das Produkt erhalten.

**2. *tert*-Butyl-2-acetoxyacetoacetate**

Eine Lösung von Eisessig (10.6 ml, 186 mmol) in DMF (169 ml) wurde mit Triethylamin (25.8 ml, 186 mmol) unter Eiskühlung neutralisiert. Zu der so erhaltenen Triethylammonium-acetatlösung wurde bei 0˚C *tert*-butyl-2-bromacetoacetate (40.0 g, 169 mmol) tropfenweise zugegeben. Das Eisbad wurde nach vollendeter Zugabe entfernt und die Reaktionsmischung wurde zwei Stunden bei Raumtemperatur gerührt. Anschliessend wurde mit Wasser (280 ml) gelöscht und mit Ethylacetat extrahiert (3 x 215 ml). Die organischen Phasen wurden vereinigt und sowohl mit Wasser (3 x 215 ml) als auch konzentrierter NaCl-Lösung (1 x 215 ml) gewaschen. Nach trocknen über $Na_2SO_4$, Filtrieren und Einengen im Vakuum erhielt man das Produkt Öl, welches durch zweifache Destillation bei 12 mbar und 128˚C gereinigt wurde.

**3. (4*Z*)-*2*-Acetoxy-2-acetyl-5,9-dimehtyl-deca-4,8-diensäure-*tert*-butylester**

*tert*-Butyl-2-acetoxyacetoacetat (19.5 g, 90 mmol) wurde langsam zu einer Suspension von NaH ( 2.59 g, 108 mmol) in DMF (180 ml) at 0˚C getropft. Nachdem die Gasentwicklung abgeschlossen war, wurde Nerylbromid (19.6 g, 90 mmol) bei 0˚C langsam zugetropft. Schliesslich wurde das Eisbad entfernt und 16 Stunden bei Umgebungstemperatur gerührt. Die erhaltene gelbe Lösung wurde mit Diethylether verdünnt (750 ml) und mit Wasser (3 x 200 ml) und konzentrierter NaCl-Lösung (1 x 200 ml) gewaschen. Nach Trocknen über $Na_2SO_4$, Filtrieren und Einengen im Vakuum erhielt man das Produkt.

**4. (4*Z*)-*2*-Acetoxy-2-acetyl-5,9-dimehtyl-10-hydroxy-deca-4,8-diensäure*tert*-butylester**

Fein pulverisiertes Selendioxid (0.158 g, 1.42 mmol) wurde in DCM (50 ml) suspensiert. Danach gab man eine 70% *tert*-Butylhydroperoxidlösung (10.2 g, 79.5 mmol) zu und rührte 30 Minuten bei Umgebungstemperatur. Schliesslich wurden 10.0 g (28.4 mmol) (4*Z*)-*2*-Acetoxy-2-acetyl-5,9-dimehtyl-deca-4,8-diensäure-*tert*-butylester zugegeben und bei Umgebungstemperatur für 48 Stunden gerührt. Nach Einengen im Vakuum wurden 50 ml Toluol zugegeben und im Vakuum entfernt (dies dient der Entfernung überschüssigen *tert*-Butylhydroperoxids). Dies wurde insgesamt dreimal wiederholt und das erhaltene Produkt wurde mittels Flashchromatographie (Parameter der Chromatographiesäule: 5.0 x 19.5 cm, Ethylacetat/Petroleumether = 1:2) getrennt.

**5. Enantiomerenangereicherter (4*Z*)-*2*-Acetoxy-2-acetyl-5,9-dimehtyl-10-hydroxy-deca-4-ensäure-*tert*-butylester**

7.94 g (21.6 mmol) (4*Z*)-*2*-Acetoxy-2-acetyl-5,9-dimehtyl-10-hydroxy-deca-4,8-diensäure-*tert*-butylester wurden in 15.0 ml absolutem Methanol gelöst und 750 µl demineralisiertes Wasser zugefügt. Die erhaltene Lösung wurde mittels dreier Einfrier-Auftau-Zyklen entgast. Anschliessend wurden 185 mg Ru(*R*-BWAP)$(OAc)_2$ sowie ein Magnetrührstab zugegeben und die erhaltene zitronengelbe Lösung in einen Autoklaven überführt. Die Stickstoffatmosphäre wurde mittels dreimaligem Füllen und Entlassen von Wasserstoffgas (5.0 Qualität) aus dem Autoklaven verdrängt. Schliesslich wurde ein Druck von 100 bar Wasserstoffgas (5.0 Qualität) angelegt und 25 Stunden bei Umgebungstemperatur gerührt. Das Gas wurde aus dem Autoklaven entlassen und die Lösung wurde im Vakuum eingeengt. Das zurückbleibende

Produkt wurde mittels Flashchromatographie (Parameter der Chromatographiesäule: 4.5 x 25.0 cm, Ethylacetat/Petroleumether = 1:2) gereinigt.

**6. Enantiomerenangereichertes 3-Acetoxy-11-hydroxy-6,10-dimethyl-5-undecen-2-on**

1.032 g (2.79 mmol) (9*S*)(4*Z*)-2-acetoxy-2-acetyl-5,9-dimehtyl-10-hydroxy-deca-4-ensäure-*tert*-butylester wurden in 28 ml DCM gelöst und 2.80 ml TFA zugegeben. Nach zweistündigem Rühren bei Umgebungstemperatur wurden alle flüchtigen Bestandteile im Vakuum entfernt und das zurückbleibende braune Öl in 28.0 ml Methanol gelöst. Anschliessend wurden 5.6 ml gesättigte $NaHCO_3$-Lösung zugegeben und die erhaltene Suspension 140 Minuten bei Umgebungstemperatur gerührt. Nach Zugabe von 200 ml Ether wurde die organische Phase zweimal mit 50 ml demineralisiertem Wasser sowie einmal mit 50 ml konzentrierter NaCl-Lösung gewaschen und anschliessend über $Na_2SO_4$ getrocknet. Nach Filtrieren und Einengen im Vakuum erhielt man das Produkt, welches mittels Flashchromatographie (Parameter der Chromatographiesäule: 2.0 x 20.0 cm, Ethylacetat/Petroleumether = 2:3) gereinigt wurde.

**7. Enantiomerenangereichertes 3-Acetoxy-11-*tert*-butyl-dimethylsilyloxy-6,10-dimethyl-5-undecen-2-on**

528 mg (1.95 mmol) 3-Acetoxy-11-hydroxy-6,10-dimethyl-5-undecen-2-on wurden in 10.0 ml absolutem DCM gelöst und mit sowohl 541 µl (395 mg, 3.90 mmol) Triethylamin als auch 12 mg (0.10 mmol) DMAP versetzt. Die Lösung wurde mit Hilfe eines Eisbades gekühlt und fünf Minuten gerührt. Danach wurden 368 mg (2.44 mmol) TBDMSCl auf einmal zugefügt und die erhaltene Lösung erst zwei Stunden bei 0˚C und schliesslich 14 Stunden bei Umgebungstemperatur gerührt. Man erhielt eine farblose Suspension, welche man auf 0˚C abkühlt, bevor man schliesslich 460 µl Methanol zufügte und 30 Minuten rührte. Anschliessend wurden 15 ml Diethylether und 15 ml gesättigte Ammoniumchloridlösung zugefügt und intensiv gerührt. Die wässrige Phase wurde zweimal mit 10 ml Diethylether extrahiert und die organischen Phasen vereinigt. Es wurde mit 15 ml ges. NaCl-Lösung gewaschen, über $Na_2SO_4$ getrocknet, filtriert und im Vakuum eingeengt. Das zurückbleibende Produkt wurde mittels Flashchromatographie (Parameter der Chromatographiesäule: 2.0 x 20.0 cm, Ethylacetat/Petroleumether = 1:10) gereinigt.

**8. 3-Acetoxy-11-(tert-butyl-dimethyl-silanyloxy)-2,6,10-trimethyl-1-(2-methyl-thiazol-4-yl)-undeca-1,5-dien**

Eine Lösung von 524 mg (1.50 mmol) 2-methyl-4-(methyl-tributylphosphoniumbromid)-1,3-thiazol in 10.0 ml abs. THF wurde auf-63˚C gekühlt und 749 µl (1.50 mmol) einer 2.0M Natrium-hexamethyldisilazid-Lösung in THF wurden langsam zugetropft. Nach fünfzehnminütigem Rühren wurde eine Lösung von 480 mg (1.25 mmol) 3-Acetoxy-11-*tert*-butyl-dimethylsilyloxy-6,10-dimethyl-5-undecen-2-on in 4.0 ml THF langsam zugetropft.

Nach 30 Minuten rühren bei -63˚C wurde die Lösung auf 55˚C erwärmt und eine weitere Stunde gerührt. Anschliessend wurde das Heizbad entfernt und 20 ml gesättigte $NH_4Cl$-Lösung zugegeben. Die Phasen wurden geschieden und die wässrige Phase dreimal mit 20 ml Diethylether extrahiert. Die organischen Phasen wurden vereinigt und dreimal mit 20 ml demineralisiertem Wasser sowie einmal mit 20 ml gesättigter NaCl-Lösung gewaschen. Schliesslich wurde über $Na_2SO_4$ getrocknet, filtriert und im Vakuum eingeengt. Das zurückbleibende Produkt wurde mittels Flashchromatographie (Parameter der Chromatographiesäule: 2.0 x 20.0 cm, Ethylacetat/Petroleumether = 1:4) gereinigt.

**9. Enantiomerenangereichertes 11-(tert-Butyl-dimethyl-silanyloxy)-2,6,10-trimethyl-1-(2-methyl-thiazol-4-yl)-undeca-1,5-dien-3-ol**

221 mg (0.461 mmol) 3-Acetoxy-11-(tert-butyl-dimethyl-silanyloxy)-2,6,10-trimethyl-1-(2-methyl-thiazol-4-yl)-undeca-1,5-dien und 64 mg (0.461 mmol) $K_2CO_3$ wurden in 5.5 ml absolutem Methanol zwei Stunden bei Umgebungstemperatur gerührt. Anschliessend wurden 30 ml Ethylacetat zugefügt und die organische Phase wurde mit demineralisiertem Wasser (3 x 10 ml) und mit gesättigter NaCl-Lösung (1 x 10 ml) gewaschen, über $Na_2SO_4$ getrocknet, filtriert and letztendlich im Vakuum eingeengt. Man erhielt ein Produkt, welches flashchromatographisch (Parameter der Chromatographiesäule: 2.0 x 20.0 cm, Ethylacetat/Petroleumether = 1:4) gereinigt wurde.

**10. (*4S*)-3-[(*3S*)-4,4-Dimethyl-1,5-dioxo-3-hydroxyheptyl]-4-benzyl-2-oxazolidinon**

Zu 2.55 g (14.4 mmol) (*S*)-4-Benzyl-2-oxazolidinon in 50 ml Tetrahydrofuran wurden bei -78˚C 9.0 ml (14.4 mmol) Butyllithium (1.6M in Hexan) getropft. 1.25 ml (14.4 mmol) Bromacetylbromid wurden auf einmal zugegeben.Man rührt 30 min at -78˚C und erwärmt auf Raumtemperatur. Im Handschuhkasten werden 2.03 g (15.8 mmol) 2,2-dimethyl-3-oxopentanal, 4.42 g (36.0 mmol) $CrCl_2$ und 193 mg (1.44 mmol) LiI zugegeben. Nach acht Stunden Reaktionszeit bei 20˚C wurden 20 ml gesättigte NaCl-Lösung zugegeben und das dunkelgrüne zweiphasige System 15 Minuten lang gerührt. Die organische Phase wurde abgetrennt und die wässrige Phase wurde dreimal mit Diethylether extrahiert. Die

vereinigten organischen Phasen wurden dreimal mit demineralisiertem Wasser sowie einmal mit gesättigter NaCl-Lösung gewaschen, über $MgSO_4$ getrocknet, filtriert und im Vakuum eingeengt.Das Produkt wurde an Kieselgel mit Essigester/Petrolether (1:1) chromatographiert.

**11. (*4S*)-3-[(*3S*)-4,4-Dimethyl-1,5-dioxo-3-(*tert*-butyl-dimethyl-silanyloxy)]-4-benzyl-2-oxazolidinon**

Zu 1.50 g (4.32 mmol) (*4S*)-3-[(*3S*)-4,4-Dimethyl-1,5-dioxo-3-hydroxyheptyl]-4-benzyl-2-oxazolidinon in 20 ml absolutem DCM wurden bei 0˚C unter Argon 1.11 ml (9.50 mmol) frisch destilliertes 2,6-Lutidin und 1.49 ml (6.48 mmol) (*tert*-Butyl-dimethylsilyl)-trifluormethansulfonat gegeben. Nach 2.5 Stunden rühren bei 0˚C gab man 4.0 ml 2N NaOH-Lösung zu und verdünnte mit 30 ml DCM. Nach Phasentrennung wurde die organische Phase zweimal mit 30 ml 2N Salzsäure und einmal mit gesättigter NaCl-Lösung gewaschen. Man trocknete mit $MgSO_4$, engte im Vakuum ein und chromatographierte das Produkt an Kieselgel mit Essigester/Petroleumether (1:4).

**12. (3*S*)-3-(*tert*-Butyl-dimethyl-silanyloxy)-4,4-dimethyl-5-oxo-heptanoic** acid

Zu 742 mg (1.61 mmol) (*4S*)-3-[(*3S*)-4,4-Dimethyl-1,5-dioxo-3-(*tert*-butyl-dimethylsilyloxy)]-4-benzyl-2-oxazolidinon in 36 ml Tetrahydrofuran/Wasser (3:1) wurden bei 0˚C 1.11 ml (9.66 mmol) Wasserstoffperoxidlösung (30%) und 135 mg (3.22 mmol) Lithiumhydroxid-Hydrat gegeben. Nach einer Stunde Rühren wurden 1.40 g Natriumsulfit in 20 ml demineralisiertem Wasser zugegeben. Man pufferte mit wässriger Natriumhydrogencarbonat-Lösung, entfernte Tetrahydrofuran im Vakuum und waschte die wässrige Lösung dreimal mit 5.0 ml DCM. Die wässrige Phase wurde mit HCl (10%) auf pH1 angesäürt und fünfmal mit 10 ml DCM extrahiert. Die organischen Phasen wurden über $MgSO_4$ getrocknet und das Produkt im Vakuum eingeengt.

**13. (3*S*)-6-Brom-3-(*tert*-butyl-dimethyl-silanyloxy)-4,4-dimethyl-5-oxoheptanoic acid**

Zu einer Lösung von 116 mg (0.383 mmol) (3*S*)-3-(*tert*-Butyl-dimethyl-silanyloxy)-4,4-dimethyl-5-oxo-heptanoic acid in 3.8 ml Tetrahydrofuran wurden bei 0˚C 152 mg (0.403 mmol) Phenytrimethylammoniumbromid-dibromid gegeben. Nach fünfzehnminütigem Rühren wurde das Eisbad entfernt und eine weitere Stunde bei Umgebungstemperatur gerührt. Zur erhaltenen Suspension wurden 5.0 ml demineralisiertes Wasser gegeben. Die wässrige Phase wurde dreimal mit 8 ml Diethylether extrahiert. Die vereinigten organischen Phasen wurden mit 10 ml 1N HCl-Lösung und mit 10 ml gesättigter NaCl-Lösung gewaschen, anschliessend über $Na_2SO_4$ getrocknet, letztendlich filtriert und im Vakuum eingeengt. Das zurückbleibende Öl wurde flashchromatographisch (Parameter der Chromatographiesäule: 1.0 x 20.0 cm, Ethylacetat/Petroleumether = 1:4 mit 2% Eisessig) gereinigt. Zwei Fraktionen konnten isoliert werden. Fraktion 1 enthielt ein einziges Diastereomer, Fraktion 2 war eine Diastereomerenmischung.

**14. 6-Brom-3-(tert-butyl-dimethyl-silanyloxy)-4,4-dimethyl-5-oxo-heptanoic acid 9-(tert-butyl-dimethyl-silanyloxy)-4,8-dimethyl-1-[1-methyl-2-(2-methylthiazol-4-yl)-vinyl]-non-3-enyl ester**

127 mg (0.291 mmol) 11-(*tert*-Butyl-dimethyl-silanyloxy)-2,6,10-trimethyl-1-(2-methyl-thiazol-4-yl)-undeca-1,5-dien-3-ol, 115 mg (0.291 mmol) (3*S*)-6-Brom-3-(*tent*-butyl-dimethyl-silanyloxy)-4,4-dimethyl-5-oxo-heptanoic acid und 7 mg (0.058 mmol) DMAP wurden in 1.60 ml absolutem DCM gelöst und auf 0˚C gekühlt. Unter Rühren wurden 73 mg (0.378 mmol) EDCI bei 0˚C zugefügt. Nach zehnminütigem Rühren, wurde das Eisbad entfernt und 18 Stunden bei Umgebungstemperatur gerührt. Das Lösungsmittel wurde im Vakuum entfernt und das Produkt mittels Flashchromatographie (Parameter der Chromatographiesäule: 2.0 x 20.0 cm, Ethylacetat/Petroleumether = 1:4) gereinigt.

**15. 6-Brom-3-(tert-butyl-dimethyl-silanyloxy)-4,4-dimethyl-5-oxo-heptanoic acid 9-hydroxy-4,8-dimethyl-1-[1-methyl-2-(2-methyl-thiazol-4-yl)-vinyl]-non-3-enyl ester**

162 mg (0.202 mmol) 6-Brom-3-(tert-butyl-dimethyl-silanyloxy)-4,4-dimethyl-5-oxo-heptanoic acid 9-(tert-butyl-dimethyl-silanyloxy)-4,8-dimethyl-1-[1-methyl-2-(2-methyl-thiazol-4-yl)-vinyl]-non-3-enyl ester wurden in 6.0 ml eines 1: 1 - Gemisches von DCM und MeOH gelöst und auf 0˚C gekühlt. Nach Zugabe von 47 mg (0.202 mmol) CSA wurde die Lösung 2.5 Stunden bei 0˚C gerührt. Nach Zugabe von 42μl (31 mg, 0.303 mmol) Triethylamin wurden die Lösemittel im Vakuum entfernt und das zurückbleibende Produkt flashchromatographisch (Parameter der Chromatographiesäule: 2.0 x 20.0 cm, Ethylacetat/Petroleumether = 1:2) gereinigt.

**16. 6-Brom-3-(tert-butyl-dimethyl-silanyloxy)-4,4-dimethyl-5-oxo-heptanoic acid 4,8-dimethyl-1-[1-methyl-2-(2-methyl-thiazol-4-yl)-vinyl]-9-oxo-non-3-enyl** ester

119 mg (0.173 mmol) 6-Brom-3-(tert-butyl-dimethyl-silanyloxy)-4,4-dimethyl-5-oxo-heptanoic acid 9-hydroxy-4,8-dimethyl-1-[1-methyl-2-(2-mefihyl-thiazol-4-yl)-vinyl]-non-3-enyl ester wurden in 2.10 ml DCM und 700 µl DMSO gelöst und auf 0°C gekühlt. Anschliessend wurden unter Rühren 120 µl (885 mg, 0.865 mmol) Triethylamin zugegeben. Nach Zugabe von 110 mg (0.692 mmol) $SO_3$•Pyridine-Komplex wurde 40 min bei 0°C gerührt. Anschliesend wurde mit 30 ml Ethylacetat verdünnt und zweimal mit 10 ml demineralisiertem Wasser sowie einmal mit 10 ml gesättigter NaCl-Lösung gewaschen. Die organische Phase wurde über $Na_2SO_4$ getrocknet, anschliessend filtriert und im Vakuum eingeengt. Das erhaltene Produkt wurde nicht weiter behandelt und konnte im folgenden Reaktionschritt direkt eingesetzt werden.

**17. 4-(tert-Butyl-dimethyl-silanyloxy)-8-hydroxy-5,5,7,9,13-pentamethyl-16-[1-methyl-2-(2-methyl-thiazol-4-yl)-vinyl]-oxacyclohexadec-13-ene-2,6-dione [=3-O-(tert-Butyl-dimethyl-silanyl)-epothilone D]**

Zu einer Suspension von 35 mg (0.285 mmol) $CrCl_2$ und 30 mg (0.224 mmol) LiI in 25 ml trockenem Tetrahydrofuran wurde eine Lösung von 80 mg (0.117 mmol) 6-Brom-3-(tert-butyl-dimethyl-silanyloxy)-4,4-dimethyl-5-oxo-heptanoic acid 4,8-dimethyl-1-[l-methyl-2-(2-methyl-thiazol-4-yl)-vinyl]-9-oxo-non-3-enyl ester in 5.0 ml absolutem Tetrahydrofuran innerhalb 80 min mit Hilfe einer Spritzenpumpe zugegeben. Nach vollendeter Zugabe wurde die erhaltene Suspension weitere zwei Stunden bei Umgebungstemperatur gerührt. Anschliessend wurde mit 20 ml halbkonzentrierter N-$H_4$Cl - Lösung gelöscht, die organische Phase mit Diethylether (5x15 ml) extrahiert. Die organischen Phasen wurden vereinigt und zweimal mit 15 ml demineralisiertem Wasser sowie zweimal mit 15 ml gesättigter NaCl-Lösung gewaschen, über $Na_2SO_4$ getrocknet, filtriert und im Vakuum eingeengt. Dabei erhielt man 67 mg eines gelben Öls.

Dieses Öl enthielt verschiedene 6,7,8,15-stereoisomere Makrocyclen, von denen drei auf folgende weise isoliert werden konnten.
Flashchromatographie (Parameter der Chromatographiesäule: 1.0 x 20.0 cm, Dieethylether/Petroleumether = 1:2) erlaubte die Isolierung mehrerer Fraktionen, welche durch präparative HPLC auf einer Econosil C18/10µ 10x250mm Säule unter isocratischen Bedingungen mit 85% MeCN, 15% Wasser (welches 1 % Ameisensäure enthält) bei 5.0 ml/min weiter aufgetrennt werden konnten, und u. a. folgende drei 3-TBS-geschützte Epothilondiastereomere lieferte.

**17.1. Diastereomer A**

| | | |
|---|---|---|
| Ausbeute | 4 mg (0.0066 mmol, 6%) farbloses Öl | |
| $R_f$-Wert | 0.30 | |
| Retentionszeit | 42.5 bis 63 Minuten | |
| HRMS: | berechnet für $C_{33}H_{56}NO_5SSi$ ($MH^+$): | 606.3648 |
| | Gefunden: | 606.3650 |

$$[\alpha]_D^{22} = -5.4^\circ$$

$C_{33}H_{55}NO_5SSi$ (605,95)

**17.2. Diastereomer B**

| | | |
|---|---|---|
| Ausbeute | 7 mg (0.0116 mmol, 10%) farbloses Öl | |
| $R_f$ - Wert | 0.15 und 0.18 | |
| Retentionszeit | 24 bis 36 Minuten | |
| HRMS: | berechnet für $C_{33}H_{56}NO_5SSi$ ($MH^+$): | 606.3648 |
| | gefunden: | 606.3663 |

$$[\alpha]_D^{22} = +4.89^\circ$$

$C_{33}H_{55}NO_5SSi$ (605,95)

**17.3. Diastereomer C**

| | |
|---|---|
| Ausbeute | 2 mg (0.0033 mmol, 3%) farbloses Öl |
| $R_f$- Wert | 0.09 |
| Retentionszeit | 37.5 bis 54.0 Minuten |
| $[\alpha]_D^{22}$ = +14.6° | |
| $C_{33}H_{55}NO_5SSi$ | (605,95) |

**18. 3-Acetoxy-11-(tetrahydro-pyran-2-yloxy)-6,10-dimethyl-5-undecen-2-on**

324 mg (1,20 mmol) eines Rohprodukts von 3-Acetoxy-11-hydroxy-6,10-dimethyl-5-undecen-2-on und 219 µl (202 mg, 2,40 mmol) Dihydropyran wurden in 8,5 ml DCM gelöst. Nach Zugabe von 30 mg (0.12 mmol) Pyridinium-paratoluolsulfonat wurde die Reaktionsmischung 18 Stunden bei Umgebungstemperatur gerührt. Danach wurden 25 ml Diethyether zugefügt, die erhaltene organische Phase einmal mit 25 ml halbkonzentrierter NaCl-Lösung gewaschen und über $Na_2SO_4$ getrocknet. Nach Filtration und Entfernen der Lösemittel im Vakuum wurde das zurückbleibende Produkt mittels Flashchromatographie (Parameter der Chromatographiesäule: 2.0 x 20.0 cm, Ethylacetat/Petroleumether = 1:4) an Kieselgel gereinigt.

**19. Essigsäure 4,8-dimethyl-1-[1-methyl-2-(2-methyl-thiazol-4-yl)-vinyl]-9-(tetrahydro-pyran-2-yloxy)-non-3-enyl ester**

Eine Lösung von 200 mg (0.57 mmol) 2-Methyl-4-(methyl-tributylphosphoniumbromide)-1,3-thiazole in 5.0 ml abs. THF wurde auf-63°C gekühlt und mit 286 µl (0.57 mmol) einer 2.0M Natrium-hexamethyldisilazid Lösung tropfenweise versetzt. Nach Fünfzehnminütigem Rühren wurde eine Lösung von 167 mg (0.48 mmol) 3-Acetoxy-11-(2-tetrahydropyranyloxy)-6,10-dimethyl-5-undecen-2-on in 2.0 ml THF langsam zugetropft. Nach Fünfzehnminütigem Rühren bei -63°C wurde die Lösung auf 55°C erwärmt und eine weitere Stunde gerührt. Danach wurde das Wärmebad entfernt und 10 ml gesättigte $NH_4Cl$ - Lösung zugegeben. Dreifache Extraktion mit 10 ml ether, Waschen der vereinigten Etherphasen mit demi water (3 x 10 ml) und mit gesättigter NaCl-Lösung (1 x 10 ml), Trocknen über $Na_2SO_4$, Filtration und Entfernen des Lösemittels im Vakuum gab ein Produkt, welches mittels Flashchromatographie (Parameter der Chromatographiesäule: 2.0 x 20.0 cm, Ethylacetat/Petroleumether = 1:4) an Kieselgel gereinigt wurde.

**20. Essigsäure 9-hydroxy-4,8-dimethyl-1-[1-methyl-2-(2-methyl-thiazol-4-yl)-vinyl]-non-3-enyl ester**

Eine Lösung von 238 mg (0.53 mmol) Essigsäure 4,8-dimethyl-1-[1-methyl-2-(2-methyl-thiazol-4-yl)-vinyl]-9-(tetrahydro-pyran-2-yloxy)-non-3-enyl ester und 13 mg (0.053 mmol) Pyridinium-paratoluolsulfonate in 5.0 ml 96% Ethanol wurde acht Stunden bei 55°C gerührt. Nach Einengen im Vakuum wurde der Rückstand in 25 ml Diethylether aufgenommen. Die organische Phase wurde mit 5% $NaHCO_3$ (2x10 ml) und mit konzentrierter NaCl-Lösung (1x101) gewaschen, dann über $Na_2SO_4$ getrocknet, schliesslich filtriert und im Vakuum eingeengt. Das zurückbleibende Produkt wurde mittels Flashchromatographie (Parameter der Chromatographiesäule: 2.0 x 20.0 cm, Ethylacetat/Petroleumether = 1:2) an Kieselgel gereinigt.

**21. Essigsäure 4,8-dimethyl-1-[1-methyl-2-(2-methyl-thiazol-4-yl)-vinyl]-9-oxo-non-3-enyl ester**

26 µl (38 mg, 0.30 mmol) Oxalylchlorid wurden in 2.0 ml abs. DCM gelöst und auf -63°C gekühlt. Nach Zugabe einer Lösung von 42 µl (46 mg, 0.59 mmol) abs. DMSO in 0.5 ml abs. DCM wurde 10 min bei -63°C gerührt und eine Lösung von 99 mg (0.27 mmol) Essigsäure 9-hydroxy-4,8-dimethyl-1-[1-methyl-2-(2-methylthiazol-4-yl)-vinyl]-non-3-enyl ester in 1.0 ml abs. DCM langsam zugetropft. Nach dreissigminütigem Rühren bei -68°C wurden 187 µl (137 mg, 1.35 mmol) Triethylamin zugefügt. Schliesslich liess man auf Umgebungstemperatur auftauen und eine Stunde bei dieser Temperatur rühren.

Nach Zugabe von 5.0 ml Wasser wurden die Phasen getrennt und die wässrige Phase zweimal mit 5.0 ml DCM extrahiert. Die vereinigten organischen Phasen wurden mit gesättigter $NH_4Cl$-Lösung (2x4.5 ml), mit Wasser (1x4.5ml) und mit konzentrierter NaCl-Lösung gewaschen (1x4.5 ml). Nach Trocknen über $Na_2SO_4$, Filtration und Einengen im Vakuum wurde das erhaltene Produkt mittels Flashchromatographie (Parameter der Chromatographiesäule: 2.0 x 20.0 cm, Ethylacetat/Petroleumether = 1:2) an Kieselgel gereinigt.

**22. Essigsäure 1-acetyl-9-hydroxy-13,13-dimethoxy-4,8,10,12,12-pentamethyl-11-oxo-tridec-3-enyl ester**

107 mg (0.86 mmol) $CrCl_2$ und 54 mg (0.4 mmol) LiI wurden in 1.0 ml abs. THF suspensiert und mit einer Lösung von 103 mg (0.38 mmol) Essigsäure 1-acetyl-4,8-dimethyl-9-oxo-non-3-enyl ester in 1.0 ml abs. THF versetzt. Anschliessend gibt man 102 mg (0.40 mmol) 4-Brom-1,1-dimethoxy-2,2-dimethyl-pentan-3-on auf einmal zu. Nach dreistündigem Rühren bei Umgebungstemperatur wurde mit 2.0 ml sauerstofffreier ges. NaCl-Lösung gelöscht und die wässrige Phase fünfmal mit 2.0 ml eines 5:1 Diethylether/Pentan Gemischs extrahiert. Die vereinigten organischen Phasen wurden dreimal mit 2.0 ml gesättigter $NH_4Cl$-Lösung gewaschen und über $Na_2SO_4$ getrocknet, filtriert und eingeengt. Das zurückbleibende Produkt wurde mittels Flashchromatographie (Parameter der Chromatographiesäule: 2.0 x 20.0 cm, Ethylacetat/Petroleumether = 1:1) an Kieselgel gereinigt. Man erhielt zwei Diastereomere.

*syn / anti - Diastereomer*
Ausbeute: 97 mg (0.22 mmol, 58%) farbloses Öl

*syn / syn- Diastereomer*
Ausbeute: 58 mg (0.13 mmol, 35%) farbloses Öl

## <u>Beispiel 3</u>

Die nachfolgenden Beispiele wurden analog zu den in Beispiel 1 und 2 oder in der Beschreibung beschriebenen Verfahren dargestellt (Standardumsetzungen sind in R. C. Larock, Comprehensive Organic Transformations, VCH, 1989, ISBN: 0-89573-710-8 beschrieben).

**1. (1*E*,5*Z*)-1-(2-Methylthiazol-4-yl)-3-acetoxy-2,6,10-trimethyl-undeca-1,5,9-trien**

Ausbeute: 93 mg (0.27 mmol, 67%)
$C_{20}H_{29}NO_2S$ (347.52)

**(4*E*)-2-Acetoxy-2-acetyl-5,9-dimethyd-deca-4,8-diensäure-*tert*-butylester**

Ausbeute: 17.6 g (50 mmol, quant.)
MS (EI): m/z (%) = 352 (0.01, M+), 296, 236, 193, 167, 153, 69, 57, 43 (100.0).
$C_{20}H_{32}O_5$ (352.47)

**(5*E*)-*3*-Acetoxy-6,10-dimethyl-undeca-5,9-dien-2-on**

Ausbeute: 11.66 g (46 mmol, 92%)
MS (ESI-MS): m/z (%) = 253.0 (100) $[M+H]^+$, 209 (42) $[M-COCH_3]^+$, 193 (23) $[M+H-AcOH]^+$.
$C_{15}H_{24}O_3$ (252.35)

**(5*E*)-3-acetoxy-11-hydroxy-6,10-dimethyl-undeca-5,9-dien-2-on**

Ausbeute: 1.63 (6.07 mmol, 27%)
$C_{15}H_{24}O_4$ (268.35)

**(5*E*)-Essigsäure 4,8-dimethyl-1-(2-methyl-[1,3]dioxolan-2-yl)-nona-3,7-dienylester**

| | |
|---|---|
| Ausbeute: | 5.78 g (19.5 mmol, 97%) |
| $C_{17}H_{28}O_4$ | (296.40) |

**(5*E*)-Essigsäure-9-hydroxy-4,8-dimethyl-1-(2-methyl-[1,3]dioxolan-2-yl)-nona-3,7-dienyl ester**

| | |
|---|---|
| Ausbeute: | 1.59 g (5.08 mmol, 26%) |
| MS (ESI-MS): | m/z (%) = 313.0 (48) $[M+H]^+$. |
| $C_{17}H_{28}O_5$ | (312.40) |

**(5*E*)-Essigsäure-9-hydroxy-4,8-dimethyl-1-(2-methyl-[1,3]dioxolan-2-yl)-non-3-enyl ester**

| | |
|---|---|
| Ausbeute: | 1.185 g (3.77 mmol, 80%) farbloses Öl |
| $C_{17}H_{30}O_5$ | (314.42) |

**(5*E*)-Essigsäure-9-(4-methoxy-benzyloxy)-4,8-dimethyl-1-(2-methyl-[1,3]dioxolan-2-yl)-non-3-enyl ester**

| | |
|---|---|
| Ausbeute: | 796 mg (1.84 mmol, 68%) |
| MS (CI, isobut.): | m/z (%) = 435 (0.3) $[M+H]^+$, 375 (0.3), 313 (0.7), 253 (0.4), 241 (1.0), 193 (0.6), 163 (0.9), 121 (52), 87 (51). |
| $C_{25}H_{38}O_6$ | (434.57) |

**(10*S*)-*3*-Acetoxy-11-hydroxy-6,10-dimethyl-5-undecen-2-on**

| | |
|---|---|
| Ausbeute: | 568 mg (2.10 mmol, 75%) farbloses Öl |
| MS (ESI-MS): | m/z (%) = 563.3 (100) $[2M+Na]^+$, 293.0 (54) $[M+Na]^+$, 271.1 (7) $[M+H]^+$. |
| $C_{15}H_{26}O_4$ | (270.36) |

**(10*S*)-3-Acetoxy-11-*tert*-butyl-dimethylsilyloxy-6,10-dimethyl-5-undecen-2-on**

| | |
|---|---|
| Ausbeute | 597 mg (1.55 mmol, 80%) |
| MS (CI, isobut.): | m/z (%) = 385 (13) $[M+H]^+$, 327 (13), 267 (26), 253 (6), 193 (40),175 (62), 117 (100). |
| $C_{21}H_{40}O_4Si$ | (384.63) |

**(10*S*)-3-Acetoxy-11-(*tert*-butyl-dimethyl-silanyloxy)-2,6,10-trimethyl-1-(2-methyl-thiazol-4-yl)-undeca-1,5-dien**

| | |
|---|---|
| Ausbeute: | 1169 mg (2.44 mmol, 94%) |
| MS (CI, isobut.): | m/z (%) = 480 (9) $[M+H]^+$, 422 (22), 420 (51), 362 (5), 210 (>100), 178 (35), 168 (>100), 164 (72), 128 (55), 117 (65). |
| $C_{26}H_{45}NO_3$ | SSi(479.79) |

**(10*S*)-3-Acetoxy-11-(tetrahydro-pyran-2-yloxy)-6,10-dimethyl-5-undecen-2-on**

| | |
|---|---|
| Ausbeute: | 429 mg (1.21 mmol, 64%) farbloses Öl |
| $C_{20}H_{34}O_5$ | (354,48) |

**(10*S*)-3-Acetoxy-11-(tetrahydro-pyran-2-yloxy)-2,6,10-trimethyl-1-(2-methylthiazol-4-yl)-undeca-1,5-dien**

| | |
|---|---|
| Ausbeute: | 216 mg (0.48 mmol, quant.) farbloses Öl |
| $C_{25}H_{39}NO_4S$ | (449,65) |

**(2*E,*6*Z*)-3,7-Dimethyl-8-(tetrahydro-pyran-2-yloxy)-octa-2,6-dien-1-ol**

| | |
|---|---|
| Ausbeute: | 10.27g (40.4 mmol, 99%) gelbes Öl |
| $C_{15}H_{26}O_3$ | (254.37) |

**1-Chloro-3,7-Dimethyl-8-(tetrahydro-pyran-2-yloxy)-2,6-octadien**

| | |
|---|---|
| Ausbeute: | 9.80 g (36.0 mmol, quant.) |
| $C_{15}H_{25}ClO_2$ | (272.81) |

**2-Acetoxy-2-acetyl-5,9-dimethyl-10-(tetrahydro-pyran-2-yloxy)-deca-4,8-dienonsäure *tert*-butyl ester**

| | |
|---|---|
| Ausbeute: | 6.67 g (14.7 mmol, 88%) |
| MS (ESI-MS): | m/z (%) = 475.3 (68) $[M+Na]^+$, 453.2 (23) $[M+H]^+$, 269.2 (80) $[M+H-DHP]^+$, 313.1 (51) $[M+H-C_4H_8-DHP]^+$, 295.1 (100) $[M+H-C_4H_8-DHP-H_2O]^+$. |
| $C_{25}H_{40}O_7$ | (452,58) |

**11-(*Tert*-butyl-dimethyl-silanyloxy)-3-hydroxy-6,10-dimethyl-undec-5-en-2-on**

| | |
|---|---|
| Ausbeute: | 1.686 g (4.92 mmol, 97%) |
| $C_{19}H_{38}O_3Si$ | (342.59) |

**(3*S*)-6-Bromo-3-(*tert*-butyt-dimethyt-slianyloxy)-4,4-dimethyl-5-oxoheptansäure-4,8-dimethyl-1-[1-methyl-2-(2-methyl-thiazol-4-yl)-vinyl]-9-oxo-non-3-enyl ester**

| | |
|---|---|
| Ausbeute: | 150 mg (0.219 mmol, 84%) leicht gelbliches Öl |
| $C_{33}H_{54}BrNO_5SSi$ | (684,84) |

**5-Hydroxy-1,1-dimethoxy-2,2,4-trimethyl-5-phenyl-pentan-3-one**

| | |
|---|---|
| Ausbeute: | 196 mg (70%) farbloses Öl |
| $C_{16}H_{24}O_4$ | (280.36) |

**5-Hydroxy-1,1-dimethoxy-2,2,4,6-tetramethyl-heptan-3-on**

| | |
|---|---|
| Ausbeute: | 206 mg (84%) farbloses Öl |
| MS (EI): | m/z (%) = 246 (0.02, $M^+$), 75 (100.0). |
| $C_{13}H_{26}O_4$ | (246.347) |

**5-(*Tert*-butyl-dimethyl-silanyloxy)-1,1-dimethoxy-2,2,4,6-tetramethyl-heptan-3-on**

| | |
|---|---|
| Ausbeute:: | 736 mg (88%) farbloses Öl |
| $C_{19}H_{40}O_4Si$ | (360.60) |

**3-Acetoxy-11-hydroxy-2,6,10-trimethyl-1-(2-methyl-thiazol-4-yl)-undeca-1,5-dien**

| | |
|---|---|
| Ausbeute: | 377 mg (1.031 mmol, 97%) |
| MS (ESI-MS): | m/z (%) = 753.3 (33) $[2M+Na]^+$, 388.1 (33) $[M+Na]^+$,366.1 (100) $[M+H]^+$, 306 (28) $[M+H-AcOH]^+$. |
| $C_{20}H_{31}NO_3S$ | (365.53) |

**3-Acetoxy-2,6,10-trimethyl-1-(2-methyl-thiazol-4-yl)-11-oxo-undeca-1,5-dien**

| | |
|---|---|
| Ausbeute: | 348 mg (0.957 mmol, 93%) farbloses Öl |
| MS (ESI-MS): | m/z (%) = 386.1 (48) $[M+Na]^+$, 364.1 (100) $[M+H]^+$, 304 (33) $[M+H-AcOH]^+$. |
| $C_{20}H_{29}NO_3S$ | (363,52) |

**1-Acetoxy-9-hydroxy-13,13-dimethoxy-4,8,10,12,12-pentamethyl-1-[1-methyl-2-(2-metbyl-thiazol-4-yl)-vinyl]-11-oxo-3-tridecene**

*Diastereomer* ***a***

| | |
|---|---|
| Ausbeute: | 24 mg (0.045 mmol, 32%) farbloses Öl |
| MS (CI, isobut.): | m/z (%) = 538 (0.86) $[M+H]^+$, 506 (0.55) [-OMe], 478 (1.14) [-AcOH], 446 (1.41) [-AcOH, MeOH], 364 (0.86), 304 (1.84), 279 (2.12), 265 (3.53), 253 (8.35), 241 (2.82), 225 (3.68), 210 (11.76), 168 (30.43), 164 (21.74), 128 (100.00). |
| HRMS: | berechnet für $C_{29}H_{48}NO_6S$ ($MH^+$): 538.32025 gefunden: 538.322854 |
| $C_{29}H_{47}NO_6S$ | (537.75) |

*Diastereomerengemisch* ***a*** *und* ***b***

(fortgesetzt)

| | |
|---|---|
| Ausbeute: | 25 mg (0.046 mmol, 33%) farbloses Öl |

**15-Acetoxy-3-(*tert*-butyl-dimethyl-silanyloxy)-7-hydroxy-4,4,6,8,12-pentamethyl-5,16-dioxo-heptadec-12-ensäure methyl ester**

| | |
|---|---|
| Ausbeute: | 52 mg (0.089 mmol, 64%) farbloses Öl |
| $C_{31}H_{56}O_8Si$ | (584.86) |

**15-Acetoxy-3-(*tert*-butyl-dimethyl-silanyloxy)-7-hydroxy-4,4,6,8,12,16-hexamethyl-17-(2-methyl-thiazol-4-yl)-5-oxo-heptadeca-12,16-diensäure methyl ester**

| | |
|---|---|
| Ausbeute: | 161 mg (0.60 mmol, 60%) farbloses Öl |
| MS (ESI-MS): | m/z (%) = 702.3 (100) $[M+Na]^+$, 680.3 (28) $[M+H]^+$, 620.3 (26) $[M+H-AcOH]^+$. |
| $C_{36}H_{61}NO_7$ | SSi(680,02) |

**15-Acetoxy-3-(*tert*-butyl-dimethyl-silanyloxy)-4,4,6,8,12,16-hexamethyl-17-(2-methyl-thiazol-4-yl)-5-oxo-7-triethylsilanyloxy-heptadeca-12,16-dienoic acid methyl ester**

| | |
|---|---|
| Ausbeute: | 138 mg (0.17 mmol, 100%) farbloses Öl |
| MS (ESI-MS): | m/z (%) = 794.4 (100) $[M+H]^+$, 756.4 (8), 734.4 (13) [-AcOH]. |
| $C_{42}H_{75}NO_7SSi_2$ | (794.28) |

**5-Acetoxy-pentan-4-on**

| | |
|---|---|
| Ausbeute: | 486 mg (3.4 mmol, 59%). |
| MS (CI): | m/z (%) = 43 (39), 45 (22), 57 (34), 91 (19), 119 (10), 145 (9), 173 (9), 189 (14), 190 (78), 191 (18), 233 (45), 246 (22), 251 (100), 252 (11), 307 (44). |

**5-Acetoxy-hexan-4-on**

| | |
|---|---|
| Ausbeute: | 2.00 g (12.6 mmol, 54%) |

**5-Acetoxy-heptan-4-on**

| | |
|---|---|
| Ausbeute | 415 mg (2.41 mmol, 90%) |
| MS(CI): | m/z (%) =143 (26), 157(11), 169 (13), 173 (100), 174 (12), 185 (18). |

**5-Acetoxy-octan-4-on**

Ausbeute: 1.6 g (8.59 mmol, 39%)

**5-Acetoxy-nonan-4-on**

Ausbeute: 419 mg (2.10 mmol, 29%) als farbloses Öl.
MS (CI): m/z (%) = 113 (28), 142 (12), 158 (15), 171 (29), 185 (16), 201 (100), 202 (12).

**Beispiel 4:**

***Tert*-butyl-2-acetoxy-acetoacetat**

*Procedure 1:* A solution of acetic acid (10.6 mL, 186 mmol) in DMF (169 mL) was neutralised with triethylamine (25.8 mL, 186 mmol) at 0°C. To the resulting triethylammoniumacetate solution was added *tert*-butyl-2-bromo-acetoacetate (**249**) (40.0 g, 169 mmol) dropwise at 0°C. Then the icebath was removed and the reaction mixture stirred for two hours at room temperature. Quenching with water (280 mL) was followed by threefold extraction with ethyl acetate (3×215 mL). The organic layers were combined and washed with water (3×215 mL) and brine (1×215 mL) and dried over $Na_2SO_4$. After filtration, the solvent was removed *in vacuo.* The resulting oil was twice distilled at 12 mbar and 128°C to give a colourless oil.

yield 20.7 g (96 mmol, 57%)

*Procedure* 2: To a suspension sodiumacetate (30.76 g, 375 mmol) in 250 mL DMF were added 59.27 g (250 mmol) of *tert*-butyl-2-bromo-acetoacetate (**249**). After stirring at ambient temperature for 90 minutes, 415 mL demi water were added and threefold extraction with 325 mL ethyl acetate followed. The combined organic layers were washed three times with 325 mL demi water and once with 325 mL brine, then dried over $Na_2SO_4$. After filtration, the solvent was removed *in vacuo.* The resulting oil was purified *via* Kugelrohrdistillation.

yield 36.52 g (169 mmol, 68%)
MS (CI): m/z (%) = 117 (19), 143 (12), 161 (100), 205 (43), 207 (12), 217 (18).
HRMS: calculated for $C_{10}H_{17}O_5$ ($MH^+$): 217.10760 found: 217.10460
$C_{10}H_{16}O_5$ (216.23)

**(1*E*,5*Z*)-1-(2-Methylthiazol-4-yl)-3-acetoxy-2,6,10-trimethyl-undeca-1,5,9-trien**

To a suspension of 19 mg (0.48 mmol) NaH (60% in mineral oil) in 2.5 mL THF were added dropwise 100μl (77 mg, 0.48 mmol) 1,1,1,3,3,3-hexamethyl-disilazane. The resulting solution was cooled to -63°C and a solution of 153 mg (0.44 mmol) tributyl-(2-methyl-thiazol-4-ylmethyl)-phosphonium chloride (9c) in 2.5 mL abs. THF was slowly added. Then 100 mg (0.40 mmol) of (5*Z*)-3-acetoxy-6,10-dimethylundeca-5,9-dien-2-one (251a) were added and the mixture was stirred for 30 min at - 63°C as well as another hour at 55°C.
After cooling down to ambient temperature, 10 mL saturated $NH_4Cl$ solution were added and the waterlayer was extracted three times with 10 mL diethylether. The combined organic layers were washed with demi water (3 □ 10 mL), with brine (1 □ 20 mL), then dried over $Na_2SO_4$, filtrated, and concentrated *in vacuo.* The remaining oil was purified *via* flash chromatography (column dimensions: 3.0×20.0 cm, ethyl acetate/petroleum ether = 1:6)

yield 93 mg (0.27 mmol, 67%)
$C_{20}H_{29}NO_2S$ (347.52)

**(4*E*)-2-Acetoxy-2-acetyl-5,9-dimethyl-deca-4,8-dienonsäure-*tert*-butylester**

*Tert*-butyl-2-acetoxy-acetoacetate (10.8 g, 50 mmol) was added dropwise to a stirred suspension of NaH (2.32 g, 58 mmol, 60% suspension in mineral oil) in DMF (100 mL) at 0°C. After the liberation of hydrogen gas stopped, geranylbromide (220d) (10.9 g, 50 mmol) was added dropwise at 0°C. Afterwards the icebath was removed and the

mixture stirred at room temperature for 16 hours. Then the mixture was diluted with ether (500 mL) and washed with water (3×200 mL) as well as with brine (1 × 200 mL). The solution was dried over $Na_2SO_4$, filtered and concentrated *in vacuo* to give a slightly yellow oil.

| | |
|---|---|
| yield | 17.6 g (50 mmol, quant.) |
| MS (EI): | m/z (%) = 352 (0.01, M+), 296, 236, 193, 167, 153, 69, 57, 43 (100.0). |
| $C_{20}H_{32}O_5$ | (352.47) |

**(5*E*)-*3*-Acetoxy-6,10-dimethyl-undeca-5,9-dien-2-on**

A solution of 17.62 g (50.0 mmol) (4*E*)-*2*-acetoxy-2-acetyl-5,9-dimehtyl-deca-4,8-dienoic-acid-*tert*-butylester (**250b**), 2.33 g (55.0 mmol) LiCl and 1.00 mL (55.0 mmol) demi water were stirred in 125 mL DMSO at 160˚C for six hours and 14 hours at ambient temperature. The brown solution was diluted with 400 mL demi water and the waterphase was extracted three times with 250 mL diethylether. The etherlayers were combined and washed three times with 250 mL demi water as well as once with 250 mL brine. Finally the etherlayer was dried over $Na_2SO_4$, filtrated and concentrated in *vacuo* to remain a redbrown oil.

| | |
|---|---|
| Yield | 11.66 g (46 mmol, 92%) |
| MS (ESI-MS): | m/z (%) =253.0 (100) $[M+H]^+$, 209 (42) $[M-COCH_3]^+$, 193 (23) $[M+H-AcOH]^+$. |
| $C_{15}H_{24}O_3$ | (252.35) |

**(5*E*)-3-acetoxy-11-hydroxy-6,10-dimethyl-undeca-5,9-dien-2-on**

127 mg (1.14 mmol) $SeO_2$ and 8.19 g *tert*-butyl hydroperoxide (70% solution in water) were stirred for 30 minutes in 15.0 mL CH2Cl2. Then 5.73 g (22.7 mmol) (5*E*)-*3*-acetoxy-6,10-dimethyl-undeca-5,9-dien-2-one (**251b**) were added and the reaction mixture was stirred at ambient temperature for 24 hours. Then 20 mL benzene were added and all volatile matter was removed *in vacuo.* The remaining oil was diluted with 100 mL diethylether and was washed twice with 30 mL of 0.2N NaOH solution as well as with 30 mL brine. The organic layer was dried over $Na_2SO_4$, filtrated and concetrated *in vacuo.* The product was isolated *via* column chromatography (column dimensions: 2.0×20.0cm, ethyl acetate/petroleum ether =1:5)

| | |
|---|---|
| yield | 1.63 (6.07 mmol, 27%) |
| $C_{15}H_{24}O_4$ | (268.35) |

**(5*E*)-Essigsäure 4,8-dimethyl-1-(2-methyl-[1,3]dioxolan-2-yl)-nona-3,7-dienylester**

5.05 g (20.0 mmol) (5*E*)-3-acetoxy-6,10-dimethyl-undeca-5,9-dien-2-one (**251b**), 1.32 mL (1.49 g, 24.0 mmol) ethylene glycol and 0.190 mg (1.00 mmol) *p*-toluenesulphonic acid were heatet at a dean stark trap under reflux in 50.0 mL benzene for five hours. The benzene solution was diluted with 200 mL diethylether, washed with saturated $NaHCO_3$ solution (2×100 mL), demi water (100 mL) and brine (100 mL). The organic layer was dried over $Na_2SO_4$, filtrated and concentrated in *vacuo.*

| | |
|---|---|
| yield | 5.78 g (19.5 mmol, 97%) yellow oil |
| $C_{17}H_{28}O_4$ | (296.40) |

**(5*E*)-Essigsäure-9-hydroxy-4,8-dimethyl-1-(2-methyl-[1,3]dioxolan-2-yl)-nona-3,7-dienyl ester**

To a suspension of 108 mg (0.975 mmol) $SeO_2$ in 13 mL $CH_2Cl_2$ were added 7.03 g (54.6 mmol) of a 70% *tert*-butyl hydroperoxide. After stirring for 30 min at ambient temperature 5.775 g (19.5 mmol) acetic acid 4,8-dimethyl-1-(2-methyl-[1,3]dloxolan-2-yl)-nona-3,7-dienyl ester (**262**) were added and the resulting suspension was stirred 38 hours at ambient temperaure. After addition of 20 mL all volatile matter was removed in *vacuo* and the remaining oil was purified by column chromatography.

| | |
|---|---|
| yield | 1.59 g (5.08 mmol, 26%) colourless oil |
| MS (ESI-MS): | m/z (%) = 313.0 (48) $[M+H]^+$. |

(fortgesetzt)

| | |
|---|---|
| $C_{17}H_{28}O_5$ | (312.40) |

**(5*E*)-Essigsäure-9-hydroxy-4,8-dimethyl-1-(2-methyl-[1,3]dioxolan-2-yl)-non-3-enyl ester**

1.474 g (4.71 mmol) acetic acid 9-hydroxy-4,8-dimethyl-1-(2-methyl-[1,3]dioxolan-2-yl)-nona-3,7-dienyl ester (**263**) was dissolved in 15.0 mL absolute methanol and 750 µl demi water were added. The solution was degassed with three freeze-thawcycles before 41 mg Ru(*R*-BINAP)$(OAc)_2$ were added and put in an autoclave under nitrogen atmosphere together with a magnetic stirring bar. After threefold purging with hydrogen (5.0 quality) the autoclave was set under a pressure of 100 bar hydrogen (5.0 quality) and stirred at room temperature for 25h.
The hydrogen pressure was released and the solution concentrated *in vacuo.* The obtained brown oil was purified by flash chromatography (column dimensions: 4.0×20.0 cm, ethyl acetate/petroleum ether = 1:2).

| | |
|---|---|
| yield | 1.185 g (3.77 mmol, 80%) colourless oil |
| $C_{17}H_{30}O_5$ | (314.42) |

**(5*E*)-Essigsäure-9-(4-methoxy-benzyloxy)-4,8-dimethyl-1-(2-methyl-[1,3] dioxolan-2-yl)-non-3-enyl ester**

849 mg (2.700 mmol) acetic acid 9-hydroxy-4,8-dimethyl-1-(2-methyl-[1,3]dioxolan-2-yl)-non-3-enyl ester (**264**) were dissolved in 10.0 mL $CH_2Cl_2$ and 48 mg (0.19 mmol) PPTS as well as 1.526 g (5.400 mmol) *para*-methoxy benzyl trochloroimidate (**268**) were added and stirred 18 hours at ambient temperature. Then the solution was diluted with 25 mL diethylether and 25 mL petroleumether, washed with demi water (2×20 mL) and with brine (20 mL), dried over $Na_2SO_4$, filtrated and concentrated in *vacuo.* The remaining yellow oil was purified *via* flash chromatography (column dimensions: 3.5×20.0 cm, ethyl acetate/petroleum ether = 1:4).

| | |
|---|---|
| yield | 796 mg (1.84 mmol, 68%) |
| MS (CI, isobut.): | m/z (%) = 435 (0.3) $[M+H]^+$, 375 (0.3), 313 (0.7), 253 (0.4), 241 (1.0), 193 (0.6), 163 (0.9), 121 (52), 87 (51). |
| $C_{25}H_{38}O_6$ | (434.57) |

(1 **0*S*)-3-Acetoxy-11-hydroxy-6,10-dimethyl-5-undecen-2-on**

1.032 g (2.79 mmol) (9*S*)-(4*Z*)-2-acetoxy-2-acetyl-5,9-dimehtyl-10-hydroxy-deca-4-enoic-acid-*tert*-butylester (**266**) were dissolved in 28 mL $CH_2Cl_2$ and 2.80 mL TFA were added. After stirring for two hours at room temperature, all volatile matter was removed *in vacuo* and the reaming oil was dissolved in 28 mL methanol. Then 5.6 mL saturated $NaHCO_3$ - solution was added and the suspension was stirred for 140 minutes at ambient temperature before dilution with 200 mL ether was performed. The organic layer was washed two times with 50 mL demi water as well as once with 50 mL brine. After drying over $Na_2SO_4$, filtration and removal of the solvents *in vacuo,* a slightly yellow oil was obtained. Purification was achieved by flash chromatography (column dimensions: 2.0x20.0 cm, ethyl acetate/petroleum ether = 2:3)

| | |
|---|---|
| yield | 568 mg (2.10 mmol, 75%) colourless oil |
| MS (ESI-MS): | m/z (%) = 563.3 (100) $[2M+Na]^+$, 293.0 (54) $[M+Na]^+$, 271.1 (7) $[M+H]^+$. |
| $C_{15}H_{26}O_4$ | (270.36) |

**(10*S*)-3-Acetoxy-11-*tert*-butyl-dimethylsilyloxy-6,10-dimetbyl-5-undecen-2-on**

528 mg (1.95 mmol) 3-acetoxy-11-hydroxy-6,10-dimethyl-5-undecen-2-one (**11a**) were dissolved in 10.0 mL absolute $CH_2Cl_2$. After addition of 541 µl (395 mg, 3.90 mmol) triethylamine and 12 mg (0.10 mmol) of DMAP, the solution was cooled with an ice bath. After stirring for five minutes 368 mg (2.44 mmol) of TBDMSCI were added at once and the resulting solution was stirred at 0˚C for two hours and additional 14 hours at room temperature. The resulting colourless suspension was again cooled to 0˚C before 460 µl of methanol were added. After stirring for 30 min at 0˚C, all solvents were removed *in vacuo.* Then 15 mL ether as well as 15 mL saturated $NH_4Cl$ - solution were added and after intensive stirring and separation, the aqueous phase was extracted twice with 10 mL ether. The combined organic layers were washed with 15 mL brine, dried over $Na_2SO_4$, filtered and concentrated *in vacuo.* The obtained oil was

purified by means of flash chromatography (column dimensions: 2.0×20.0 cm, ethyl acetate/petroleum ether = 1:10).

| | |
|---|---|
| yield | 597 mg (1.55 mmol, 80%) colourless oil |
| $R_f$-value | 0.32 |
| MS (CI, isobut.): | m/z (%) = 385 (13) [M+H]$^+$, 327 (13), 267 (26), 253 (6), 193 (40), 175 (62), 117 (100). |
| HRMS: | calculated for $C_{21}H_{41}O_4Si$ (MH$^+$): 385.27740 found: 385.278524 |
| $C_{21}H_{40}O_4Si$ | (384.63) |

**(10*S*)-3-Acetoxy-11-(*tert*-butyl-dimethyl-silanyloxy)-2,6,10-trimethyl-1 -(2-methyl-thiazol-4-yl)-undeca-1,5-dien**

A solution of 1092 mg (3.12 mmol) tributyl-(2-methyl-thiazol-4-ylmethyl)-phosphonium chloride (**9c**) in 21.0 mL abs. THF was cooled to -63°C and 1.56 mL (3.12 mmol) of a 2.0M solution of sodium-hexamethyldisilazid were added dropwise. After stirring for 15 min, a solution of 1000 mg (2.60 mmol) 3-acetoxy-11-*tert*-butyl-dimethylsilyloxy-6,10-dimethyl-5-undecen-2-one (**11b**) in 8.0 mL THF was added slowly. After 15 min stirring at -63°C, the solution was heated to 55°C and stirred for an additional hour. Then the heating bath was removed and 42 mL saturated $NH_4Cl$- solution were added. Threefold extraction with 20 mL ether, washing the combined etherlayers with demi water (3×20 mL) and with brine (1×30 mL), drying over $Na_2SO_4$, filtration and removal of the solvent *in vacuo* gave a brown oil. Purification was achieved *via* flash chromatography (column dimensions: 3.5×20.0 cm, ethyl acetate/petroleum ether = 1:4).

| | | | | | |
|---|---|---|---|---|---|
| yield | 1169 mg (2.44 mmol, 94%) colourless oil | | | | |
| MS (CI, isobut.): | m/z (%) = 480 (9) [M+H]$^+$, 422 (22), 420 (51), 362 (5), 210 (>100), 178 (35), 168 (>100), 164 (72), 128 (55), 117 (65). | | | | |
| HRMS: | calculated for $C_{26}H_{46}NO_3SSi$ (MH$^+$): 480.295912 found: 480.29678 | | | | |
| EA: | calculated | C 65.09 | H 9.45 | N 2.92 | S 6.68 |
| | found | C 64.79 | H 9.53 | N 2.96 | S 6.54 |
| $C_{26}H_{45}NO_3$ | SSi(479.79) | | | | |

**(10*S*)-3-Acetoxy-11-(tetrahydro-pyran-2-yloxy)-6,10-dimethyl-5-undecen-2-one**

*Procedure 1:* 324 mg (1,20 mmol) of a crude product of 3-acetoxy-11-hydroxy-6,10-dimethyl-5-undecen-2-one (**11a**) and 219 µl (202 mg, 2,40 mmol) dihydropyrane were dissolved in 8,5 mL $CH_2Cl_2$. After addition of 30 mg (0.12 mmol) pyridinium paratoluenesulphonate, the solution was stirred at ambient temperature for 18 hours. Then 25 mL diethylether were added, the organic layer washed once with 25 mL halfconcentrated NaCl-solution and dried over $Na_2SO_4$. After filtration and evaporation of the solvents *in vacuo,* the remaining oil was purified by flash chromatography (column dimensions: 2.0×20.0cm, ethyl acetate/petroleum ether = 1:4).

yield 176 mg (0,50 mmol, 41 %) colourless oil

*Procedure 2:* 508 mg (1.88 mmol) of 3-acetoxy-11-hydroxy-6,10-dimethyl-5-undecen-2-one (**11a**) and 344 µl (320 mg, 3.80 mmol) dihydropyrane were dissolved in 5.0 mL $CH_2Cl_2$. After addition of 165 mg (1.90 mmol) LiBr the mixture was stirred four hours at ambient temperature. After addition of 20.0 mL diethylether, the organic layer was washed once with 5.0 mL water and once with 5.0 mL brine. The organic layer was dried over $Na_2SO_4$, filtrated and concentrated *in vacuo* and the remaining oil was purified *via* flash chromatography (column dimensions: 2.0×20.0 cm, ethyl acetate/petroleum ether = 1:4).

| | |
|---|---|
| yield | 429 mg (1.21 mmol, 64%) colourless oil |
| $C_{20}H_{34}O_5$ | (354,48) |

**(10*S*)-3-Acetoxy-11-(tetrahydro-pyran-2-yloxy)-2,6,10-trimethyl-1-(2-methylthiazol-4-yl)-undeca-1,5-dien**

A solution of 200 mg (0.57 mmol) tributyl-(2-methyl-thiazol-4-ylmethyl)-phosphonium chloride (**9c**) in 5.0 mL abs. THF was cooled to -63°C and 286 µl (0.57 mmol) of a 2.0M solution of sodium-hexamethyldisilazid were added dropwise. After stirring for 15 min, a solution of 167 mg (0.48 mmol) 3-acetoxy-11-(2-tetrahydropyranyloxy)-6,10-dimethyl-5-undecen-2-one (**11c**) in 2.0 mL THF was added slowly. After 15 min stirring at -63°C, the solution was heated to 55°C and stirred for an additional hour. Then the heating bath was removed and 10 mL saturated $NH_4Cl$- solution were added.

Threefold extraction with 10 mL ether, washing the combined etherlayers with demi water (3×10mL) and with brine (1×10 mL), drying over $Na_2SO_4$, filtration and removal of the solvent *in vacuo* gave a brown oil. Purification was achieved *via* flash chromatography (column dimensions: 2.0×20.0 cm, ethyl acetate/petroleum ether = 1:4).

| | |
|---|---|
| yield | 216 mg (0.48 mmol, quant.) colourless oil |
| $C_{25}H_{39}NO_4S$ | (449,65) |

**(2*E*,6*Z*)-3,7-Dimethyt-8-(tetrahydro-pyran-2-yloxy)-octa-2,6-dien-1-ol**

8.66 g (40.8 mmol) 1-acetoxy-8-hydroxy-3,7-dimethyl-octa-2,6-dien (**257a**) and 7.44 mL (6.86 g, 81.6 mmol) DHP were dissolved in 20 mL $CH_2Cl_2$. After addition of 513 mg (2.04 mmol) PPTS, the mixture was stirred seven hours at ambient temperature. After removal of all volatile matter *in vacuo,* the obtained oil was dissolved in 200 mL methanol and 14.96 g (151 mmol) $K_2CO_3$ were added and the suspension was stirred at ambient temperature for one hour. After addition of 500 mL demi water, fivefold extraction with 150 mL diethylether was performed. The organic layers were combined and washed with demi water (2□100 mL) as well as brine (1□100 mL), dried over $Na_2SO_4$, filtrated and concentrated *in vacuo.*

| | |
|---|---|
| yield | 10.27 g (40.4 mmol, 99%) yellow oil |
| $C_{15}H_{26}O_3$ | (254.37) |

**1-Chloro-3,7-Dimethyl-8-(tetrahydro-pyran-2-yloxy)-2,6-octadien**

In a dry 500 mL Schlenk flask, 5.29 g (39.6 mmol) NCS is were dissolved in 185 mL dry $CH_2Cl_2$ and the solution was then cooled to -5˚C before 3.17 mL (2.68 g, 43.2 mmol) dimethylsulfide were added dropwise. After stirring for five minutes at - 5˚C, the resulting white suspension was cooled to -63˚C and 9.16 g (36.0 mmol) 3,7-dimethyl-8-(tetrahydro-pyran-2-yloxy)-octa-2,6-dien-1-ol (**271**) were added dropwise. After the addition was complete, the reaction mixture was allowed to reach -5˚C again and was stirred one hour at that temperature in which the white suspension tumed slowly into a colourless solution. Then 90 mL saturated ammoniumchloride solution were added, the layers separated and the aqueous layer extracted twice with 75 mL diethylether. The organic layers were combined and washed twice with 75 mL brine, then dried over $Na_2SO_4$, filtered and concentrated *in vacuo.* A slightly yellow oil was obtained in high purity.

| | |
|---|---|
| yield | 9.80 g (36.0 mmol, quant.) |
| $C_{15}H_{25}ClO_2$ | (272.81) |

**2-Acetoxy-2-acetyl-5,9-dimethyl-10-(tetrahydro-pyran-2-yloxy)-deca-4,8-dienonsäure *tert*-butyl ester**

*Tert*-butyl-2-acetoxy-acetoacetate (**219a**) (3.80 g, 17.6 mmol) was added dropwise to a stirred suspension of NaH (60% suspension in mineral oil, 0.870 g, 21.8 mmol) in DMF (35 mL) at 0˚C. After the liberation of hydrogen gas stopped, 1-chloro-3,7-dimethyl-8-(tetrahydro-pyran-2-yloxy)-2,6-octadiene (**272**) (4.57 g, 16.7 mmol) was added dropwise at 0˚C. After stirring for one hour at 0˚C the icebath was removed and the mixture stirred at room temperature for three hours. Then the mixture was diluted with ether (225 mL) and washed with water (3×75 mL) as well as with brine (1×75 mL). The solution was dried over $Na_2SO_4$, filtered and concentrated *in vacuo,* to give a slightly yellow oil.

| | |
|---|---|
| yield | 6.67 g (14.7 mmol, 88%) |
| MS (ESI-MS): | m/z (%) = 475.3 (68) $[M+Na]^+$, 453.2 (23) $[M+H]^+$, 269.2 (80) $[M+H-DHP]^+$, 313.1 (51) $[M+H-C_4H_8-DHP]^+$, 295.1 (100) $[M+H-C_4H_8-DHP-H_2O]^+$. |
| $C_{25}H_{40}O_7$ | (452,58) |

**11-(*Tert*-butyl-dimethyl-silanyloxy)-3-hydroxy-6,10-dimethyl-undec-5-en-2-on**

1.943 g (5.05 mmol) 3-acetoxy-11-(*tert*-butyl-dimethylsilyloxy)-6,10-dimethyl-5-undecen-2-one (**11b**) were dissolved in 20.0 mL methanol and 400 µl of a saturated potassium carbonate solution were added. After stirring at ambient temperature for 14 minutes, 30 mL brine were added and fivefold extraction with 30 mL diethylether followed. (The hydrolysis actually was already complete after five minutes according to TLC. Workup right on time is crucial, for the yield drop drastically if the reaction is not stopped in time. Stirring for example for ninety minutes resulted in dramatic

drop of the yield to 43%!!!) The combined organic layers were washed with 50 mL brine and dried over $Na_2SO_4$. After filtration and removal of the solvent *in vacuo,* the remaining oil was purified *via* flash chromatography (column dimensions: 2.0×20.0 cm, ethyl acetate/petroleum ether = 1:4).

| | |
|---|---|
| yield | 1.686 g (4.92 mmol, 97%) |
| $C_{19}H_{38}O_3Si$ | (342.59) |

**(3*S*)-6-Bromo-3-(*tert*-butyl-dimethyl-silanyloxy)-4,4-dimethyl-5-oxo-heptansäure-4,8-dimethyl-1-[1-methyl-2-(2-methyl-thiazol-4-yl)-vinyl]-9-oxo-non-3-enyl ester**

180 mg (0.262 mmol) (3*S*)-6-bromo-3-(*tert*-butyl-dimethyl-silanyloxy)-4,4-dimethyl-5-oxo-heptanoic acid 9-hydroxy-4,8-dimethyl-1-[1-methyl-2-(2-methylthiazol-4-yl)-vinyl]-non-3-enyl ester (**277b**) were dissolved in 5.30 mL $CH_2Cl_2$ and 1.72 mL DMSO at 0°C and 212 µl (155 mg, 1.53 mmol) triethylamine were added. After addition of 195 mg (1.223 mmol) $SO_3$•Pyridine - complex the mixture was stirred 60 min at 0°C. Dilution with 40 mL diethylether was followed by twofold washing with 10 mL demi water and with brine (2□10 mL). The organic layer was the dried over $Na_2SO_4$, filtered and concentrated *in vacuo* to give a slightly yellow oil which was used in the following reaction step without further purification.

| | |
|---|---|
| yield | 150 mg (0.219 mmol, 84%) slightly yellow oil |
| $C_{33}H_{54}BrNO_5SSi$ | (684,84) |

**Macrolacton Strategie**

**5-Hydroxy-1,1-dimethoxy-2,2,4-trimethyl-5-phenyl-pentan-3-one**

To a solution of 298 mg (2.42 mmol) $CrCl_2$ and 20 mg (0.15 mmol) LiI in 6.0 mL abs. THF were added 117 µl (1.1 mmol) benzaldehyde and 253 mg (1.0 mmol) 4-bromo-1,1-dimethoxy-2,2-dimethyl-pentan-3-one (**225**). After stirring at ambient temperature for 30 minutes the brown solution was quenched with 5.0 mL brine. The aqueous phase was extracted three times with 5.0 mL diethylether. The organic layers were combined, washed with conc. $NH_4Cl$ solution (2□5.0 mL), dried over $MgSO_4$, filtrated and concentrated *in vacuo.* The remaining green oil (196 mg) was purified *via* flash chromatography (column dimensions: 3.5×20.0 cm, ethyl acetate/petroleum ether = 1:6).

| | |
|---|---|
| yield: | 196 mg (70%) colourless oil |
| $C_{16}H_{24}O_4$ | (280.36) |

**5-Hydroxy-1,1-dimethoxy-2,2,4,6-tetramethyl-heptan-3-on**

To a solution of 298 mg (2.42 mmol) $CrCl_2$ and 29 mg (0.22 mmol) LiI in 6.0 mL abs. THF were added 100 µl (1.1 mmol) isobutyric aldehyde and 253 mg (1.0 mmol) 4-bromo-1,1-dimethoxy-2,2-dimethyl-pentan-3-one (**225**). After stirring at ambient temperature for one hour the brown solution was quenched with 5.0 mL brine. The aqueous phase was extracted three times with 5.0 mL of a diethylether-pentane mixture (5:1). The organic layers were combined, washed with conc. $NH_4Cl$ solution (3□5.0 mL), dried over $MgSO_4$, filtrated and concentrated *in vacuo.* The remaining colourless oil (206 mg) was purified *via* flash chromatography (column dimensions: 3.5×20.0 cm, ethyl acetate/petroleum ether = 1:8).

| | |
|---|---|
| yield: | 206 mg (84%) colourless oil |
| MS (EI): | m/z (%) = 246 (0.02, $M^+$), 75 (100.0). |
| $C_{13}H_{26}O_4$ | (246.347) |

**5-(*Tert*-butyl-dimethyl-silanyloxy)-1,1-dimethoxy-2,2,4,6-tetramethyl-heptan-3-on**

To a solution of 571 mg (2.31 mmol) 5-hydroxy-1,1-dimethoxy-2,2,4,6-tetramethylheptan-3-one (**281**) and 539 µl (4.63 mmol) 2,6-lutidine in 2.3 mL $CH_2Cl_2$ at 0°C were added 797 µl (3.47 mmol) *tert*-butyl-dimethylsilyltriflate dropwise. After two hours stirring at 0°C, 2.0 mL 0.2N NaOH solution was added and the mixture stirred ten minutes at ambient temperature. After addition of 30 mL CH2Cl2, the organic layer was washed twice with 0.2N HCl solution as well as

twice with 30 mL brine. The organic layer was dried over $MgSO_4$, filtrated and concentrated *in vacuo.* The obtained yellow oil was purified y flash chromatography (column dimensions: 3.5×20.0 cm, ethyl acetate/petroleum ether= 1:10).

| | |
|---|---|
| yield: | 736 mg (88%) colourless oil |
| $R_f$-value | 0.53 (Essigester/Petrolether, V:V = 1:10) |
| $C_{19}H_{40}O_4Si$ | (360.60) |

**3-Acetoxy-11-hydroxy-2,6,10-trimethyl-1-(2-methyl-thiazol-4-yl)-undeca-1,5-dien**

*Procedure 1 (from THP-ether):* A solution of 238 mg (0.53 mmol) 3-acetoxy-11-(tetrahydro-pyran-2-yloxy)-2,6,10-trimethyl-1-(2-methyl-thiazol-4-yl)-undeca-1,5-diene (**270b**) and 13 mg (0.053 mmol) pyridinium paratoluenesulphonate in 5.0 mL 96% ethanol was stirred at 55˚C for eight hours. After concentration *in vacuo,* the residue was dissolved in 25 mL diethylether. The organic layer was washed with 5% $NaHCO_3$ (2□10 mL) and with brine (1□101), then dried over $Na_2SO_4$, filtrated and concentrated *in vacuo.* The remaining oil was purified *via* flash chromatography (column dimensions: 2.0×20.0 cm, ethyl acetate/petroleum ether = 1:2).

yield 99 mg (0.27 mmol, 51%) colourless oil

*Procedure 2 (from TBS-ether):* 512 mg (1.067 mmol) 3-acetoxy-11-(*tert*-butyldimethyl-silanyloxy)-2,6,10-trimethyl-1-(2-methyl-thiazol-4-yl)-undeca-1,5-diene (**270a**) were dissolved in 15.0 mL $CH_2Cl_2$ and 15.0 mL MeOH and cooled to 0˚C. After addition of 248 mg (1.067 mmol) CSA the solution was stirred for 4.0 hours at 0˚C. Then 222μl (162 mg, 1.601 mmol) triethylamine were added, the solvents removed *in vacuo* and the remaining oil was purified by flash chromatography (column dimensions: 2.0x20.0 cm, ethyl acetate/petroleum ether= 1:1),

| | |
|---|---|
| yield | 377 mg (1.031 mmol, 97%) |
| MS(ESI-MS): | m/z (%) = 753.3 (33) $[2M+Na]^+$, 388.1 (33) $[M+Na]^+$,366.1 (100) $[M+H]^+$, 306 (28) $[M+H-AcOH]^+$. |
| $C_{20}H_{31}NO_3S$ | (365.53) |

**3-Acetoxy-2,6,10-trimethyl-1-(2-methyl-thiazol-4-yl)-11-oxo-undeca-1,5-dien**

*Procedure 1 (Swern-oxidation):* 26 μl (38 mg, 0.30 mmol) oxalyl chloride were dissolved in 2.0 mL abs. $CH_2Cl_2$ and cooled to -63˚C. After addition of a solution of 42 μl (46 mg, 0.59 mmol) abs. DMSO in 0.5 mL abs. $CH_2Cl_2$, the mixture was stirred for 10 min at -63˚C and a solution of 99 mg (0.27 mmol) 3-acetoxy-11-hydroxy-2,6,10-trimethyl-1-(2-methyl-thiazol-4-yl)-undeca-1,5-diene (**270e**) in 1.0 mL abs. $CH_2Cl_2$ was added dropwise. Then the mixture was stirred at -63˚C for an additional 30 minutes before 187 μl (137 mg, 1.35 mmol) triethylamine were added. Finally the mixture was allowed to reach ambient temperature and stirred for one more hour.
Then 5.0 mL water were added, the layers separated and the water layer extracted twice with 5.0 mL $CH_2Cl_2$. The combined organic layers were washed with saturated $NH_4Cl$-solution (2□4.5 mL), with water (1□4.5ml) and with brine (1□4.5 mL). After drying over $Na_2SO_4$, filtration and concentrating *in vacuo,* the obtained oil was purified by flash chromatography (column dimensions: 2.0×20.0 cm, ethyl acetate/petroleum ether = 1:2).

yield 74 mg (0.204 mmol, 75%) colourless oil

*Procedure 2 ($SO_3$•Py/DMSO):* A solution of 377 mg (1.031 mmol) 3-acetoxy-11-hydroxy-2,6,10-trimethyl-1-(2-methyl-thiazol-4-yl)-undeca-1,5-diene (**270e**) and 715 μl (522 mg, 5.157 mmol) triethylamine in 12.5 mL abs. $CH_2Cl_2$ and 4.2 mL DMSO was cooled to 0˚C. Then 657 mg (4.123 mmol) of $SO_3$•Py complex were added at once and the resulting solution was stirred 30 min at 0˚C. Finally, 100 mL diethylether were added and the organic layer was washed twice with 50 mL demi water as well as once with 50 mL brine. After drying over $Na_2SO_4$, filtration and concentrating *in vacuo,* the obtained oil was purified by flash chromatography (column dimensions: 2.0×20.0 cm, ethyl acetate/petroleum ether = 1:2).

| | |
|---|---|
| yield | 348 mg (0.957 mmol, 93%) colourless oil |
| MS (ESI-MS): | m/z (%) = 386.1 (48) $[M+Na]^+$, 364.1 (100) $[M+H]^+$, 304 (33) $[M+H-AcOH]^+$. |
| $C_{20}H_{29}NO_3S$ | (363,52) |

**1-Acetoxy-9-hydroxy-13,13-dimethoxy-4,8,10,12,12-pentamethyl-1-[1-methyl-2-(2-methyl-thiazol-4-yl)-vinyl]-11-oxo-3-tridecene**

To a suspension of 39 mg (0.316 mmol) $CrCl_2$ and 40 mg (0.299 mmol) LiI in 1.0 mL absolute THF was added a solution of 51 mg (0.140 mmol) 3-acetoxy-2,6,10-trimethyl-1-(2-methyl-thiazol-4-yl)-11-oxo-undeca-1,5-diene (**8b**) in 1.0 mL absolute THF. After five minutes stirring at ambient temperature, 37 mg (0.147 mmol) 4-bromo-1,1-dimethoxy-2,2-dimethyl-pentan-3-one (**225**) were added. After stirring for 3h 40min at ambient temperature, 2.5 mL brine were added and stirring was continued for five minutes. The waterphase was extracted five times with 2.0 mL of a pentane-diethylether-mixture (1:5) and the combined organic layers were washed three times with 2.0 mL saturated $NH_4Cl$ solution. After drying over $Na_2SO_4$, filtration and concentrating *in vacuo,* the obtained oil was purified by flash chromatography (column dimensions: 1.0×20.0 cm, diethylether/petroleum ether = 5:4). Two fractions were collected, fraction A containing only the major diastereomer, fraction B containing the major and the minor diastereomer.

*Diastereomer **a***

| | |
|---|---|
| yield | 24 mg (0.045 mmol, 32%) colourless oil |
| MS (CI, isobut.): | m/z (%) = 538 (0.86) $[M+H]^+$, 506 (0.55) [-OMe], 478(1.14) [-AcOH], 446 (1.41) [-AcOH,-MeOH], 364 (0.86), 304 (1.84), 279 (2.12), 265 (3.53), 253 (8.35), 241 (2.82), 225 (3.68), 210 (11.76), 168 (30.43), 164 (21.74), 128 (100.00). |
| HRMS: | calculated for $C_{29}H_{48}NO_6S$ ($MH^+$): 538.32025 found: 538.322854 |
| $C_{29}H_{47}NO_6S$ | (537.75) |

*mixture of Diastereomers **a** and **b***

| | |
|---|---|
| yield | 25 mg (0.046 mmol, 33%) colourless oil |

**15-Acetoxy-3-(*tert*-butyl-dimethyl-silanyloxy)-7-hydroxy-4,4,6,8,12-pentamethyl-5,16-dioxo-heptadec-12-ensäure methyl ester**

To a suspension of 79 mg (0.640 mmol) $CrCl_2$ and 41 mg (0.306 mmol) LiI in 2.0 mL absolute THF were added 51 mg (0.140 mmol) 3-acetoxy-6,10-dimethyl-11-oxo-5-undecen-2-one (**285**). After ten minutes stirring at ambient temperature, a solution of 37 mg (0.147 mmol) (3*S*)-6-bromo-3-(*tert*-butyl-dimethyl-silanyloxy)-4,4-dimethyl-5-oxo-heptanoic acid methyl ester (**235**) in 1.0 mL THF was added. After stirring for three hours at ambient temperature, 3.0 mL brine were added and stirring was continued for five minutes. The waterphase was extracted five times with 3.0 mL of a pentane-diethylether-mixture (1:5) and the combined organic layers were washed three times with 3.0 mL saturated $NH_4Cl$ solution. After drying over $Na_2SO_4$, filtration and concentrating *in vacuo,* the obtained oil was purified by flash chromatography (column dimensions: 2.0×20.0 cm, diethylether/petroleum ether = 1:1).

| | |
|---|---|
| yield | 52 mg (0.089 mmol, 64%) colourless oil |
| $C_{31}H_{56}O_8Si$ | (584.86) |

**15-Acetoxy-3-(*tert*-butyl-dimethyl-silanyloxy)-7-hydroxy-4,4,6,8,12,16-hexamethyl-17-(2-methyl-thiazol-4-yl)-5-oxo-heptadeca-12,16-diensäure methyl ester**

To a suspension of 240 mg (1.95 mmol) $CrCl_2$ and 125 mg (0.93 mmol) in 10.0 mL absolute THF was added 322 mg (0.886 mmol) 3-acetoxy-2,6,10-trimethyl-1-(2-methyl-thiazol-4-yl)-11-oxo-undeca-1,5-diene (**8b**) in 1.0 mL absolute THF and 384 mg (0.971 mmol) (3*S*)-6-bromo-3-(*tert*-butyl-dimethyl-silanyloxy)-4,4-dimethyl-5-oxo-heptanoic acid methyl ester (**235**) at ambient temperature. After stirring for 2h15min at ambient temperature, 12 mL brine were added and stirring was continued for five minutes. The waterphase was extracted five times with 15 mL of a pentane-diethylether-mixture (1:5) and the combined organic layers were washed twice with 20 mL demi water and twice with 20 mL brine. After drying over $Na_2SO_4$, filtration and concentrating *in vacuo,* the obtained oil was purified by flash chromatography (column dimensions: 2.0x20.0 cm, diethylether/petroleum ether = 5:4).

| | |
|---|---|
| yield | 161 mg (0.60 mmol, 60%) colourless oil |
| MS (ESI-MS): | m/z (%) = 702.3 (100) $[M+Na]^+$, 680.3 (28) $[M+H]^+$, 620.3 (26) $[M+H-AcOH]^+$. |
| $C_{36}H_{61}NO_7$ | SSi(680,02) |

**15-Acetoxy-3-(*tert*-butyl-dimethyl-silanyloxy)-4,4,6,8,12,16-hexamethyl-17-(2-methyl-thiazol-4-yl)-5-oxo-7-triethylsilanyloxy-heptadeca-12,16-dienoic acid methyl ester**

A solution of 115 mg (0.17 mmol) 15-acetoxy-3-(*tert*-butyl-dimethyl-sitanyloxy)-7-hydroxy-4,4,6,8,12,16-hexamethyl-17-(2-methyl-thiazol-4-yl)-5-oxo-heptadeca-12,16-dienoic acid methyl ester (**289**) and 36 mg (0.50 mmol) imidazole in 1000□1 absolute DMF was cooled to 0˚C. After addition of 84□1 (0.50 mmol) triethylsilylchloride, the icebath was removed and the solution was stirred for 3h15min at ambient temperature before 5.0 mL diethylether were added. The organic layer was washed with demi water (2□1000□1) as well as with brine (1□1000□1), dried over $Na_2SO_4$, filtrated and concentrated *in vacuo.* The remaining oil was purified *via* flash chromatography (column dimensions: 1.0×20.0 cm, diethylether/petroleum ether = 1:1).

| | |
|---|---|
| yield | 138 mg (0.17 mmol, 100%) colourless oil |
| MS (ESI-MS): | m/z (%) = 794.4 (100) $[M+H]^+$, 756.4 (8), 734.4 (13) [-AcOH]. |
| $C_{42}H_{75}NO_7SSi_2$ | (794.28) |

**8.6 Lipase Resolution of Acyloin Esters**

**5-Acetoxy-pentan-4-on**

According to the general alkylation method, *tert*-butyl-2-acetoxy-aceto-acetate (**219a**) (1.08 g, 5.00 mmol), NaH (156 mg, 6.5 mmol) and ethylbromide (**auch propyl** + **pentyl**) (373 µL, 549 mg, 5.00 mmol) were reacted in DMF (10 mL) to give 2-acetoxy-2-acetylbutanoic-acid-*tert*-butylester (**250g**) (970 mg, 3.97 mmol, 79%) as a slightly yellow oil. Then, **250g** (1.40 g, 5.73 mmol) and 109 mg (0.57 mmol) *p*-TsOH•$H_2O$ were stirred in 18.0 mL benzene according to decarboxylation method A. Kugelrohr distillation gave 486 mg (3.4 mmol, 59%) of **251g** as a colourless oil. MS (CI): m/z (%) = 43 (39), 45 (22), 57 (34), 91 (19), 119 (10), 145 (9), 173 (9), 189 (14), 190 (78), 191 (18), 233 (45), 246 (22), 251 (100), 252 (11), 307 (44). HRMS: calculated for $C_{17}H_{23}O_5$ ($MH^+$): 307.15454 found: 307.15070

**5-Acetoxy-hexan-4-on**

According to the general alkylation method, *tert*-butyl-2-acetoxy-aceto-acetate (**219a**) (7.12 g, 32.9 mmol), NaH (900 mg, 37.5 mmol) and propylbromide (4.05 g, 32.9 mmol) were reacted in DMF (64 mL) to give 2-acetoxy-2-acetyl-pentanoicacid-tert-butylester (**250h**) (6.00 g, 23.2 mmol, 71%) as a slightly yellow oil after Kugelrohr distillation at 160˚C and 1.0 mbar. Then, **250h** (6.00 g, 23.2 mmol) and 393 mg (2.07 mmol) *p*-TsOH•$H_2O$ were stirred in 60 mL benzene according to decarboxylation method A. Kugelrohr distillation at 1.0 mbar and 150˚C, followed by column chromatography (diethylether:petroleum ether = 1:8) gave **251h** (2.00 g, 12.6 mmol, 54%) as a colourless oil.

**5-Acetoxy-heptan-4-on**

According to the general alkylation method, *tert*-butyl-2-acetoxy-aceto-acetate (**219a**) (1.08 g, 5.00 mmol), NaH (156 mg, 6.5 mmol) and butylbromide (538 µL, 685 mg, 5.00 mmol) were reacted in DMF (10 mL) to give 2-acetoxy-2-acetylhexanoic-acid-*tert*-butylester (**250i**) (1026 mg, 3.77 mmol, 75%) as a slightly yellow oil. Then, **250i** (731 mg, 2.68 mmol) and 51 mg (0.27 mmol) *p*-TsOH•$H_2O$ were stirred in 9.0 mL benzene according to decarboxylation method A. Kugelrohr distillation at 1.0 mbar and 60˚C gave **251i** (415 mg, 2.41 mmol, 90%) as a colourless oil.

| | | |
|---|---|---|
| MS (CI): | m/z (%) = 143 (26), 157 (11), 169 (13), 173 (100), 174 (12), 185 (18). | |
| HRMS: | calculated for $C_9H_{17}O_3$ ($MH^+$): 173.11777 | found: 173.11535 |

**5-Acetoxy-octan-4-on**

According to the general alkylation method, *tert*-butyl-2-acetoxy-aceto-acetate (**219a**) (7.12 g, 32.9 mmol), NaH (900 mg, 37.5 mmol) and pentylbromide (4.97 g, 32.9 mmol) were reacted in DMF (64 mL) to give 2-acetoxy-2-acetyl-heptanoicacid-*tert*-butylester (**250j**) (6.40 g, 22.3 mmol, 68%) as a slightly yellow oil after Kugelrohr distillation at 185˚C and 1.0 mbar. Then, **250j** (6.40 g, 22.3 mmol) and 402 mg (2.11 mmol) *p*-TsOH•$H_2O$ were stirred in 65 mL benzene according to decarboxylation method A. Kugelrohr distillation at 1.0 mbar and 125˚C, followed by column chromatography (ethyl acetate:petroleum ether = 1:4) gave **251j** (1.6 g, 8.59 mmol, 39%) as a colourless oil.

**5-Acetoxy-nonan-4-on**

According to the general alkylation method, *tert*-butyl-2-acetoxy-aceto-acetate (**219a**) (1.08 g, 5.00 mmol), NaH (156 mg, 6.5 mmol) and hexylbromide (702 μL, 825 mg, 5.00 mmol) were reacted in DMF (10 mL) to give 2-acetoxy-2-acetyloctanoic-acid-*tert*-butylester (**250k**) (1063 mg, 3.54 mmol, 71%) as a slightly yellow oil. Then, **250k** (2.19 g, 7.29 mmol) and 139 mg (0.73 mmol) *p*-TsOH•$H_2O$ were stirred in 22.0 mL benzene according to decarboxylation method A. Kugelrohr distillation at 0.8 mbar and 80˚C and subsequent flash-chromatography (column dimensions: 2.0 □ 20.0 cm, diethylether/petroleum ether = 1:6) gave **251k** (419 mg 2.10 mmol, 29%) as a colourless oil.

MS(CI): m/z (%) = 113 (28), 142 (12), 158 (15), 171 (29), 185 (16), 201 (100), 202 (12).
HRMS: calculated for $C_{11}H_{21}O_3$ ($MH^+$): 201.14906 found: 201.14587

## Patentansprüche

**1.** α-Hydroxyketon-Verbindung der allgemeinen Formel I:

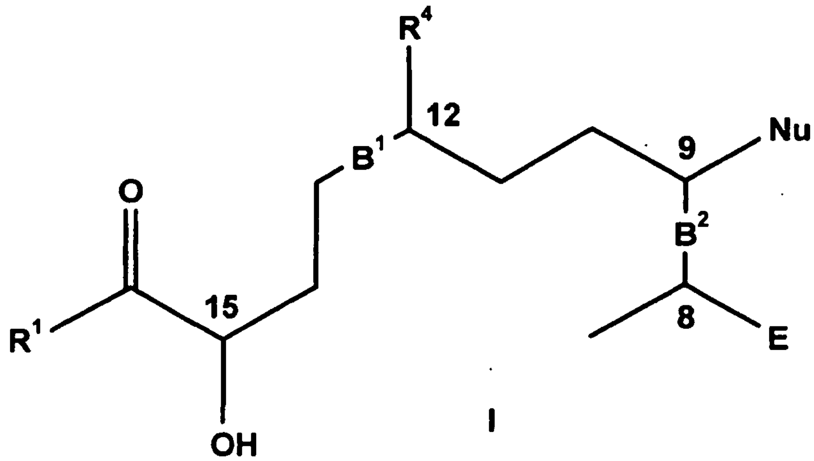

I

wobei

$R^1$ ausgewählt wird aus der Gruppe bestehend aus H, Methyl, $CH_nF_{3-n}$ (n = 0-3), Ethyl oder Propyl;
$B^1$ und/oder $B^2$ für eine Einfach- oder eine Doppelbindung als *E*-(*trans*)-Form, *Z*-(*cis*)-Form oder *E*/*Z*-Gemisch oder ein Epoxid als *E*-(*trans*)-Form, *Z*-(*cis*)-Form oder *E*/*Z*-Gemisch und/oder Kombinationen davon steht;
$R^4$ Methyl, Ethyl, $CH_nF_{3-n}$ (n = 0-3), oder Halogen ist;
Nu H oder Alkyl ist;
E $CH_3$, $CH_2OH$, $CH_2OPG$, $CO_2PG$, $CO_2R$ oder CH=O ist;
R H, Methyl oder Ethyl ist; und
PG für das angegebene Verknüpfungsatom oder die angegebene funktionelle Gruppe übliche Schutzgruppen bezeichnet,
ausgeschlossen eine Verbindung mit $R^1$ = Methyl, $B^1$ = Doppelbindung, $R^4$ = Methyl, Nu = H, $B^2$ = Doppelbindung und E = Methyl.

**2.** Verbindungen nach Anspruch 1, wobei $B^1$ für eine Z-Doppelbindung oder ein Epoxid steht.

**3.** Verbindungen nach Anspruch 1 oder 2, wobei $R^1$ Methyl ist.

**4.** Verbindungen nach einem der Ansprüche 1-3, wobei $B^2$ für eine Einfachbindung steht.

**5.** Verbindungen nach einem der Ansprüche 1 - 4, wobei PG Allyl, t-Butyl, Methyl, Benzyl, Silyl, Acyl, ein aktiviertes Methylenderivat wie Methoxymethyl, Alkoxyalkyl, 2-Oxacycloalkyl, Trimethylsilyl, Triethylsilyl, Dimethyl-tert-butylsilyl, Acetyl, Propionyl, Benzoyl oder Tetrahydropyranyl ist.

6. Verbindungen nach einem der Ansprüche 1 - 5, **dadurch gekennzeichnet, dass** die Verbindung an der α-Hydroxyposition an Position 15 der allgemeinen Formel I nicht racemisch bzw. optisch aktiv ist.

7. Verbindungen nach Anspruch 6, **dadurch gekennzeichnet, dass** die absolute Konfiguration an der α-Hydroxyposition an Position 15 der allgemeinen Formel I der natürlichen Konfiguration von Epothilonen an Position C15 entspricht.

8. Verbindungen nach einem der Ansprüche 1-7, **dadurch gekennzeichnet, dass** die α-Hydroxygruppe an Position 15 der allgemeinen Formel I durch eine Schutzgruppe PG geschützt ist.

9. Verbindungen nach Anspruch 8, **dadurch gekennzeichnet, dass** die Schutzgruppe PG ausgewählt ist aus der Gruppe bestehend aus einer Acetyl-, Propionyl-, Butyroyl und Benzoyl-Gruppe.

10. Verbindungen nach Anspruch 8, **dadurch gekennzeichnet, dass** die α-Hydroxygruppe an Position 15 der allgemeinen Formel I durch eine nicht-racemische chirale Acylgruppe geschützt ist.

11. Verbindungen nach Anspruch 10, **dadurch gekennzeichnet, dass** die α-Hydroxygruppe an Position 15 der allgemeinen Formel I mit optisch aktiver 2-Methoxymandelsäure verestert ist.

12. Verfahren zur Herstellung von Verbindungen nach einem der Ansprüche 1 bis 11, umfassend mindestens einen der nachfolgenden Schritte:

a) Umsetzung von Verbindungen der allgemeinen Formel II

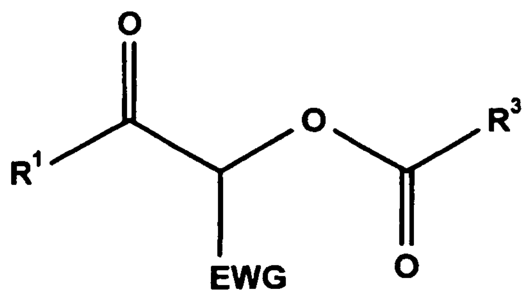

**(II)**

wobei $R^3$ ausgewählt wird aus der Gruppe bestehend aus Alkyl, Benzyl und Phenyl oder ein chiraler, nichtracemischer Rest R* ist; und EWG $CO_2PG$ ist;
mit einem Allylderivat vom Typ A (Anknüpfung bei X)

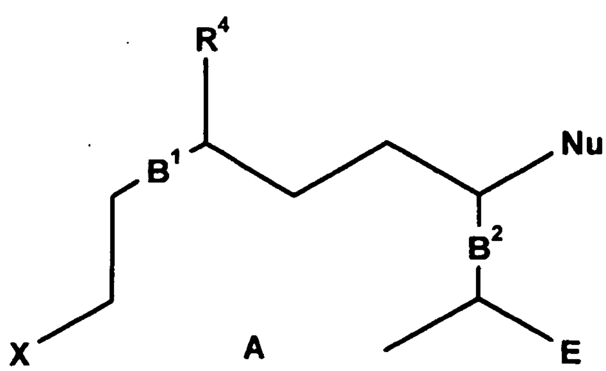

wobei X ausgewählt wird aus der Gruppe bestehend aus Cl, Br, I, O-Tosyl, Methylsulfonat, Trifluormethylsulfonat, Alkanoaten und Arylcarboxylaten;
zu Verbindungen der allgemeinen Formel III

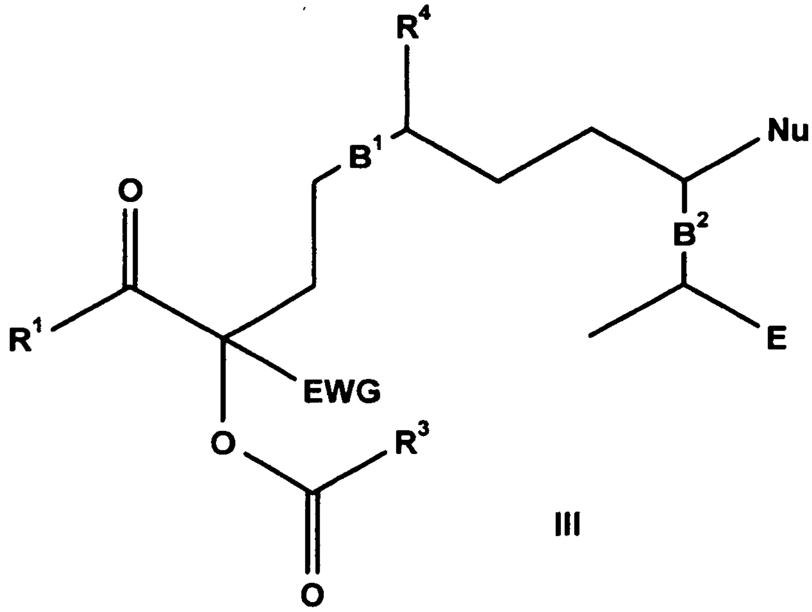

III

b) Umsetzung der Verbindungen der allgemeinen Formel III zu Verbindungen der allgemeinen Formel IV

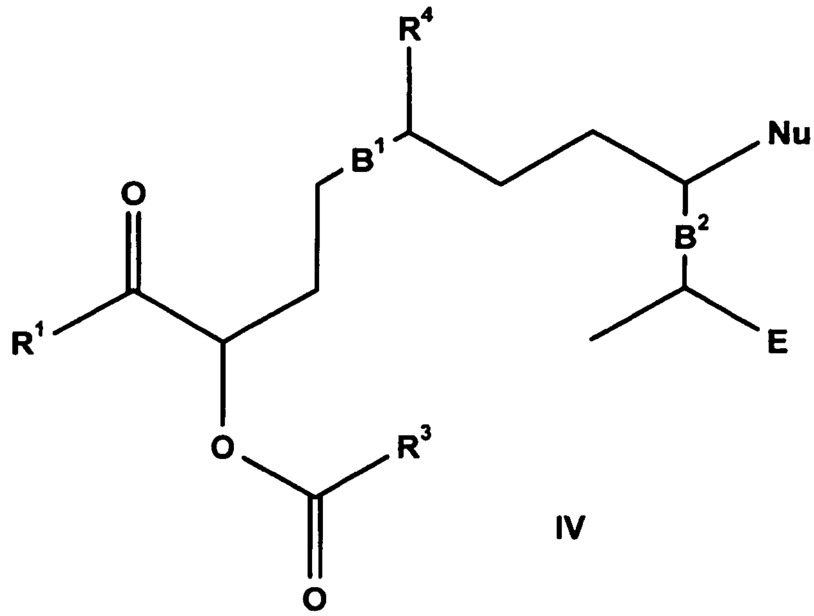

IV

c) Solvolyse der Verbindungen der allgemeinen Formel IV zu freien α-Hydroxyketonen der allgemeinen Formel I; und/oder

d) Racematspaltende Veresterung von Verbindungen der allgemeinen Formel I.

**13.** Verfahren nach Anspruch 12, **dadurch gekennzeichnet, dass** die Herstellung von an der α-Hydroxyposition nicht-racemischen Verbindungen der allgemeinen Formel I durch Umsetzung von Verbindungen mit den allgemeinen Formeln II, III und IV erfolgt, deren α-Hydroxygruppe durch eine nicht-racemische chirale Acylgruppe ($R^3$ = R*, wobei R* ein chiraler, nichtracemischer Rest ist) geschützt ist.

**14.** Verfahren nach Anspruch 12, **dadurch gekennzeichnet, dass** die Herstellung von an der α-Hydroxyposition nicht-racemischen Verbindungen der allgemeinen Formel I bzw. IV durch racematspaltende enzymatische Veresterung

von Verbindungen des Typs I erfolgt.

**15.** Verfahren nach Anspruch 14, **dadurch gekennzeichnet, dass** die Veresterung in Gegenwart eines Enzyms ausgewählt aus der Gruppe bestehend aus Lipasen und Esterasen erfolgt.

**16.** Verfahren nach Anspruch 15, **dadurch gekennzeichnet, dass** Lipasen aus *Pseudomonas cepacia* und/oder *Candida antarctica* bzw. Esterasen aus *Pseudomonas fluorescens* und/oder *Streptomyces diastatochromogenes* eingesetzt werden.

**17.** Verfahren nach Anspruch 12, **dadurch gekennzeichnet, dass** die Herstellung von an der α-Hydroxyposition nicht-racemischen Verbindungen der allgemeinen Formel I bzw. IV durch racematspaltende enzymatische Hydrolyse von Verbindungen des Typs IV erfolgt.

**18.** Verfahren nach Anspruch 17, **dadurch gekennzeichnet, dass** die Hydrolyse in Gegenwart eines Enzyms ausgewählt aus der Gruppe bestehend aus Lipasen und Esterasen erfolgt.

**19.** Verfahren nach Anspruch 18, **dadurch gekennzeichnet, dass** Lipasen aus *Pseudomonas cepacia* und/oder *Candida antarctica* bzw. Esterasen aus *Pseudomonas fluorescens* und/oder *Streptomyces diastatochromogenes* eingesetzt werden.

**20.** Verwendung einer Verbindung nach einem der Ansprüche 1 bis 11 zur Herstellung von Epothilonen und deren Derivaten.

## Claims

**1.** An α-hydroxyketone compound with the general formula I:

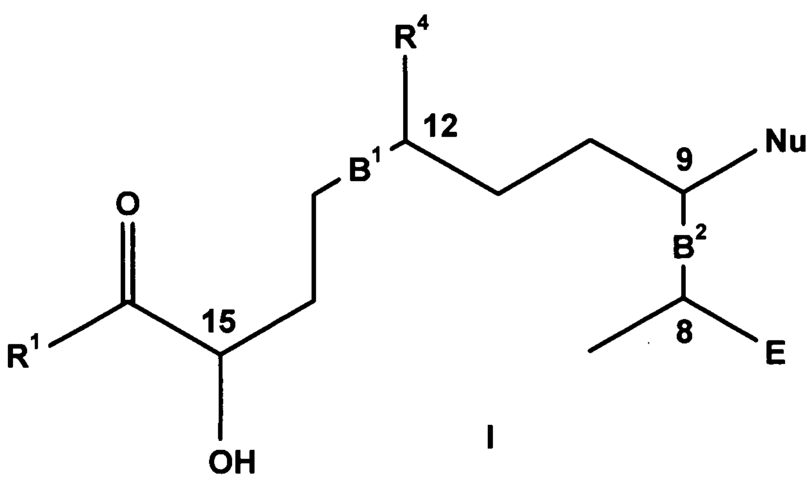

I

wherein

$R^1$ is selected from the group consisting of H, methyl, $CH_nF_{3-n}$ (n = 0-3), ethyl and propyl;
$B^1$ and/or $B^2$ stands for a single or a double bond in *E*-(*trans*) form, *Z-(cis)* form or *E*/*Z* mixture; or an epoxide in *E*-(*trans*) form, *Z*-(*cis*) form or *E*/*Z* mixture and/or combinations thereof;
$R^4$ is methyl, ethyl, $CH_nF_{3-n}$ (n = 0-3) or halogen;
Nu is H or alkyl;
E is $CH_3$, $CH_2OH$, $CH_2OPG$, $CO_2PG$, $CO_2R$ or CH=O;
R is H, methyl or ethyl; and
PG designates a common protective group for the specified linking atom or the specified functional group, wherein a compound with $R^1$ = methyl, $B^1$ = double bond, $R^4$ = methyl, Nu = H, $B^2$ = double bond and E = methyl is disclaimed.

**2.** A compound according to claim 1, **wherein** $B^1$ stands for a Z-double bond or an epoxide.

**3.** A compound according to claims 1 or 2, **wherein** $R^1$ is methyl.

**4.** A compound according to any of claims 1-3, **wherein** $B^2$ stands for a single bond.

**5.** A compound according to any of claims 1 - 4, **wherein** PG is allyl, t-butyl, methyl, benzyl, silyl, acyl, an activated methylen derivative such as methoxymethyl, alkoxyalkyl, 2-oxacycloalkyl, trimethylsilyl, triethylsilyl, dimethyl-*tert*-butylsilyl, acetyl, propionyl, benzoyl or tetrahydropyranyl.

**6.** A compound according to any of claims 1 - 5, **characterised in that** the compound is not racemic and/or optically active at the α-hydroxy position at position 15 of the general formula I.

**7.** A compound according claim 6, **characterised in that** the absolute configuration at the α-hydroxy position at position 15 of the general formula I corresponds to the natural configuration of epothilones at position C15.

**8.** A compound according to any of claims 1 - 7, **characterised in that** the α-hydroxy group at position 15 of the general formula I is protected by a protective group PG.

**9.** A compound according claim 8, **characterised in that** the protective group PG is selected from the group consisting of an acetyl group, a propionyl group, a butyroyl group and a benzoyl group.

**10.** A compound according claim 8, **characterised in that** the α-hydroxy group at position 15 of the general formula I is protected by a non-racemic chiral acyl group.

**11.** A compound according claim 10, **characterised in that** the α-hydroxy group at position 15 of the general formula I is esterified with optically active 2-methoxy mandelic acid.

**12.** A method for preparing compounds according to any of claims 1 - 11, **comprising** at least one of the following steps:

a) converting compounds with the general formula II:

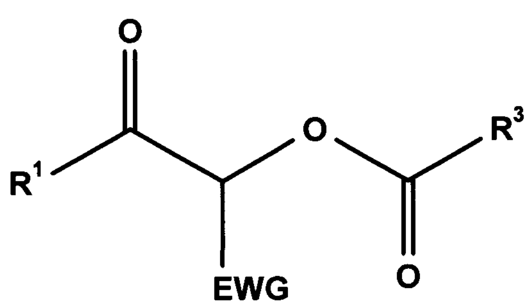

(II)

wherein
$R^3$ is selected from the group consisting of alkyl, benzyl and phenyl or is a chiral non-racemic moiety R*; and

EWG is $CO_2PG$;
with an allyl derivative of type A (linkage at X)

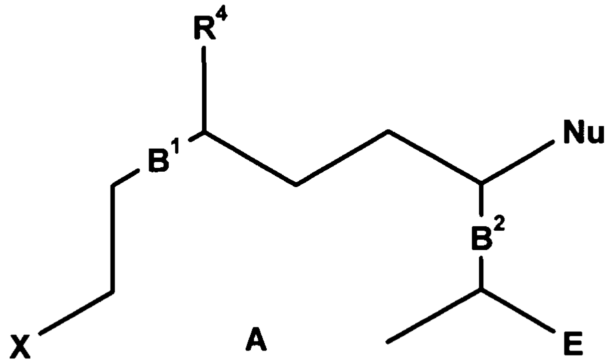

A

wherein X is selected from the group consisting of Cl, Br, I, O-tosyl, methyl sulfonate, trifluoromethyl sulfonate, alkanoates and arylcarboxylates;
to compounds of the general formula III

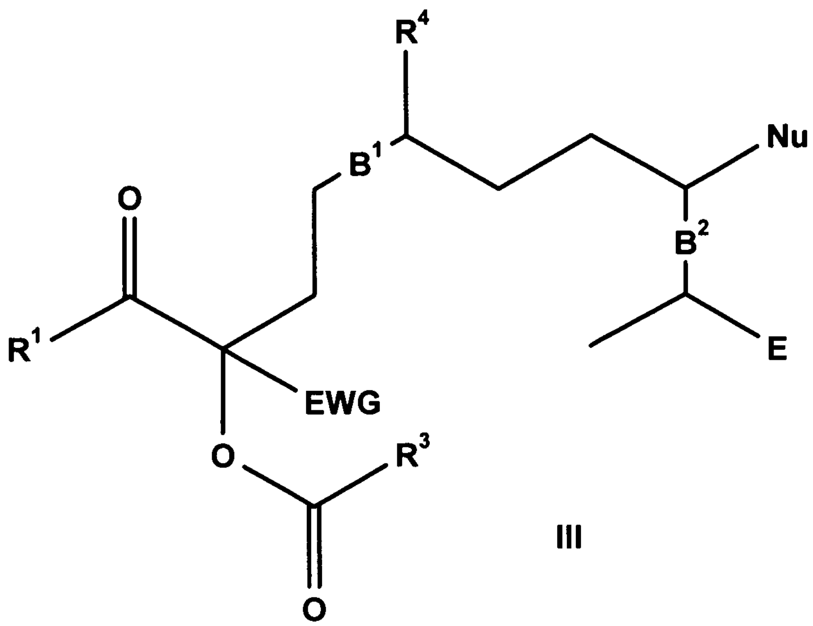

III

b) converting compounds with the general formula III to compounds with the general formula IV:

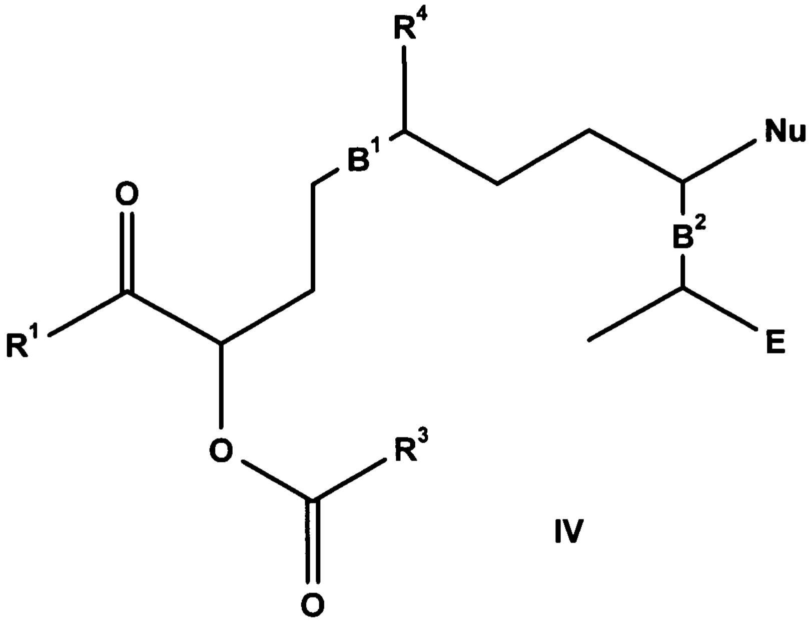

IV

c) solvolysing compounds with the general formula IV to free α-hydroxyketones of the general formula I; and/or
d) racemate cleavable esterification of compounds with the general formula I.

**13.** A method according to claim 12,
**characterised in that** the preparation of compounds with the general formula I being non-racemic at the α-hydroxy position is carried out by a reaction of compounds with the general formulas II, III and IV with their α-hydroxy group being protected by a non-racemic chiral acyl group ($R^3 = R^*$, wherein $R^*$ is a chiral non-racemic moiety).

**14.** A method according to claim 12,
**characterised in that** the preparation of compounds with the general formula I and/or IV being non-racemic at the α-hydroxy position is carried out by racemate cleavable enzymatic esterification of type I compounds.

**15.** A method according to claim 14,
**characterised in that** the esterification is carried out in the presence of an enzyme selected from the group consisting of lipases und esterases.

**16.** A method according to claim 15,
**characterised in that** lipases from *Pseudomonas cepacia* and/or *Candida antarctica* are used and/or esterases from *Pseudomonas fluorescens* and/or *Streptomyces diastatochromogenes* are used.

**17.** A method according to claim 12,
**characterised in that** the preparation of compounds with the general formula I and/or IV being non-racemic at the α-hydroxy position is carried out by a racemate cleavable enzymatic hydrolysis of type IV compounds.

**18.** A method according to claim 17,
**characterised in that** the hydrolysis is carried out in the presence of an enzyme selected from the group consisting of lipases und esterases.

**19.** A method according to claim 18,
**characterised in that** lipases from *Pseudomonas cepacia* and/or *Candida antarctica* are used and/or esterases from *Pseudomonas fluorescens* and/or *Streptomyces diastatochromogenes* are used.

**20.** Use of a compound according to any of claims 1 to 11 for the preparation of epothilones and derivatives thereof.

## Revendications

1. Composé α-hydroxycétonique de formule générale:

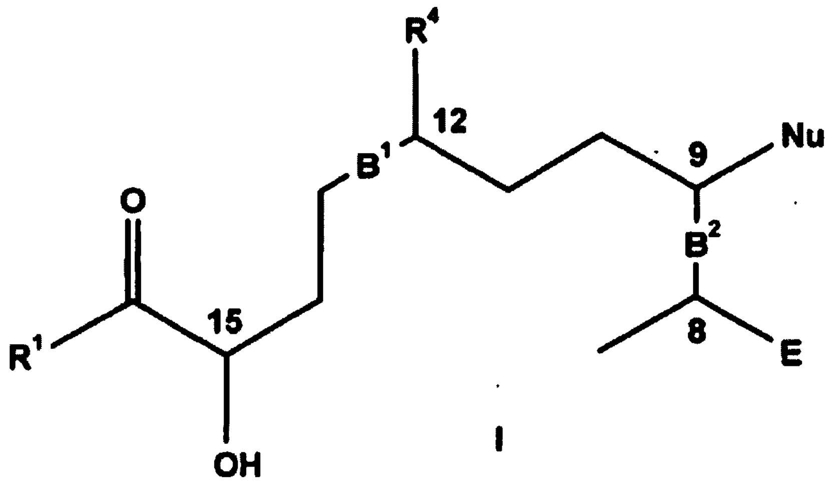

I

dans laquelle

$R^1$ est choisi dans le groupe se composant de H, méthyle, $CH_nF_{3-n}$ (n=0-3), éthyle ou propyle ;
$B^1$ et/ou $B^2$ représente une liaison simple ou double, en tant que forme E-(*trans*), forme *Z-(cis)* ou mélange E/Z ou un époxyde en tant que forme E-(*trans*), forme Z-(*cis*) ou mélange E/Z et/ou des combinaisons de ces formes ;
$R^4$ est le méthyle, l'éthyle, un $CH_nF_{3-n}$ (n=0-3), ou un halogène ;
Nu est H ou un alkyle ;
E est $CH_3$, $CH_2OH$, $CH_2OPG$, $CO_2PG$, $CO_2R$ ou CH=O;
R est H, méthyle ou éthyle ; et
PG désigne des groupes protecteurs usuels pour l'atome de combinaison par jonction indiqué ou pour le groupe fonctionnel indiqué,
à l'exception d'un composé avec $R^1$ = méthyle, B1 = double liaison, $R^4$= méthyle, Nu = H, $B^2$ = double liaison et E = méthyle.

2. Composés selon la revendication 1, dans lesquels $B^1$ représente une double liaison Z ou un époxyde.

3. Composés selon la revendication 1 ou 2, dans lesquels $R^1$ est le méthyle.

4. Composés selon l'une des revendications 1-3, dans lesquels $B^2$ représente une simple liaison.

5. Composés selon l'une des revendications 1-4, dans lesquels PG est un radical allyle, tertio-butyle, méthyle, benzyle, silyle, acyle, un dérivé méthylènique activé, tel que méthoxyméthyle, alcoxyalkyle, 2-oxacycloalkyle, triméthylsilyle, triéthylsilyle, diméthyl-tertio-butylsilyle, acétyle, propionyle, benzoyle ou tétrahydropyranyle.

6. Composés selon l'une des revendications 1-5, **caractérisés en ce que** le composé est, au niveau de la position du groupe hydroxy situé en α de la position 15 de la formule générale I, non racémique, respectivement optiquement actif.

7. Composés selon la revendication 6, **caractérisé en ce que** la configuration absolue au niveau de la position du groupe hydroxy, situé en α de la position 15 de la formule générale I, correspond à la configuration naturelle d'épothilones en position C15.

8. Composés selon l'une des revendications 1-7, **caractérisés en ce que** le groupe hydroxy, situé en α de la position 15 de la formule générale I, est protégé par un groupe protecteur PG.

**9.** Composés selon la revendication 8, **caractérisés en ce que** le groupe protecteur PG est choisi dans le groupe se composant de groupements acétyle, propionyle, butyroyle et benzoyle.

**10.** Composés selon la revendication 8, **caractérisés en ce que** le groupe hydroxy, situé en α de la position 15 de la formule générale I, est protégé par un groupe acyle chiral, non racémique.

**11.** Composés selon la revendication 10, **caractérisés en ce que** le groupe hydroxy, situé en α de la position 15 de la formule générale I, est complètement estérifié par de l'acide 2-méthoxymandélique optiquement actif.

**12.** Procédé pour la préparation de composés selon l'une des revendications 1 à 11, comportant au moins l'une des étapes suivantes :

a) réaction de composés de formule générale II

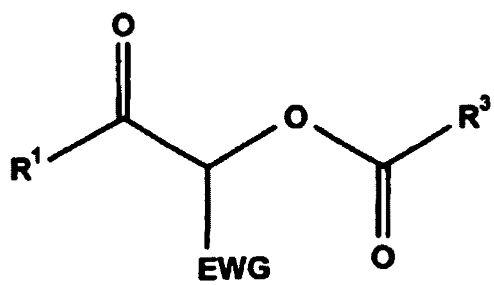

(II)

dans laquelle $R^3$ est choisi dans le groupe se composant de : alkyle, benzyle et phényle ou d'un radical R* chiral, non racémique ; et EWG est du $CO_2PG$ ;
avec un dérivé allylique de type A (jonction en position X)

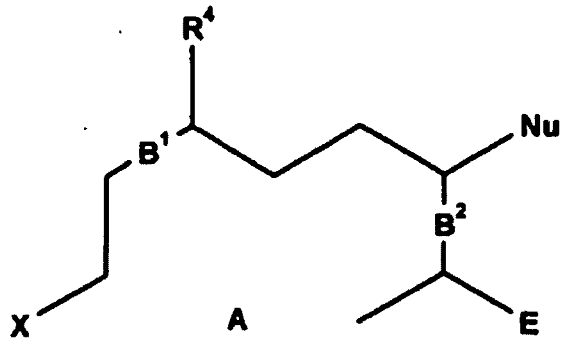

formule dans laquelle X est choisi dans le groupe se composant de Cl, Br, I, O-tosyle, sulfonate de méthyle, sulfonate de trifluorométhyle, alcanoates et arylcarboxylates ;
pour donner des composés de formule générale III

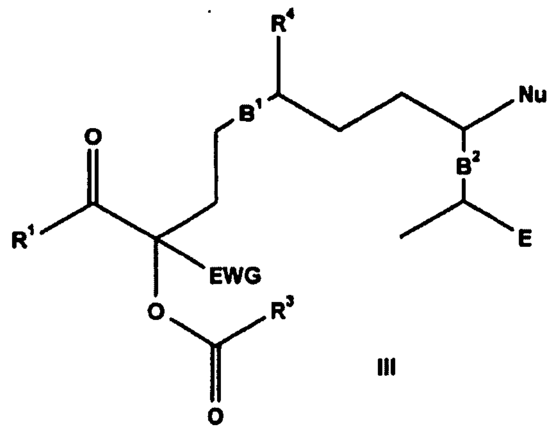

III

b) conversion des composés de formule générale III en composés de formule générale IV

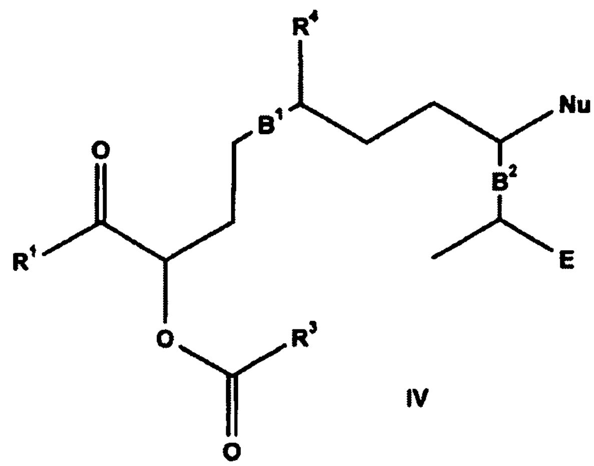

IV

c) solvolyse des composés de formule générale IV en α- hydroxycétones libres de formule générale I ; et/ou
d) estérification complète, impliquant une séparation du racémate, de composés de formule générale I.

**13.** Procédé selon la revendication 12, **caractérisé en ce que** la préparation de composés non racémiques au niveau de la position α du radical hydroxy et répondant à la formule générale 1 s'effectue par conversion de composés ayant les formules générales II, III et IV, dont le groupe hydroxy en α est protégé par un groupe acyle chiral, non racémique ($R^3$ = R*, dans lequel R* est un radical chiral, non racémique).

**14.** Procédé selon la revendication 12, **caractérisé en ce que** la préparation de composés non racémiques au niveau de la position α du radical hydroxy et répondant à la formule générale I, respectivement IV,
s'effectue par estérification enzymatique complète, impliquant une séparation du racémate, de composés du type I.

**15.** Procédé selon la revendication 14, **caractérisé en ce que** l'estérification complète s'effectue en présence d'un enzyme choisi dans le groupe se composant de lipases et d'estérases.

**16.** Procédé selon la revendication 15, **caractérisé en ce que** sont mises en oeuvre des lipases issues de *Pseudomonas cepacia* et/ou de *Candida antarctica,* respectivement des estérases issues de *Pseudomonas fluorescens* et/ou de *Streptomyces diastatochromogenes.*

**17.** Procédé selon la revendication 12, **caractérisé en ce que** la préparation de composés non racémiques au niveau de la position α du radical hydroxy et répondant à la formule générale I, respectivement IV, s'effectue par hydrolyse

enzymatique, impliquant une séparation du racémate, de composés du type IV.

**18.** Procédé selon la revendication 17, **caractérisé en ce que** l'hydrolyse s'effectue en présence d'un enzyme choisi dans le groupe se composant de lipases et d'estérases.

**19.** Procédé selon la revendication 18, **caractérisé en ce que** sont mises en oeuvre des lipases issues de *Pseudomonas cepacia* et/ou de *Candida antarctica,* respectivement des estérases issues de *Pseudomonas fluorescens* et/ou de *Streptomyces diastatochromogenes.*

**20.** Utilisation d'un composé selon l'une des revendications 1 à 11 en vue de la préparation d'épothilones et de leurs dérivés.